# FASCICULE DE BREVET EUROPEEN

(11) **EP 2 356 108 B1**
(45) Date de publication et mention de la délivrance du brevet: **15.10.2014**
(21) Numéro de dépôt: 09768186.0
(22) Date de dépôt: 13.11.2009
(51) Int. Cl.: C07D 401/14, C07D 403/14, C07D 407/14, C07D 413/12, C07D 413/14, C07D 417/14, A61P 25/00, A61K 31/445, A61K 31/497, A61K 31/506, A61K 31/501, A61K 31/498, A61K 31/4709, A61K 31/502, A61P 29/00

(54) **DERIVES DE CARBAMATES D'ALKYL-HETEROCYCLES, LEUR PREPARATION ET LEUR APPLICATION EN THERAPEUTIQUE**
CARBAMATDERIVATE VON ALKYLHETEROCYCLEN, DEREN HERSTELLUNG UND DEREN THERAPEUTISCHE VERWENDUNG
CARBAMATE DERIVATIVES OF ALKYL-HETEROCYCLES, PREPARATION THEREOF AND THERAPEUTIC USE THEREOF

(30) Priorité: 14.11.2008 FR 0806371
(43) Date de publication de la demande: 17.08.2011
(73) Titulaire: SANOFI, 75008 Paris (FR)
(72) Inventeur: ABOUABDELLAH, Ahmed, F-75013 Paris (FR); CHEREZE, Nathalie, F-75013 Paris (FR); FAYOL, Aude, F-75013 Paris (FR); SAADY, Mourad, F-75013 Paris (FR); VACHE, Julien, F-75013 Paris (FR); VERONIQUE, Corinne, F-75013 Paris (FR); YAICHE, Philippe, F-75013 Paris (FR)
(74) Mandataire: Le Coupanec, Pascale A.M.P.
(86) Numéro de dépôt international: PCT/FR2009/052179
(87) Numéro de publication internationale: WO 2010/055267

(56) Documents cités:
- EP-A- 1 780 210
- WO-A-2004/033422
- WO-A-2004/099176
- WO-A2-2010/010288

## Description

L'invention a pour objet des dérivés de carbamates d'alkyl-hétérocycles, leur préparation et leur application en thérapeutique.

Il existe toujours une nécessité de trouver et de développer des produits inhibiteurs de l'enzyme FAAH (Fatty Acid Amide Hydrolase). Les composés de l'invention répondent à ce but. Ces composés doivent présenter des propriétés métaboliques, pharmacocinétiques et toxicologiques permettant leur utilisation commme médicaments. Parmi ces propriétés, on peut notamment citer l'effet inhibiteur sur les cytochromes P450 et plus particulièrement sur l'iso-enzyme CYP3A4.

Le document WO2004/099176 décrit des composés ayant une activité inhibitrice de l'enzyme FAAH ayant un groupe carbamate de glycolamide.

La présente invention décrit des composés répondant à la formule générale (I) : 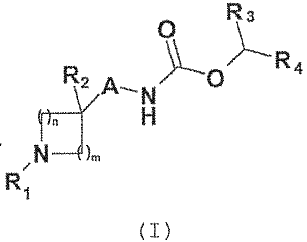 dans laquelle
R₂ représente un atome d'hydrogène, de fluor ou un groupe hydroxyle, cyano, trifluorométhyle, C₁₋₆-alkyle, C₁₋₆-alcoxy, NR₈R₉ ;
n représente un nombre entier égal à 1, 2 ou 3 et m représente un nombre entier égal à 1 ou 2;
A représente une liaison covalente ou un groupe C₁₋₈-alkylène ;
R₁ représente un groupe R₅ éventuellement substitué par un ou plusieurs groupes R₆ et/ou R₇ ;
   R₅ représente un groupe choisi parmi un phényle, pyridinyle, pyridazinyle, pyrimidinyle, pyrazinyle, triazinyle, naphtalènyle, quinolinyle, isoquinolinyle, phtalazinyle, quinazolinyle, quinoxalinyle, cinnolinyle, naphthyridinyle ;
   R₆ représente un atome d'halogène, un groupe cyano,
   -CH₂CN, nitro, hydroxyle, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₃-alkylène, C₃₋₇-cycloalkyle-C₁₋₃-alkylène-O-, NR₈R₉, NR₈COR₉, NR₈CO₂R₉, NR₈SO₂R₉, NR₈SO₂NR₈R₉, COR₈, CO₂R₉, CONR₈R₉, SO₂R₈, SO₂NR₈R₉ ou -O-(C₁₋₃-alkylène)-O- ;
   R₇ représente un groupe choisi parmi un furanyle, pyrrolyle, thiènyle, oxazolyle, isoxazolyle, thiazolyle, isothiazolyle, imidazolyle, pyrazolyle, oxadiazolyle, thiadiazolyle, phényle, pyridinyle, pyridazinyle, pyrimidinyle, pyrazinyle, triazinyle, naphtalènyle, quinolinyle, isoquinolinyle, phtalazinyle, quinazolinyle, quinoxalinyle, cinnolinyle, naphthyridinyle, imidazopyrimidinyle, thiènopyrimidinyle, benzofuranyle, benzothiènyle, benzimidazolyle, benzoxazolyle, benzisoxazolyle, benzothiazolyle, benzisothiazolyle, indolyle, isoindolyle, indazolyle, pyrrolopyridinyle, furopyridinyle, thiènopyridinyle, imidazopyridinyle, pyrazolopyridinyle, oxazolopyridinyle, isoxazolopyridinyle, thiazolopyridinyle, phényloxy, benzyloxy, pyrimidinoxy; le ou les groupes R₇ pouvant être substitués par un ou plusieurs groupes R₆ identiques ou différents l'un de l'autre ;
R₃ représente un atome d'hydrogène, de fluor, un groupe C₁₋₆-alkyle ou un groupe trifluorométhyle ;
R₄ représente un groupe choisi parmi un furanyle, pyrrolyle, thiènyle, isothiazolyle, oxazolyle, isoxazolyle, pyrazolyle, oxadiazolyle, thiadiazolyle, imidazolyle, triazolyle, tétrazolyle;
ce groupe étant éventuellement substitué par un ou plusieurs substituants choisis parmi un atome d'halogène, un groupe C₁₋₆-alkyle, C₁₋₆-haloalkyle, C₃₋₇-cycloalkyle, C₃₋₇-cycloalkyle-C₁₋₃-alkylène, C₁₋₆-haloalcoxy, cyano, NR₈R₉, NR₈COR₉, NR₈CO₂R₉, NR₈SO₂R₉, NR₉SO₂NR₈R₉, COR₈, CO₂R₈, CONR₈R₉, CON(R₈) (C₁₋₃-alkylène-NR₁₀R₁₁), SO₂R₈, SO₂NR₈R₉, -O-(C₁₋₃-alkylène)-O-, phényle, phényloxy, benzyloxy, pyridinyle, pyrazinyle, pyridazinyle, triazinyle ou pyrimidinyle; les groupes phényle, phényloxy, pyridinyle, pyrazinyle, pyridazinyle, triazinyle et pyrimidinyle pouvant être substitués par un ou plusieurs substituants choisis parmi un atome d'halogène, un groupe cyano, nitro, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₃-alkylène
R₈, R₉, R₁₀ et R₁₁ représentent indépendamment l'un de l'autre un atome d'hydrogène, ou un groupe C₁₋₆-alkyle,
ou forment avec le ou les atomes qui les portent,
dans le cas de NR₈R₉, un cycle choisi parmi les cycles azétidine, pyrrolidine, pipéridine, morpholine, thiomorpholine, azépine, oxazépine ou pipérazine, ce cycle étant éventuellement substitué par un groupe C₁₋₆-alkyle ou benzyle ;
dans le cas de NR₈COR₉, un cycle lactame ; dans le cas de NR₈CO₂R₉, un cycle oxazolidinone, oxazinone ou oxazépinone; dans le cas de NR₈SO₂R₉, un cycle sultame ; dans le cas de NR₈SO₂NR₈R₉, un cycle dioxyde de thiazolidine ou dioxyde de thiadiazinane.

La présente invention concerne les composés de formule générale (I) pour lesquels R₂ représente un atome d'hydrogène ou de fluor ou un groupe hydroxyle, C₁₋₆-alkyle ou NR₈R₉ et R₃ représente un trifluorométhyle, un C₁₋₆ alkyle ou un atome d'hydrogène. Plus particulièrement, R₈ et R₉ représentent un groupe C₁₋₆-alkyle.

Parmi les composés de formule générale (I), un premier sous-groupe de composés est constitué des composés pour lesquels R₂ représente un atome d'hydrogène.

Parmi les composés de formule générale (I), un deuxième sous-groupe de composés est constitué des composés pour lesquels n représente un nombre entier égal à 2 et m représente un nombre entier égal à 2.

Parmi les composés de formule générale (I), un troisième sous-groupe de composés est constitué des composés pour lesquels A représente un groupe C₁₋₈-alkylène, plus particulièrement un groupe éthylène, propylène ou éthylène. Parmi les composés de formule générale (I), un quatrième sous-groupe de composés est constitué des composés pour lesquels A représente une liaison covalente.

Parmi les composés de formule générale (I), un cinquième sous-groupe de composés est constitué des composés pour lesquels R₁ représente un groupe R₅ éventuellement substitué par un ou plusieurs groupes R₆ et/ou R₇ ;
R₅ représente un groupe phényle, pyridinyle, pyridazinyle, pyrimidinyle, pyrazinyle, quinolinyle, phtalazinyle ou quinoxalinyle ;
R₆ représente un groupe nitro, C₁₋₆-alkyle, C₁₋₆-alcoxy, C₁₋₆-haloalkyle, C₁₋₆-haloalcoxy, -O-(C₁₋₃-alkylène)-O- ou un atome d'halogène, plus particulièrement un atome de chlore ou de fluor;
R₇ représente un groupe phényle pouvant être substitué par un ou plusieurs groupes R₆ identiques ou différents l'un de l'autre.

Parmi les composés de formule générale (I), un sixième sous-groupe de composés est constitué des composés pour lesquels R₁ représente un groupe R₅ éventuellement substitué par un ou plusieurs groupes R₆ et/ou R₇ ;
R₅ représente un groupe pyridinyle, pyridazinyle, pyrimidinyle, pyrazinyle, quinolinyle, phtalazinyle ou quinoxalinyle ;
R₆ représente un groupe nitro, C₁₋₆-alkyle, C₁₋₆-alcoxy, C₁₋₆-haloalkyle, C₁₋₆-haloalcoxy, -O-(C₁₋₃-alkylène)-O- ou un atome d'halogène, plus particulièrement un atome de chlore ou de fluor;
R₇ représente un groupe phényle pouvant être substitué par un ou plusieurs groupes R₆ identiques ou différents l'un de l'autre.

Parmi les composés de formule générale (I), un septième sous-groupe de composés est constitué des composés pour lesquels R₁ représente un groupe R₅ éventuellement substitué par un ou plusieurs groupes R₆ et/ou R₇ ;
R₅ représente un groupe phényle, pyridinyle, pyrazinyle ou quinolinyle ;
R₆ représente un atome d'halogène, plus particulièrement un atome de chlore ou de fluor;
R₇ représente un groupe phényle pouvant être substitué par un ou plusieurs groupes R₆ identiques ou différents l'un de l'autre.

Parmi les composés de formule générale (I), un huitième sous-groupe de composés est constitué des composés pour lesquels R₃ représente un trifluorométhyle, un C₁₋₆-alkyle, plus particulièrement un isobutyle, ou un atome d'hydrogène. Parmi les composés de formule générale (I), un neuvième sous-groupe de composés est constitué des composés pour lesquels R₃ représente un atome d'hydrogène.

Parmi les composés de formule générale (I), un dixième sous-groupe de composés est constitué des composés pour lesquels R₄ représente un groupe choisi parmi un oxazolyle, isoxazolyle, furanyle, imidazolyle, pyrazolyle, oxadiazolyle, thiadiazolyle, triazolyle ;
ce groupe éventuellement substitué par un ou plusieurs substituants choisis parmi un groupe C₁₋₆-alkyle, plus particulièrement méthyle, éthyle, isopropyle ou *tert*-butyle, COOR₈, CON(R₆) (C₁₋₃-alkylène-NR₁₀R₁₁), CONR₈R₉, phényle ; le groupe phényle pouvant être substitué par un ou plusieurs substituants choisis parmi un atome d'halogène, plus particulièrement de chlore ou de fluor, un groupe C₁₋₆-alcoxy, plus particulièrement méthoxy ;
R₈ et R₉ représentent indépendamment l'un de l'autre un atome d'hydrogène ou un groupe C₁₋₆-alkyle, plus particulièrement un méthyle ou éthyle, ou forment ensemble avec l'atome qui les porte un cycle pipérazine,
R₁₀ et R₁₁ représentent indépendamment l'un de l'autre un atome d'hydrogène ou un groupe C₁₋₆-alkyle.

Parmi les composés de formule générale (I), un onzième sous-groupe de composés est constitué des composés pour lesquels R₄ représente un groupe choisi parmi un oxazolyle, isoxazolyle, pyrazolyle, oxadiazolyle, thiadiazolyle, imidazolyle, triazolyle, tétrazolyle;
ce groupe éventuellement substitué par un ou plusieurs substituants choisis parmi un groupe C₁₋₆-alkyle, plus particulièrement méthyle, éthyle, isopropyle ou *tert*-butyle, COOR₈, CON (R₈) (C₁₋₃-alkylène-NR₁₀R₁₁), CONR₈R₉, phényle ; le groupe phényle pouvant être substitué par un ou plusieurs substituants choisis parmi un atome d'halogène, plus particulièrement de chlore ou de fluor, un groupe C₁₋₆-alcoxy, plus particulièrement méthoxy ;
R₈ et R₉ représentent indépendamment l'un de l'autre un atome d'hydrogène ou un groupe C₁₋₆-alkyle, plus particulièrement un méthyle ou éthyle, ou forment ensemble avec l'atome qui les porte un cycle pipérazine,
R₁₀ et R₁₁ représentent indépendamment l'un de l'autre un atome d'hydrogène ou un groupe C₁₋₆-alkyle.

Parmi les composés de formule générale (I), un douzième sous-groupe de composés est constitué des composés pour lesquels R₄ représente un groupe choisi parmi un oxazolyle, isoxazolyle, pyrazolyle, oxadiazolyle, thiadiazolyle, triazolyle ; ce groupe éventuellement substitué par un ou plusieurs substituants choisis parmi un groupe C₁₋₆-alkyle, plus particulièrement méthyle, éthyle, isopropyle ou *tert-*butyle, CONR₈R₉, phényle ; le groupe phényle pouvant être substitué par un ou plusieurs substituants choisis parmi un atome d'halogène, plus particulièrement de chlore ou de fluor, un groupe C₁₋₆-alcoxy, plus particulièrement méthoxy ; R₈ et R₉ représentent indépendamment l'un de l'autre un atome d'hydrogène ou un groupe C₁₋₆-alkyle, plus particulièrement un méthyle.

Parmi les composés de formule générale (I), un treizième sous-groupe de composés est constitué des composés pour lesquels R₄ représente un groupe 3-carbamoyl-isoxazol-5-yle.

Parmi les composés de formule générale (I), un quatorzième sous-groupe de composés est constitué des composés pour lesquels R₄ représente un groupe 2-méthyl-2H-[1,2,4]triazol-3-yle.

Parmi les composés de formule générale (I), un quinzième sous-groupe de composés est constitué des composés pour lesquels R₄ représente un groupe 3-(4-chloro-phényl)-[1,2,4]oxadiazol-5-yle.

Parmi les composés de formule générale (I), un seizième sous-groupe de composés est constitué des composés pour lesquels R₄ représente un groupe 3-(4-chloro-phényl)-isoxazol-5-yle.

Parmi les composés de formule générale (I), un dix-septième sous-groupe de composés est constitué des composés pour lesquels R₄ représente un groupe 3-éthyl-[1,2,4]oxadiazol-5-yle.

Parmi les composés de formule générale (I), un dix-huitième sous-groupe de composés est constitué des composés pour lesquels R₄ représente un groupe 5-méthyl-3-phényl-isoxazol-4-yle.

Parmi les composés de formule générale (I), dix-neuvième sous-groupe de composés est constitué des composés pour lesquels R₄ représente un groupe 3-isopropyl-[1,2,4]oxadiazol-5-yle.

Parmi les composés de formule générale (I), un vingtième sous-groupe de composés est constitué des composés pour lesquels R₄ représente un groupe 1-méthyl-1H-pyrazol-3-yle.

Parmi les composés de formule générale (I), un vingt-et-unième sous-groupe de composés est constitué des composés pour lesquels R₄ représente un groupe [1,2,3]thiadiazol-4-yle.

Parmi les composés de formule générale (I), un vingt-deuxième sous-groupe de composés est constitué des composés

pour lesquels R₄ représente un groupe 5-tert-butyl-[1,3,4]thiadiazol-2-yle.

Parmi les composés de formule générale (I), un vingt-troisième sous-groupe de composés est constitué des composés pour lesquels R₄ représente un groupe 5-isopropyl-[1,2,4]oxadiazol-3-yle.

Parmi les composés de formule générale (I), un vingt-quatrième sous-groupe de composés est constitué des composés pour lesquels R₄ représente un groupe 5-(4-fluoro-phényl)-[1,3,4]oxadiazol-2-yle.

Parmi les composés de formule générale (I), un vingt-cinquième sous-groupe de composés est constitué des composés pour lesquels R₄ représente un groupe 5-(4-chloro-phényl)-[1,3,4]oxadiazol-2-yle.

Parmi les composés de formule générale (I), un vingt-sixième sous-groupe de composés est constitué des composés pour lesquels R₄ représente un groupe 5-(4-méthoxy-phényl)-[1,3,4]oxadiazol-2-yle.

Parmi les composés de formule générale (I), un vingt-septième sous-groupe de composés est constitué des composés pour lesquels R₄ représente un groupe 3-(4-fluoro-phényl)-[1,2,4]oxadiazol-5-yle.

Parmi les composés de formule générale (I), un vingt-huitième sous-groupe de composés est constitué des composés pour lesquels R₄ représente un groupe 3-(3-fluoro-phényl)-[1,2,4]oxadiazol-5-yle.

Parmi les composés de formule générale (I), un vingt-neuvième sous-groupe de composés est constitué des composés pour lesquels R₄ représente un groupe 5-(4-chloro-phényl)-[1,2,4]thiadiazol-3-yle.

Parmi les composés de formule générale (I), un trentième sous-groupe de composés est constitué des composés pour lesquels R₄ représente un groupe 3-méthylcarbamoyl-isoxazol-5-yle.

Parmi les composés de formule générale (I), un trente-et-unième sous-groupe de composés est constitué des composés pour lesquels R₄ représente un groupe 4-carbamoyl-oxazol-2-yle.

Parmi les composés de formule générale (I), un trente-deuzième sous-groupe de composés est constitué des composés pour lesquels R₄ représente un groupe 3-diméthylcarbamoyl-isoxazol-5-yle.

Parmi les composés de formule générale (I), un trente-troisième sous-groupe de composés est constitué des composés pour lesquels R₁ représente un groupe R₅ éventuellement substitué par un ou plusieurs groupes R₆ et/ou R₇ ;
R₅ représente un groupe phényle, pyridinyle, pyrazinyle ou quinolinyle ; R₆ représente un atome d'halogène, plus particulièrement un atome de chlore ou de fluor;
R₇ représente un groupe phényle pouvant être substitué par un ou plusieurs groupes R₆ identiques ou différents l'un de l'autre.
R₂ et R₃ représentent un atome d'hydrogène ; R₄ représente un groupe 3-carbamoyl-isoxazol-5-yle ; n représente un nombre entier égal à 2 et m représente un nombre entier égal à 2;
A représente un groupe alkylène.

Parmi les composés de formule générale (I), un trente-quatrième sous-groupe de composés est constitué des composés pour lesquels R₁ représente un groupe R₅ éventuellement substitué par un ou plusieurs groupes R₆ et/ou R₇ ;
R₅ représente un groupe pyridinyle, pyridazinyle, pyrimidinyle, pyrazinyle, quinolinyle, phtalazinyle ou quinoxalinyle ;
R₆ représente un groupe nitro, C₁₋₆-alkyle, C₁₋₆-alcoxy, C₁₋₆,-haloalkyle, C₁₋₆-haloalcoxy, -O-(C₁₋₃-alkylène)-O- ou un atome d'halogène, plus particulièrement un atome de chlore ou de fluor;
R₇ représente un groupe phényle pouvant être substitué par un ou plusieurs groupes R₆ identiques ou différents l'un de l'autre.
R₂ et R₃ représentent un atome d'hydrogène ;
R₄ représente un groupe choisi parmi un furanyle, pyrrolyle, thiènyle, isothiazolyle, oxazolyle, isoxazolyle, pyrazolyle, oxadiazolyle, thiadiazolyle, imidazolyle, triazolyle, tétrazolyle éventuellement substitué par CONR₈R₉ où R₈ et R₉ représentent indépendamment l'un de l'autre un atome d'hydrogène ou un groupe C₁₋₆-alkyle ;
n représente un nombre entier égal à 2 et m représente un nombre entier égal à 2; A représente un groupe alkylène.

Parmi les composés de formule générale (I), un trente-cinquième sous-groupe de composés est constitué par les composés de formule générale (I) dans laquelle à la fois R₁ et/ou R₂ et/ou R₃ et/ou R₄ et/ou n et/ou m et/ou A sont tels que définis dans les groupes ci-dessus.

Parmi les composés de formule générale (I), les composés suivants peuvent être cités (nomenclature IUPAC générée par le logiciel AutoNom) :
1. 2-[1-(6-Chloro-quinolin-2-yl)-pipéridin-4-yl]-éthylcarbamate de 3-carbamoyl-isoxazol-5-ylméthyle
2. 2-[1-(6-Chloro-quinolin-2-yl)-pipéridin-4-yl]-éthylcarbamate de 2-méthyl-2H-[1,2,4]triazol-3-ylméthyle
3. 2-[1-(6-Chloro-quinolin-2-yl)-pipéridin-4-yl]-éthylcarbamate de 3-(4-chloro-phényl)-isoxazol-5-ylméthyle
4. 2-[1-(6-Chloro-quinolin-2-yl)-pipéridin-4-yl]-éthylcarbamate de 3-(4-chloro-phényl)-[1,2,4]oxadiazol-5-ylméthyle
5. 2-[5'-(4-Fluoro-phényl)-3,4,5,6-tétrahydro-2H-[1,2']bipyridinyl-4-yl]-éthylcarbamate de 3-éthyl-[1,2,4]oxadiazol-5-ylméthyle
6. 2-[1-(6-Chloro-quinolin-2-yl)-pipéridin-4-yl]-éthylcarbamate de 5-méthyl-3-phényl-isoxazol-4-ylméthyle
7. 2-[5'-(4-Fluoro-phényl)-3,4,5,6-tétrahydro-2H-[1,2']bipyridinyl-4-yl]-éthylcarbamate de 3-isopropyl-[1,2,4]oxadiazol-5-ylméthyle
8. 2-[5'-(4-Fluoro-phényl)-3,4,5,6-tétrahydro-2H-[1,2']bipyridinyl-4-yl]-éthylcarbamate de 1-méthyl-1H-pyrazol-3-ylméthyle
9. 2-[5'-(4-Fluoro-phényl)-3,4,5,6-tétrahydro-2H-[1,2']bipyridinyl-4-yl]-éthylcarbamate de [1,2,3]thiadiazol-4-ylméthyle
10. 2-[5'-(4-Fluoro-phényl)-3,4,5,6-tétrahydro-2H-[1,2']bipyridinyl-4-yl]-éthylcarbamate de 5-tert-butyl-[1,3,4]thiadiazol-2-ylméthyle
11. 2-[5'-(4-Fluoro-phényl)-3,4,5,6-tétrahydro-2H-[1,2']bipyridinyl-4-yl]-éthylcarbamate de 5-isopropyl-[1,2,4]oxadiazol-3-ylméthyle
12. 2-[1-(6-Chloro-quinolin-2-yl)-pipéridin-4-yl]-éthylcarbamate de 5-(4-fluoro-phényl)-[1,3,4]oxadiazol-2-ylméthyle
13. 2-[1-(6-Chloro-quinolin-2-yl)-pipéridin-4-yl]-éthylcarbamate de 5-(4-chloro-phényl)-[1,3,4]oxadiazol-2-ylméthyle
14. 2-[1-(6-Chloro-quinolin-2-yl)-pipéridin-4-yl]-éthylcarbamate de 5-(4-méthoxy-phényl)-[1,3,4]oxadiazol-2-ylméthyle
15. 2-[1-(6-Chloro-quinolin-2-yl)-pipéridin-4-yl]-éthylcarbamate de 3-(4-fluoro-phényl)-[1,2,4]oxadiazol-5-ylméthyle
16. 2-[1-(6-Chloro-quinolin-2-yl)-pipéridin-4-yl]-éthylcarbamate de 3-(3-fluoro-phényl)-[1,2,4]oxadiazol-5-ylméthyle
17. 2-[1-(6-Chloro-quinolin-2-yl)-pipéridin-4-yl]-éthylcarbamate de 5-(4-chloro-phényl)-[1,2,4]thiadiazol-3-ylméthyle
18. 2-[1-(6-Chloro-quinolin-2-yl)-pipéridin-4-yl]-éthylcarbamate de [1,2,3]thiadiazol-4-ylméthyle
19. 2-[5'-(4-Fluoro-phényl)-3,4,5,6-tétrahydro-2H-[1,2']bipyridinyl-4-yl]-éthylcarbamate de 3-carbamoyl-isoxazol-5-ylméthyle
20. 2-[1-(6-Chloro-quinolin-2-yl)-pipéridin-4-yl]-éthylcarbamate de 3-méthylcarbamoyl-isoxazol-5-ylméthyle
21. 2-[5'-(4-Fluoro-phényl)-3,4,5,6-tétrahydro-2H-[1,2']bipyridinyl-4-yl]-éthylcarbamate de 4-carbamoyl-oxazol-2-ylméthyle
22. 2-[6'-(4-Fluoro-phényl)-3,4,5,6-tétrahydro-2H-[1,2']bipyridinyl-4-yl]-éthylcarbamate de 3-méthylcarbamoyl-isoxazol-5-ylméthyle
23. 2-[1-(6-Fluoro-quinolin-2-yl)-pipéridin-4-yl]-éthylcarbamate de 3-carbamoyl-isoxazol-5-ylméthyle
24. 2-[6'-(4-Fluoro-phényl)-3,4,5,6-tétrahydro-2H-[1,2']bipyridinyl-4-yl]-éthylcarbamate de 3-carbamoyl-isoxazol-5-ylméthyle
25. 2-[1-(6-Fluoro-quinolin-2-yl)-pipéridin-4-yl]-éthylcarbamate de 3-méthylcarbamoyl-isoxazol-5-ylméthyle
26. 2-[5'-(4-Fluoro-phényl)-3,4,5,6-tétrahydro-2H-[1,2']bipyridinyl-4-yl]-éthylcarbamate de 3-méthylcarbamoyl-isoxazol-5-ylméthyle
27. 2-[6'-(4-Fluoro-phényl)-3,4,5,6-tétrahydro-2H-[1,2']bipyridinyl-4-yl]-éthylcarbamate de 3-diméthylcarbamoyl-isoxazol-5-ylméthyle
28. 2-[5'-(3-Fluoro-phényl)-3,4,5,6-tétrahydro-2H-[1,2']bipyridinyl-4-yl]-éthylcarbamate de 3-carbamoyl-isoxazol-5-ylméthyle
29. 2-[1-(4'-Fluoro-biphényl-4-yl)-pipéridin-4-yl]-éthylcarbamate de 3-méthylcarbamoyl-isoxazol-5-ylméthyle
30. 2-[5'-(4-Fluoro-phényl)-3,4,5,6-tétrahydro-2H-[1,2']bipyridinyl-4-yl]-éthylcarbamate de 3-diméthylcarbamoyl-isoxazol-5-ylméthyle
31. 2-[1-(6-Chloro-quinolin-2-yl)-pipéridin-4-yl]-éthylcarbamate de 3-diméthylcarbamoyl-isoxazol-5-ylméthyle
32. 2-{1-[6-(4-Fluoro-phényl)-pyrazin-2-yl]-pipéridin-4-yl}-éthylcarbamate de 3-méthylcarbamoyl-isoxazol-5-ylméthyle
33. (2-{1-[6-(4-Fluoro-phényl)-pyrazin-2-yl]-pipéridin-4-yl]-éthylcarbamate de 3-carbamoyl-isoxazol-5-ylméthyle
34. (2-{1-[6-(4-Fluoro-phényl)-pyrazin-2-yl]-pipéridin-4-yl]-éthylcarbamate de 3-diméthylcarbamoyl-isoxazol-5-ylméthyle
35. (2-{1-[5-(4-Fluoro-phényl)-pyrimidin-2-yl]-pipéridin-4-yl]-éthylcarbamate de 3-méthylcarbamoyl-isoxazol-5-ylméthyle
36. (2-{1-[5-(4-Fluoro-phényl)-pyrimidin-2-yl]-pipéridin-4-yl]-éthylcarbamate de 3-diméthylcarbamoyl-isoxazol-5-ylméthyle
37. {2-[1-(4-Trifluorométhyl-pyrimidin-2-yl)-pipéridin-4-yl]-éthylcarbamate de 3-méthylcarbamoyl-isoxazol-5-ylméthyle
38. {2-[1-(4-Trifluorométhyl-pyrimidin-2-yl)-pipéridin-4-yl]-éthylcarbamate de 3-carbamoyl-isoxazol-5-ylméthyle
39. (2-{1-[5-(4-Fluoro-phényl)-pyrimidin-2-yl]-pipéridin-4-yl]-éthylcarbamate de 3-carbamoyl-isoxazol-5-ylméthyle
40. {2-[1-(4-Trifluorométhyl-pyrimidin-2-yl)-pipéridin-4-yl]-éthylcarbamate de 3-diméthylcarbamoyl-isoxazol-5-ylméthyle
41. (2-{1-[6-(4-Fluoro-phényl)-pyridazin-3-yl]-pipéridin-4-yl]-éthylcarbamate de 3-carbamoyl-isoxazol-5-ylméthyle
42. {2-[5'-(4-Fluoro-phényl)-3,4,5,6-tétrahydro-2H-[1,2']bipyridinyl-4-yl]-éthylcarbamate de 3-(2-diméthylamino-éthylcarbamoyl)-isoxazol-5-ylméthyle
43. [2-(5'-Isobutyl-3,4,5,6-tétrahydro-2H-[1,2']bipyridinyl-4-yl]-éthylcarbamate de 3-carbamoyl-isoxazol-5-ylméthyle
44. {2-[5'-(2,2-Diméthyl-propyl)-3,4,5,6-tétrahydro-2H-[1,2']bipyridinyl-4-yl]-éthylcarbamate de 3-carbamoyl-isoxazol-5-ylméthyle
45. [2-(5'-m-Tolyl-3,4,5,6-tétrahydro-2H-[1,2']bipyridinyl-4-yl]-éthylcarbamate de 3-carbamoyl-isoxazol-5-ylméthyle
46. {2-[5'-(4-Fluoro-phényl)-3,4,5,6-tétrahydro-2H-[1,2']bipyridinyl-4-yl]-éthylcarbamate de 3-(4-méthyl-pipérazine-1-carbonyl)-isoxazol-5-ylméthyle
47. {2-[5'-(3-Trifluorométhoxy-phényl)-3,4,5,6-tétrahydro-2H-[1,2']bipyridinyl-4-yl]-éthylcarbamate de 3-carbamoyl-isoxazol-5-ylméthyle
48. {2-[5'-(3-Chloro-phényl)-3,4,5,6-tétrahydro-2H-[1,2']bipyridinyl-4-yl]-éthylcarbamate de 3-carbamoyl-isoxazol-5-ylméthyle
49. {2-[5'-(3-Fluoro-5-méthoxy-phényl)-3,4,5,6-tétrahydro-2H-[1,2']bipyridinyl-4-yl]-éthylcarbamate de 3-carbamoyl-isoxazol-5-ylméthyle
50. [2-(5'-Benzo[1,3]dioxol-5-yl-3,4,5,6-tétrahydro-2H-[1,2']bipyridinyl-4-yl]-éthylcarbamate de 3-carbamoyl-isoxazol-5-ylméthyle
51. 5-[4-Fluoro-1-(4-trifluorométhyl-pyrimidin-2-yl)-pipéridin-4-ylméthylcarbamoyloxyméthyl]-isoxazole-3-carboxylate d'éthyle
52. {3-[1-(4-Trifluorométhyl-pyrimidin-2-yl)-pipéridin-4-yl]-propylcarbamate de 1-méthyl-1H-pyrazol-3-ylméthyle
53. 5-{2-[1-(6-Chloro-quinolin-2-yl)-pipéridin-4-yl]-éthylcarbamoyloxyméthyl}-isoxazole-3- acide carboxylique
54. [1-(6-Chloro-quinoxalin-2-yl)-pipéridin-4-yl]-méthylcarbamate de 5-isopropyl-[1,2,4]oxadiazol-3-ylméthyle
55. [1-(4-Trifluorométhyl-pyrimidin-2-yl)-pipéridin-4-yl]-méthylcarbamate de 5-isopropyl-[1,2,4]oxadiazol-3-ylméthyle
56. 5-[1-(6-Chloro-quinolin-2-yl)-4-fluoro-pipéridin-4-ylméthylcarbamoyloxyméthyl]-isoxazole-3-carboxylate d'éthyle
57. [4-Fluoro-1-(4-trifluorométhyl-pyrimidin-2-yl)-pipéridin-4-yl]-méthylcarbamate de 3-méthylcarbamoyl-isoxazol-5-ylméthyle
58. [1-(4-Nitro-2-trifluorométhyl-phényl)-pipéridin-4- yl]-méthylcarbamate de 5-isopropyl-[1,2,4]oxadiazol-3-ylméthyle
59.[1-(4-Chloro-phthalazin-1-yl)-pipéridin-4-yl]-méthylcarbamate de 5-isopropyl-[1,2,4]oxadiazol-3-ylméthyle
60. {2-[3-Diméthylamino-1-(4-trifluorométhyl-pyrimidin-2-yl)-azétidin-3-yl]-éthylcarbamate de 3-méthylcarbamoyl-isoxazol-5-ylméthyle
61. {2-[4-Ethyl-1-(4-trifluorométhyl-pyrimidin-2-yl)-pipéridin-4-yl]-éthylcarbamate de 3-méthylcarbamoyl-isoxazol-5-ylméthyle
62. {2-[4-Hydroxy-1-(4-trifluorométhyl-pyrimidin-2-yl)-pipéridin-4-yl]-éthylcarbamate de 3-méthylcarbamoyl-isoxazol-5-ylméthyle
63. {2-[1-(4-Chloro-phthalazin-1-yl)-3-diméthylamino-azétidin-3-yl]-éthyl}-carbamate de 2,2,2-trifluoro-1-(1-méthyl-1H-imidazol-2-yl)-éthyle
64. {2-[1-(6-Chloro-quinoxalin-2-yl)-4-éthyl-pipéridin-4-yl]-éthylcarbamate de 3-méthylcarbamoyl-isoxazol-5-ylméthyle
65. {2-[1-(6-Chloro-quinoxalin-2-yl)-4-isobutyl-pipéridin-4-yl]-éthylcarbamate de 3-méthylcarbamoyl-isoxazol-5-ylméthyle
66. {2-[4-Isobutyl-1-(4-nitro-2-trifluorométhyl-phényl)-pipéridin-4-yl]-éthyl}-carbamate de 3-méthylcarbamoyl-isoxazol-5-ylméthyle
67. (1-Isoquinolin-1-yl-pipéridin-4-ylméthyl)-carbamate de 5-isopropyl-[1,2,4]oxadiazol-3-ylméthyle
68. (2-{1-[5-(4-Fluoro-phényl)-pyrazin-2-yl]-pipéridin-4-yl}-éthyl)-carbamate de 3-carbamoyl-isoxazol-5-ylméthyle
69. (2-{1-[5-(4-Fluoro-phényl)-pyrazin-2-yl]-pipéridin-4-yl}-éthyl)-carbamate de 3-méthylcarbamoyl-isoxazol-5-ylméthyle
70. [1-(4-Trifluorométhyl-pyrimidin-2-yl)-pipéridin-4-yl]-carbamate de 3-méthylcarbamoyl-isoxazol-5-ylméthyle
71. 5-{2-[1-(6-Chloro-quinolin-2-yl)-pipéridin-4-yl]-éthylcarbamoyloxyméthyl}-isoxazole-3-carboxylate d'éthyle
72. {2-[4-Méthyl-1-(4-trifluorométhyl-pyrimidin-2-yl)-pipéridin-4-yl]-éthyl}-carbamate de 3-méthylcarbamoyl-isoxazol-5-ylméthyle
73. {2-[1-(6-Chloro-quinolin-2-yl)-pipéridin-4-yl]-éthyl}-carbamate de 2,2,2-trifluoro-1-(1-méthyl-1H-imidazol-2-yl)-éthyle
74. (+/-)-[1-(4-Trifluorométhyl-pyrimidin-2-yl)-azépan-4-yl]-carbamate de 3-carbamoyl-isoxazol-5-ylméthyle
75. [1-(4-Chloro-phthalazin-1-yl)-pipéridin-4-yl]-carbamate de 3-méthylcarbamoyl-isoxazol-5-ylméthyle
76. [1-(4-Nitro-2-trifluorométhyl-phényl)-pipéridin-4-yl]-carbamate de 3-méthylcarbamoyl-isoxazol-5-ylméthyle
77. [1-(6-Chloro-quinoxalin-2-yl)-pipéridin-4-yl]-carbamate de 3-méthylcarbamoyl-isoxazol-5-ylméthyle
78. {2-[1-(6-Chloro-quinoxalin-2-yl)-4-méthyl-pipéridin-4-yl]-éthyl}-carbamate de 3-méthylcarbamoyl-isoxazol-5-ylméthyle
79. (+/-)-[1-(4-Chloro-phthalazin-1-yl)-pyrrolidin-3-ylméthyl]-carbamate de 3-méthylcarbamoyl-isoxazol-5-ylméthyle
80. {2-[1-(6-Chloro-quinolin-2-yl)-pipéridin-4-yl]-éthyl}-carbamate de 1-furan-3-yl-3-méthyl-butyle
81. {2-[6'-(4-Fluoro-phényl)-3,4,5,6-tétrahydro-2H-[1,2']bipyridinyl-4-yl]-éthyl}-carbamate de 1-furan-3-yl-3-méthyl-butyle
82. {2-[1-(4-Trifluorométhyl-pyrimidin-2-yl)-azétidin-3-yl]-éthyl}-carbamate de 3-carbamoyl-isoxazol-5-ylméthyle
83. {2-[1-(4-Trifluorométhyl-pyrimidin-2-yl)-azétidin-3-yl]-éthyl}-carbamate de 3-méthylcarbamoyl-isoxazol-5-ylméthyle
84. (-)-[1-(4-Chloro-pyrimidin-2-yl)-pyrrolidin-3-ylméthyl]-carbamate de 3-méthylcarbamoyl-isoxazol-5-ylméthyle
85. (+)-[1-(4-Chloro-pyrimidin-2-yl)-pyrrolidin-3-ylméthyl]-carbamate de 3-méthylcarbamoyl-isoxazol-5-ylméthyle
à l'état de base ou de sel d'addition à un acide.

Les composés de formule générale (I) peuvent comporter un ou plusieurs carbones asymétriques. Ils peuvent exister sous forme d'énantiomères ou de diastéréoisomères. Les composés de formule générale (I) peuvent également exister sous forme de stéréoisomères *cis (Z)* ou *trans (E).* Ces stéréoisomères, énantiomères et diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Dans le cadre de l'invention, on entend par :
- C_{t-z} où t et z peuvent prendre les valeurs de 1 à 8, une chaîne carbonée pouvant avoir de t à z atomes de carbone, par exemple C₁₋₃ une chaîne carbonée qui peut avoir de 1 à 3 atomes de carbone ;
- alkyle, un groupe aliphatique saturé, linéaire ou ramifié; par exemple un groupe C₁₋₆-alkyle représente une chaîne carbonée de 1 à 6 atomes de carbone, linéaire ou ramifiée, plus particulièrement un méthyle, éthyle, propyle, isopropyle, butyle, isobutyle, *tert*-butyle, pentyle, hexyle ;
- alkylène, un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;
- cycloalkyle, un groupe alkyle cyclique, par exemple un groupe C₃₋₇-cycloalkyle représente un groupe carboné cyclique de 3 à 7 atomes de carbone, plus particulièrement un cyclopropyle, cyclobutyle, cyclopentyle, cyclohexyle, cycloheptyle ;
- alcoxy, un groupe -O-alkyle à chaîne aliphatique saturée, linéaire ou ramifiée ;
- thioalkyle, un groupe -S-alkyle à chaîne aliphatique saturée, linéaire ou ramifiée ;
- haloalkyle, un groupe alkyle dont un ou plusieurs atomes d'hydrogène ont été substitués par un atome d'halogène ;
- haloalcoxy, un groupe alcoxy dont un ou plusieurs atomes d'hydrogène ont été substitués par un atome d'halogène ;
- halothioalkyle, un groupe thioalkyle dont un ou plusieurs atomes d'hydrogène ont été substitués par un atome d'halogène ;
- atome d'halogène, un fluor, un chlore, un brome ou un iode.

Les composés de l'invention peuvent être préparés selon différentes méthodes, illustrées par les schémas qui suivent.

Ainsi une première méthode (schéma 1) consiste à faire réagir une amine de formule générale (II), dans laquelle A, R₁, R₂, m et n sont tels que définis dans la formule générale (I) définie ci-dessus, avec un carbonate de formule générale (III) dans laquelle Z représente un atome d'hydrogène ou un groupe nitro, R₃ et R₄ sont tels que définis dans la formule générale (I) définie ci-dessus, en présence d'une base telle que la triéthylamine, la pyridine, la *N,N-*diméthylaminopyridine ou la diisopropyléthylamine, dans un solvant tel que le toluène ou le 1,2-dichloroéthane, à une température comprise entre la température ambiante et la température de reflux du solvant.

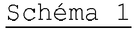

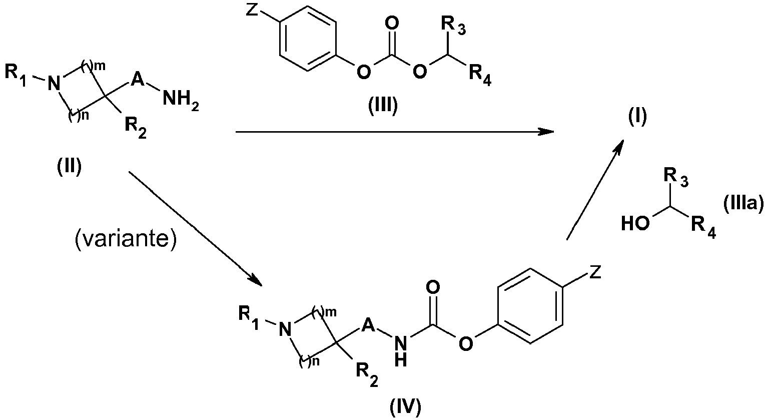

Une variante d'obtention des composés de formule générale (I) (schéma 1) consiste à faire réagir une amine de formule générale (II), telle que définie ci-dessus, avec le chloroformiate de phényle ou de 4-nitro-phényle, en présence d'une base telle que la triéthylamine ou la diisopropyléthylamine, dans un solvant tel que le dichlorométhane ou le tétrahydrofurane, à une température comprise entre 0°C et la température ambiante, pour conduire au dérivé carbamate de formule générale (IV), dans laquelle A, R₁, R₂, m et n sont tels que définis dans la formule générale (I) définie ci-dessus, et Z représente un atome d'hydrogène ou un groupe nitro. Le dérivé carbamate de formule générale (IV) ainsi obtenu est ensuite transformé en composé de formule générale (I), par action d'un alcool de formule générale HOCHR₃R₄ (IIIa), telle que définie ci-dessus, en présence d'une base telle que la triéthylamine, la pyridine, la *N,N*-diméthylaminopyridine ou la diisopropyléthylamine, dans un solvant tel que le toluène ou le dichloroéthane, à une température comprise entre la température ambiante et la température de reflux du solvant.

Une seconde méthode (schéma 2) permet l'obtention de composés de formule générale (I), dans laquelle R₁ représente un groupe R₅ substitué notamment par un groupe R₆ de type C₁₋₆-alkyle, C₃₋₇-cycloalkyle ou C₃₋₇-cycloalkyle-C₁₋₃-alkylène, ou par un groupe R₇ tel que défini dans la formule générale (I) définie ci-dessus.

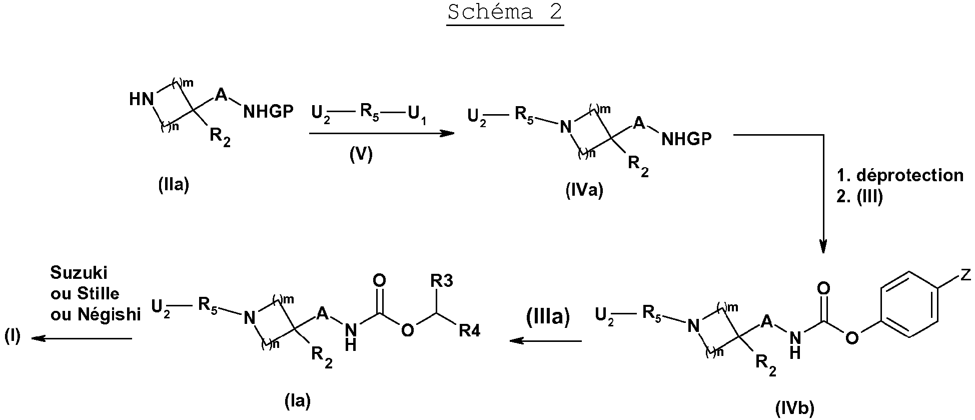

Ainsi, la première étape consiste à faire réagir une amine de formule générale (IIa), dans laquelle A, R₂, m et n sont tels que définis dans la formule générale (I) définie ci-dessus, et GP représente un groupe protecteur tel qu'un Boc *(tert-*butyloxycarbonyl), un Cbz (benzyloxycarbonyl), un benzyle ou un benzhydrile, avec un dérivé de formule générale (V), dans laquelle R₅ est tel que défini ci-dessus, U₁ représente un atome d'halogène ou un groupe O-triflate et U₂ représente un atome de chlore, de brome, d'iode ou un groupement O-triflate, en utilisant les réactions de substitution nucléophile aromatique, hétéroaromatique ou de N-arylation, *N*-hétéroarylation de Buchwald, par exemple au moyen d'un catalyseur au Palladium ou au Cuivre, pour obtenir l'intermédiaire de formule générale (IVa), dans laquelle A, R₂, R₅, m, n, U₂ et GP sont tels que définis ci-dessus. Le composé (IVa) ainsi obtenu est engagé, dans un premier temps, dans une réaction de déprotection par exemple en présence d'acide trifluoroacétique ou d'une solution d'acide chlorhydrique (5N) dans l'isopropanol ou le dioxane, suivie d'une réaction de condensation avec un carbonate de formule générale (III) telle que définie ci-dessus, dans les conditions décrites pour le schéma 1 ci-dessus, pour conduire au dérivé carbamate de formule générale (IVb), dans laquelle A, R₂, R₅, m, n, U₂ et Z sont tels que définis ci-dessus. Le composé de formule générale (IVb) ainsi obtenu est ensuite transformé en dérivé carbamate de formule générale (Ia), par action d'un alcool de formule générale HOCHR₃R₄ (IIIa), telle que définie ci-dessus, en présence d'une base telle que la triéthylamine, la pyridine, la N,N-diméthylaminopyridine ou la N,N-diisopropyléthylamine, dans un solvant tel que le toluène ou le dichloroéthane, à une température comprise entre la température ambiante et la température de reflux du solvant. La dernière étape consiste à effectuer une réaction de couplage catalysée au moyen d'un métal de transition comme le Palladium (0), sur l'intermédiaire de formule générale (Ia), dans laquelle A, R₂, R₃, R₄, R₅, m, n et U₂ sont tel que définis ci-dessus, U₂ étant dans la position où l'on souhaite introduire le groupe R₆ ou R₇ :
- soit par une réaction de type Suzuki, par exemple au moyen d'un acide boronique d'alkyle, de cycloalkyle, d'aryle ou d'hétéroaryle,
- soit selon une réaction de type Stille, par exemple en utilisant un dérivé *tri*-alkylstanneux d'aryle ou d'hétéroaryle
- soit par une réaction de type Négishi, par exemple en utilisant un dérivé zincate d'halogénure d'alkyle, de cycloalkyle, d'aryle ou d'hétéroaryle.

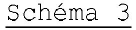

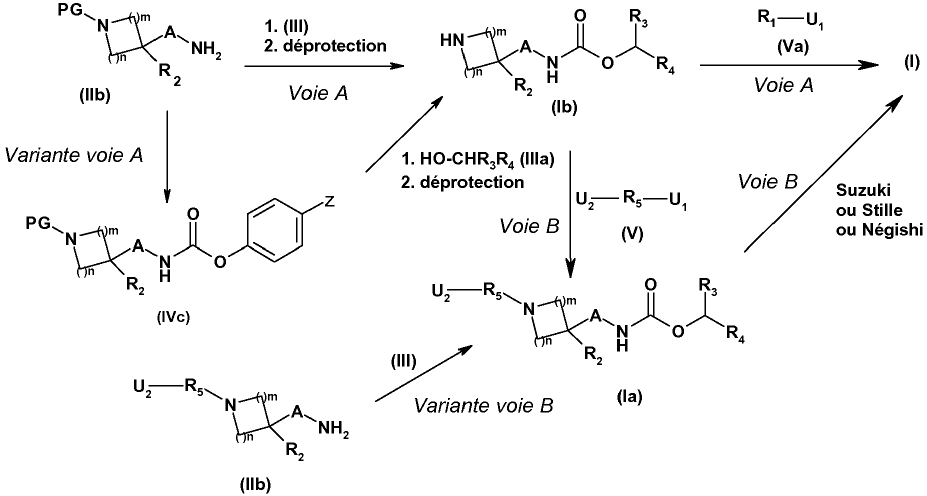

Une troisième méthode (schéma 3) consisterait à faire réagir dans un premier temps une amine de formule générale (IIb), dans laquelle A, R₂, m et n sont tels que définis dans la formule générale (I) définie ci-dessus, et PG est tel que défini ci-dessus, avec un carbonate de formule générale (III) telle que définie ci-dessus, dans les conditions décrites ci-dessus lors de la réaction de l'amine de formule générale (II) avec le carbonate de formule générale (III), suivie d'une réaction de déprotection, par exemple en présence d'une solution d'acide chlorhydrique (5N) dans l'isopropanol ou le dioxane, pour obtenir l'intermédiaire de formule générale (Ia), dans laquelle A, R₂, R₃, R₄, m et n sont tels que définis dans la formule générale (I).

Une variante d'obtention des intermédiaires de formule générale (Ib) (schéma 3, variante voie A) consisterait à faire réagir une amine de formule générale (IIa), telle que définie ci-dessus, avec le chloroformiate de phényle ou de 4-nitro-phényle, dans les conditions décrites ci-dessus lorsque le composé de formule(II) réagit avec le composé de formule (IV) (schéma 1, variante), pour conduire au dérivé carbamate de formule générale (IVc), dans laquelle A, R₂, m et n sont tels que définis dans la formule générale (I) définie ci-dessus, PG et Z sont tels que définis ci-dessus. Le dérivé carbamate de formule générale (IVc) ainsi obtenu est ensuite transformé en composé de formule générale (Ia), par action d'un alcool de formule générale HOCHR₃R₄ (IIIa), dans les conditions décrites ci-dessus lorsque le composé de formule(IV) réagit avec le composé de formule (IIIa) (schéma 1, variante).

Le composé de formule générale (I) est ensuite obtenu par réaction du composé de formule générale (Ib) avec un dérivé de formule générale (Va), dans laquelle R₁ et U₁ sont tels que défini dans la formule générale (I), en utilisant les conditions de réactions de substitution nucléophiles aromatiques ou hétéroaromatiques, par exemple au moyen d'une base telle que la triéthylamine, la diisopropyléthylamine, la pyridine ou la N,N-diméthylaminopyridine dans un solvant tel que le dichlorométhane, le dichloroéthane, l'acétonitrile, la N,N-diméthylformamide, le dioxane ou le tétrahydrofurane, à une température comprise entre 0°C et la température de reflux du solvant. Cette transformation peut être également réalisée en utilisant les conditions de N-arylation ou N-hétéroarylation de Buchwald, par exemple au moyen d'un catalyseur au Palladium ou au Cuivre.

Selon le schéma 3 voie B, les composés de formule générale (I), dans laquelle R₁ représente un groupe R₅ substitué notamment par un groupe R₆ de type C₁₋₆-alkyle, C₃₋₇-cycloalkyle ou C₃₋₇-cycloalkyle-C₁₋₃-alkylène, ou par un groupe R₇ tel que défini dans la formule générale (I) définie ci-dessus, peuvent être également préparés selon une réaction de couplage, catalysée au moyen d'un métal de transition, par exemple le Palladium (0), réalisée sur le composé de formule générale (Ia), dans laquelle A, R₂, R₃, R₄, R₅, m, n, o et p sont tels que définis dans la formule générale (I) et U₂ représente un atome de chlore, de brome, d'iode ou un groupement triflate, U₂ étant dans la position où l'on souhaite introduire le groupe R₆ ou R₇ (schéma 3, voie B) selon les conditions réactionnelles employées pour transformer le composé de formule (Ia) en composé de formule (I) (voir schéma 2).

L'intermédiaire de formule générale (Ia) telle que définie ci-dessus est préalablement obtenu en faisant réagir une amine de formule générale (Ib) telle que définie ci-dessus avec un dérivé de formule générale (Vb) dans laquelle R₅, U₁ et U₂ sont tels que définis ci-dessus selon les conditions décrites ci-dessus au schéma 2 lorsque le composé de formule (IIa) réagit avec le composé de formule (V) pour donner le composé de formule (IVa).

Une variante d'obtention des intermédiaires de formule générale (Ia) (schéma 3, variante voie B) consisterait à faire réagir dans un premier temps une amine de formule générale (IIb), dans laquelle A, R₅, R₂, m et n sont tels que définis dans la formule générale (I) définie ci-dessus, et U₂ est tel que défini ci-dessus, avec un carbonate de formule générale (III) telle que définie ci-dessus, dans les conditions décrites ci-dessus lors de la réaction de l'amine de formule générale (II) avec le carbonate de formule générale (III), pour obtenir l'intermédiaire de formule générale (Ia), dans laquelle A, R₅, R₂, R₃, R₄, m et n sont tels que définis dans la formule générale (I), et U₂ est tel que défini ci-dessus.

La présente invention décrit les intermédiaires de formule (Ib) suivants :
- Chlorydrate de pyrrolidin-3-ylméthyl-carbamate de 3-méthylcarbamoyl-isoxazol-5-ylméthyle
   PF(°C) : 187-189, LC-MS : M+H = 283
   RMN ¹H (DMSO) δ (ppm) : 8,70 (sl, 1H) ; 8,00 (m, 2H); 6,80 (m, 1H); 5,25 (s, 2H); 3,60 (m, 1H); 3,45 (m, 1H); 3,30 (m, 1H); 3,10 (m, 1H); 2,90 (m, 2H); 2,80 (s, 3H); 2,50 (m, 1H); 2,05 (m, 1H); 1,70 (m, 1H)
- [2-(4-Méthyl-pipéridin-4-yl)-éthyl]-carbamate de 3-méthylcarbamoyl-isoxazol-5-ylméthyle
   PF(°C) : 188-190, LC-MS : M+H = 361
   RMN ¹H (DMSO) δ (ppm) : 8,70 (large s, 1H) ; 8,50 (large s, 1H) ; 7,45 (large s, 1H) ; 6,80 (s, 1H) ; 5,20 (s, 2H) ; 3,00 (m, 6H) ; 2,75 (d, 3H); 1,60-1,40 (m, 6H) ; 0,95 (m, 3H)
- [2-(4-Isobutyl-pipéridin-4-yl)-éthyl]-carbamate de 3-méthylcarbamoyl-isoxazol-5-ylméthyle
   LC-MS : M+H = 367
   RMN ¹H (DMSO) δ (ppm) : 8,70 (large s, 1H); 8,50 (large s, 1H) ; 7, 40 (m, 1H) ; 6,80 (s, 1H) ; 5,20 (s, 2H) ; 3,00 (m, 6H) ; 2,75 (d, 3H) ; 1,70 (m, 1H) ; 1,50 (m, 6H) ; 1,30 (m, 2H) ; 0,90 (d, 6H)
- Chlorydrate de [2-(4-éthyl-pipéridin-4-yl)-éthyl]-carbamate de 3-méthylcarbamoyl-isoxazol-5-ylméthyle
   PF(°C) : 222-224, LC-MS : M+H = 339 RMN ¹H (DMSO) δ (ppm) : 8,70 (large s, 2H) ; 7,40 (t, 1H) ; 6,80 (s, 1H) ; 5,20 (s, 2H) ; 2,95 (m, 6H) ; 2,75 (d, 3H) ; 1,55 (q, 2H) ; 1,45 (t, 2H) ; 1,35 (m, 4H) ; 0,80 (t, 3H).
- Pipéridin-4-yl-carbamate de 3-méthylcarbamoyl-isoxazol-5-ylméthyle
   LC-MS : M+H = 283
   RMN ¹H (DMSO) δ (ppm) : 8,80 (sl, 1H); 8,70 (m, 1H); 7,75 (m, 1H); 6,80 (s, 1H); 5,25 (s, 1H); 3,65 (m, 1H); 3,25 (m, 2H); 3,00 (m, 2H); 2,80 (d, 3H); 1,95 (m, 2H); 1,70 (m, 2H).
- Pipéridin-4-ylméthyl-carbamate de 5-isopropyl-[1,2,4]oxadiazol-3-ylméthyle
   LC-MS : M+H = 282
   RMN ¹H (DMSO) δ (ppm) : 7,25 (tl, 1H); 4,90 (s, 2H); 2,80 (m, 2H); 2,70 (m, 2H); 2,30 (m, 2H); 1,40 (m, 2H); 1,30 (m, 1H); 1,10 (d, 6H); 0,85 (m, 2H).
- Chlorhydrate de [2-(3-diméthylamino-azétidin-3-yl)-éthyl]-carbamate de 2,2,2-trifluoro-1-(1-méthyl-1H-imidazol-2-yl)-éthyle
   RMN ¹H (DMSO) δ (ppm) : 12,50 (large s, 1H) ; 10,00 (large s, 1H) ; 9,20 (large s, 1H) ; 8,30 (t, 1H) ; 7,60 (s, 1H) ; 7,30 (s, 1H) ; 6,80 (m, 1H) ; 4,50 (m, 2H) ; 4,10 (m, 2H) ; 3,90 (s, 3H); 3,40 (m, 2H) ; 2,70 (s, 6H) ; 2,15 (m, 2H)
- Chlorhydrate de [2-(3-diméthylamino-azétidin-3-yl)-éthyl]-carbamate de 3-méthylcarbamoyl-isoxazol-5-ylméthyle
   PF(°C) : 210-212°C
   RMN ¹H (DMSO) δ (ppm) : 12,50 (large s, 1H) ; 9,80 (large s, 1H) ; 9,20 (large s, 1H) ; 8,80(large s, 1H) ; 7,80 (large s, 1H) ; 6,80 (s, 1H) ; 5,20 (s, 2H) ; 4,50 (m, 2H) ; 4,10 (m, 2H) ; 3,40 (m, 2H) ; 2,80 (s, 3H) ; 2,55 (s, 6H) ; 2,10 (t, 2H)
- Trifluoroacétate de 5-(4-fluoro-pipéridin-4-ylméthylcarbamoyloxyméthyl)-isoxazole-3-carboxylate d'éthyle
- (3-Pipéridin-4-yl-propyl)-carbamate de 1-méthyl-1H-pyrazol-3-ylméthyle
   LC-MS : M+H = 281
   RMN ¹H (DMSO) δ (ppm) : 9,10 (sl, 1H) ; 8,85 (sl, 1H) ; 7,65 (s, 1H); 7,15 (sl, 1H); 6,20 (s, 1H); 4,90 (s, 2H); 3,80 (s, 3H) ; 3,20 (m, 2H); 3,00 (m, 2H); 2,80 (m, 2H); 1,80 (m, 2H); 1,50-1,20 (m, 6H).
- Azépan-4-yl-carbamate de 3-carbamoyl-isoxazol-5-ylméthyle.

La présente invention décrit les intermédiaires de formule (II) suivants :
- [1-(4-Trifluorométhyl-pyrimidin-2-yl)-pyrrolidin-3-yl]-méthylamine
- 4-(2-Amino-éthyl)-1-(4-trifluorométhyl-pyrimidin-2-yl)-pipéridin-4-ol
   RMN ¹H (CDCl₃) δ (ppm) : 8,40 (d, 1H) ; 6,60 (d, 1H) ; 4,50 (m, 2H) ; 3,50-3,20 (m, 2H) ; 3,20 (m, 2H) ; 2,90-2,60 (large s, 2H) ; 1,70 (m, 2H) ; 1,50-1,30 (m, 4H).
- 2-[5'-(3-Fluoro-phényl)-3,4,5,6-tétrahydro-2H-[1,2']bipyridinyl-4-yl]-éthylamine
- 2-[1-(4'-Fluoro-biphényl-4-yl)-pipéridin-4-yl]-éthylamine
   LC-MS : M+H = 299
   RMN ¹H (DMSO) δ (ppm): 7,60 (m, 2H) ; 7,50 (d, 2H) ; 7,25 (m, 2H) ; 7,00 (d, 2H) ; 3,75 (m, 2H) ; 2,85 (m, 2H) ; 2,75 (m, 2H) ; 1,70 (m, 2H) ; 1,50 (m, 1H) ; 1,35 (m, 2H) ; 1,25 (m, 2H)
- 2-(5'-Isobutyl-3,4,5,6-tétrahydro-2H-[1,2']bipyridinyl-4-yl)-éthylamine
   LC-MS : M+H = 262
   RMN ¹H (CDCl₃) δ (ppm) : 8,00 (m, 1H) ; 7,30 (m, 1H) ; 6,65 (d, 1H) ; 4,25 (m, 2H) ; 2,80 (m, 4H) ; 2,35 (d, 2H) ; 1,80 (m, 3H) ; 1,60 (m, 1H) ; 1,45 (m, 2H) ; 1,30 (m, 4H) ; 0,90 (d, 6H)
- 2-{1-,[6-(4-Fluoro-phényl)-pyridazin-3-yl]-pipéridin-4-yl}-éthylamine
- 2-[5'-(2,2-Diméthyl-propyl)-3,4,5,6-tétrahydro-2H-[1,2']bipyridinyl-4-yl]-éthylamine
- 2-[1-(4-Trifluorométhyl-pyrimidin-2-yl)-pipéridin-4-yl]-éthylamine
- 2-{1-[6-(4-Fluoro-phényl)-pyrazin-2-yl]-pipéridin-4-yl}-éthylamine
- 2-{1-[5-(4-Fluoro-phényl)-pyrimidin-2-yl]-pipéridin-4-yl}-éthylamine
- 2-[1-(4-Trifluorométhyl-pyrimidin-2-yl)-azétidin-3-yl]-éthylamine
   LC-MS ; M+H = 247
   RMN ¹H (CDCl3) δ (ppm) : 8,50 (d, 1H) ; 6,80 (d, 1H) ; 4,30 (m, 2H) ; 3,90 (m, 2H) ; 2,85 (m, 1H) ; 2,75 (m, 2H) ; 1,85 (m, 2H) ; 1,30 (sl, 2H).

La présente invention décrit les intermédiaires de formule (IIa) suivants :
- 4-(2-Amino-éthyl)-4-éthyl-pipéridine-1-carboxylate de *tert*-butyle
- 4-(2-Amino-éthyl)-4-isobutyl-pipéridine-1-carboxylate de *tert*-butyle
- 3-(2-Amino-éthyl)-3-diméthylamino-azétidine-1-carboxylate de *tert*-butyle

La présente invention décrit les intermédiaires de formule (IV) suivants :
- {2-[5'-(4-Fluoro-phényl)-3,4,5,6-tétrahydro-2H-[1,2']bipyridinyl-4-yl]-éhyl}-carbamate de nitro-phényle
- {2-[5'-(3-Fluoro-phényl)-3,4,5,6-tétrahydro-2H-[1,2']bipyridinyl-4-yl]-éthyl}-carbamate de 4-nitro-phényle
- [2-(5'-Bromo-3,4,5,6-tétrahydro-2H-[1,2']bipyridinyl-4-yl)-éthyl]-carbamate de 4-nitro-phényle
- {2-[1-(4-Trifluorométhyl-pyrimidin-2-yl)-pipéridin-4-yl]-éthyl}-carbamate de 4-nitro-phényle
- (2-{1-[6-(4-Fluoro-phényl)-pyrazin-2-yl]-pipéridin-4-yl}-éthyl)-carbamate de 4-nitro-phényle
- (2-{1-[5-(4-Fluoro-phényl)-pyrimidin-2-yl]-pipéridin-4-yl}-éthyl)-carbamate de 4-nitro-phényle
- [2-(5'-Bromo-3,4,5,6-tétrahydro-2H-[1,2']bipyridinyl-4-yl)-éthylcarbamate de 4-nitro-phényle.

Les autres composés de formules générales (II), (IIa), (III), (IIIa) et (V) ainsi que les autres réactifs sont disponibles dans le commerce ou décrits dans la littérature, ou bien peuvent être préparés selon des méthodes qui y sont décrites ou qui sont connues de l'homme du métier.

Notamment, le carbonate de formule générale (III) peut être préparé selon toute méthode décrite dans la littérature, par exemple par réaction d'un alcool de formule générale HOCHR₃R₄ (IIIa),dans laquelle R₃ et R₄ sont tels que définis dans la formule générale (I) telle que définie ci-dessus, avec le chloroformiate de phényle ou de 4-nitrophényle, en présence d'une base telle que la triéthylamine, la N-méthylmorpholine ou la diisopropyléthylamine, dans un solvant tel que le dichlorométhane ou le tétrahydrofurane, à une température comprise entre 0°C et la température ambiante.

Les exemples qui suivent illustrent la préparation de quelques composés de l'invention. Ces exemples ne sont pas limitatifs et ne font qu'illustrer l'invention. Les microanalyses, les spectres I.R. et R.M.N. et/ou la LC-MS (Liquid Chromatography coupled to Mass Spectroscopy) confirment les structures et les puretés des composés obtenus. Méthode A :UPLC / TOF - Gradient 3 min - H2O / ACN / TFA T0 : 98%A - T1, 6 à T2, 1min : 100%B - T2,5 à T3min : 98%A Voie A : H2O + 0,05%TFA ; Voie B : ACN + 0,035%TFA Débit : 1.0mL/min-T°=40°C - Injection 2µL Colonne Acquity BEH C18 (50*2,1mm ; 1,7µm); 220 nm.

Méthode B : HPLC / ZQ - Gradient 10 min - CH3COONH4 5mM / ACN T0 : 100%A - T5, 5 à T7min : 100%B - T7, 1 à T10min : 100%A Voie A : CH3COONH4 + 3%ACN ; Voie B : ACN Débit : 0.8mL/min-T°=40°C - Injection 5µL Colonne Kromasil C18 (50*2,1mm ; 3,5µm); 220 nm.

PF(°C) représente le point de fusion en degrés Celsius.

Les numéros indiqués entre parenthèses dans les titres des exemples correspondent à ceux de la 1ère colonne du tableau ci-après.

La nomenclature UICPA (Union Internationale de Chimie Pure et Appliquée - IUPAC en anglais) a été utilisée pour la dénomination des composés dans les exemples ci-dessous.

### Exemple 1 (Composé N°9)

### 2-[5'-(4-Fluoro-phényl)-3,4,5,6-tétrahydro-2H-[1,2']bipyridinyl-4-yl]-éthylcarbamate de [1,2,3]thiadiazol-4-ylméthyle

### 1.1. 2-(5'-Bromo-3,4,5,6-tétrahydro-2H-[1,2']bipyridinyl-4-yl)-éthanol

Dans un autoclave, on introduit 11,00 g (46,43 mmoles) de 2,5-dibromopyridine, 6,00 g (46,43 mmoles) de pipéridin-4-yl-éthanol et 6,74 g (48,76 mmoles) de carbonate de potassium dans 8 ml de DMSO. On chauffe ensuite à 160°C pendant 20 heures.

On laisse revenir à température ambiante puis on reprend le mélange réactionnel par de l'acétate d'éthyle et de l'eau. On sépare la phase aqueuse, on l'extrait deux fois avec de l'acétate d'éthyle, on lave les phases organiques réunies avec une solution aqueuse saturée en chlorure de sodium, on les sèche sur sulfate de sodium et on concentre le filtrat sous pression réduite.

On obtient ainsi 11,00 g de produit sous forme d'huile utilisé tel quel dans l'étape suivante.

### 1.2. 2-[5'-(4-Fluoro-phényl)-3,4,5,6-tétrahydro-2H-[1,2']bipyridinyl-4-yl]-éthanol

Sous atmosphère inerte, on introduit 3,60 g (12,62 mmoles) de 2-(5'-bromo-3,4,5,6-tétrahydro-2H-[1,2']bipyridinyl-4-yl)-éthanol, préparé à l'étape 1.1., 3,53 g (25,25 mmoles) de l'acide 4-fluorophénylboronique, 5,23 g (37,87 mmoles) de carbonate de potassium et 4,88 g (15,15 mmoles) de bromure de tétrabutylammonium en suspension dans 20 ml d'eau. On ajoute ensuite 0,142 g (0,63 mmole) de Pd(OAc)₂. On porte le mélange réactionnel au reflux pendant 24 heures.

On laisse revenir à température ambiante, on sépare les sels par filtration sur célite, puis on reprend le filtrat avec de l'acétate d'éthyle, on sépare la phase aqueuse, on l'extrait deux fois avec de l'acétate d'éthyle, on lave les phases organiques réunies avec une solution aqueuse saturée en chlorure de sodium et on les sèche sur sulfate de sodium. Après évaporation du solvant, le résidu obtenu est purifié par chromatographie sur gel de silice en éluant avec un mélange 50/50 d'acétate d'éthyle et de cyclohexane.

On obtient ainsi 1,6 g de produit sous forme de poudre blanche.
PF (°C) = 118-120°C

### 1.3. 2-{2-[5'-(4-Fluoro-phényl)-3,4,5,6-tétrahydro-2H-[1,2']bipyridinyl-4-yl]-éthyl}-isoindole-1,3-dione

A une solution de 2,00 g (6,66 mmoles) de 2-[5'-(4-fluoro-phényl)-3,4,5,6-tétrahydro-2H-[1,2']bipyridinyl-4-yl]-éthanol, préparé à l'étape 1.2., de 2,096 g (7,99 mmoles) de triphénylphosphine et de 1,077 g (7,32 mmoles) de phtalimide dans 40 ml de tétrahydrofurane, refroidie à environ -2°C, on ajoute goutte à goutte sous atmosphère inerte, une solution de 1,61 g (7,99 mmoles) de diisopropylazodicarboxylate (DIAD) dans 4 ml de tétrahydrofurane tout en maintenant la température du milieu réactionnel entre -2°C et 0°C. On poursuit l'agitation à 0°C pendant 1 heure, puis à température ambiante pendant 12 heures.

On concentre sous pression réduite, on reprend le résidu avec du dichlorométhane et de l'eau. On sépare la phase aqueuse puis on l'extrait 2 fois avec du dichlorométhane. On réunit les phases organiques et on les lave successivement avec une solution aqueuse d'acide chlorhydrique (1N), puis une solution aqueuse saturée d'hydrogénocarbonate de sodium et une solution aqueuse saturée en chlorure de sodium. On sèche la phase organique sur sulfate de sodium et on concentre le filtrat sous pression réduite. On purifie le résidu ainsi obtenu par chromatographie sur gel de silice en éluant avec un mélange 20/80 d'acétate d'éthyle et de cyclohexane.

On obtient ainsi 2,1 g du produit attendu sous forme de poudre blanche.
PF (°C) = 180-182°C

### 1.4. 2-[5'-(4-Fluoro-phényl)-3,4,5,6-tétrahydro-2H-[1,2']bipyridinyl-4-yl]-éthylamine

A une solution de 1,3 g (3,03 mmoles) de 2-{2-[5'-(4-fluoro-phényl)-3,4,5,6-tétrahydro-2H-[1,2']bipyridinyl-4-yl]-éthyl}-isoindole-1,3-dione, préparé à l'étape 1.3. dans 30 ml d'éthanol, on ajoute lentement à température ambiante 0,485 g (15,13 mmoles) d'hydrazine monohydrate. On porte ensuite le mélange réactionnel au reflux pendant trois heures.

On laisse revenir à température ambiante, on sépare l'insoluble par filtration et on concentre le filtrat sous pression réduite. On reprend le résidu par 20 ml d'éther et on laisse sous agitation à température ambiante pendant une heure. On sépare de nouveau l'insoluble et on concentre le filtrat sous pression réduite.

On obtient ainsi 0,70 g du produit attendu sous forme de poudre blanche.
PF (°C) = 88-94°C
RMN ¹H (CDCl₃) δ (ppm) : 8,3 (d, 1H) ; 7,55 (dd, 1H) ; 7,35 (m, 2H) ; 7,05 (d, 1H) ; 7,1 (d, 1H) ; 6,65 (d, 1H) ; 4,25 (large d, 2H) ; 3,0-2,8 (m, 4H) ; 1,8 (m, 2H) ; 1,6-1,1 (m, 5H).

### 1.5. 2-[5'-(4-Fluoro-phényl)-3,4,5,6-tétrahydro-2H-[1,2']bipyridinyl-4-yl]-éthylcarbamate de 4-nitro-phényle

A une solution de 5 g (16,7 mmoles) de 2-[5'-(4-fluoro-phényl)-3,4,5,6-tétrahydro-2H-[1,2']bipyridinyl-4-yl]-éthylamine, préparé à l'étape 1.4., de 4,32 g (33,40 mmoles) de N,N-diisopropyléthylamine et de 0,10 g (0,84 mmole) de *N,N*-diméthylaminopyridine dans 50 ml de dichlorométhane, refroidie à environ 0°C, on ajoute par petites portions 3,7 g (18,37 mmoles) de chloroformiate de 4-nitrophényle. On poursuit l'agitation à 0°C pendant 1 heure puis à température ambiante pendant 2 heures.

On ajoute de l'eau au milieu réactionnel, on sépare la phase aqueuse, on l'extrait plusieurs fois avec du dichlorométhane, on lave les phases organiques réunies avec une solution aqueuse saturée en chlorure de sodium, on les sèche sur sulfate de sodium et on concentre le filtrat sous pression réduite.

On obtient ainsi 4,6 g de produit sous forme de solide beige amorphe utilisé tel quel dans l'étape suivante.
LC-MS : M+H = 465
RMN ¹H (DMSO) δ (ppm) : 8,40 (s, 1H); 8,30 (d, 2H); 8,10 (tl, 1H); 7,80 (m, 1H); 7,70 (m, 2H); 7,45 (d, 2H); 7,25 (m, 2H); 6,90 (d, 1H); 4,35 (m, 2H); 3,20 (m, 2H); 2,80 (m, 2H); 1,80 (m, 2H); 1,65 (m, 1H); 1,50 (m, 2H); 1,20 (m, 2H).

### 1.6. 2-[5'-(4-Fluoro-phényl)-3,4,5,6-tétrahydro-2H-[1,2']bipyridinyl-4-yl]-éthylcarbamate de [1,2,3]thiadiazol-4-ylméthyle

On chauffe dans un tube réacteur à 80°C pendant 12 heures, une solution de 0,50 g (0,50 mmole) de 2-[5'-(4-fluoro-phényl)-3,4,5,6-tétrahydro-2H-[1,2']bipyridinyl-4-yl]-éthylcarbamate de 4-nitro-phényle, préparé à l'étape 1.5., de 0,128 g (0,99 mmole) de N,N-diisopropyléthylamine, de 0,030 g (0,25 mmole) de N,N-diméthylaminopyridine et de 0,079 g (0,5 mmole) de [1,2,3]thiadiazol-4-ylméthanol (Acta Pharmaceutica Suecica (1973), 10(4), 285-96) dans 5 ml de 1,2-dichloroéthane.

On laisse revenir à température ambiante. On reprend le résidu par du dichlorométhane et une solution aqueuse de soude 1N, on sépare la phase aqueuse, on l'extrait deux fois avec du dichlorométhane, on lave successivement les phases organiques réunies avec une solution aqueuse de soude 1N puis une solution aqueuse saturée en chlorure de sodium et on les sèche sur sulfate de sodium. Après évaporation du solvant, on purifie le résidu obtenu par chromatographie sur gel de silice en éluant avec un mélange 99/1/0,1 de dichlorométhane, de méthanol et d'ammoniaque à 28%.

On obtient ainsi 0,23 g de produit pur sous forme de poudre blanche.
PF(°C) : 139-141°C ; LC-MS : M+H = 442
RMN ¹H (DMSO) δ (ppm) : 9,15 (s, 1H) ; 8,4 (s, 1H) ; 7,8 (d, 1H) ; 7,7 (dd, 2H) ; 7,40 (large t, 1H) ; 7,25 (t, 2H) ; 6,90 (d, 1H) ; 5,5 (s, 2H) ; 4,35 (large d, 2H) ; 3,1 (m, 2H) ; 2,85 (large t, 2H) ; 1,75 (large d, 2H) ; 1,55 (m, 1H) ; 1,4 (m, 2H) ; 1,15 (m, 2H).

### Exemple 2 (Composé N'5)

### 2-[5'-(4-Fluoro-phényl)-3,4,5,6-tétrahydro-2H-[1,2']bipyridinyl-4-yl]-éthylcarbamate de 3-éthyl-[1,2,4]oxadiazol-5-ylméthyle

On procède suivant le mode opératoire décrit dans l'exemple 1 (étape 1.6.). A partir de 0,23 g (0,5 mmole) de 2-[5'-(4-fluoro-phényl)-3,4,5,6-tétrahydro-2H-[1,2']bipyridinyl-4-yl]-éthylcarbamate de 4-nitro-phényle, décrit dans l'exemple 1 (étape 1.5.), de 0,128 g (0,99 mmole) de *N,N-*diisopropyléthylamine, de 0,030 g (0,25 mmole) de *N,N-*diméthylaminopyridine, de 0,067 g (0,50 mmole) de 3-éthyl-[1,2,4]oxadiazol-5-ylméthanol , et après chromatographie sur gel de silice en éluant avec un mélange 99/1/0,1 de dichlorométhane, de méthanol et d'ammoniaque à 28%, on obtient 0,138 g de produit pur sous forme de poudre blanche.
PF(°C) : 110-112°C, LC-MS : M+H = 454
RMN ¹H (DMSO) δ (ppm) : 8,40 (s, 1H) ; 7,8 (d, 1H) ; 7,7 (dd, 2H) ; 7,65 (large t, 1H) ; 7,30 (t, 2H) ; 6,90 (d, 1H) ; 5,30 (s, 2H) ; 4,35 (large d, 2H) ; 3,1 (m, 2H) ; 2,85 (large t, 2H) ; 2,75 (q, 2H) ; 1,75 (large d, 2H) ; 1,60 (m, 1H) ; 1,45 (m, 2H) ; 1,25 (t, 3H) ; 1,15 (m, 2H).

### Exemple 3 (Composé N°26)

### 2-[5'-(4-Fluoro-phényl)-3,4,5,6-tétrahydro-2H-[1,2']bipyridinyl-4-yl]-éthylcarbamate de 3-méthylcarbamoyl-isoxazol-5-ylméthyle

On procède suivant le mode opératoire décrit dans l'exemple 1 (étape 1.6.). A partir de 0,20 g (0,43 mmole) de 2-[5'-(4-fluoro-phényl)-3,4,5,6-tétrahydro-2*H*-[1,2']bipyridinyl-4-yl]-éthylcarbamate de 4-nitro-phényle, décrit dans l'exemple 1 (étape 1.5.), de 0,122 g (0,95 mmole) de *N,N-*diisopropyléthylamine, de 0,026 g (0,22 mmole) de *N,N-*diméthylaminopyridine, de 0,074 g (0,47 mmole) de 3-méthylcarbamoyl-isoxazol-5-ylméthanol, et après chromatographie sur gel de silice en éluant avec un mélange 98/2/0,2 de dichlorométhane, de méthanol et d'ammoniaque à 28%, on obtient 0,170 g de produit pur sous forme de poudre blanche.
PF(°C) : 191-193°C, LC-MS : M+H = 482
RMN ¹H (DMSO) δ (ppm) : 8,7 (large s, 1H) ; 8,40 (s, 1H) ; 7,85 (d, 1H) ; 7,65 (dd, 2H) ; 7,45 (large t, 1H) ; 7,30 (t, 2H) ; 6,90 (d, 1H) ; 6,8 (s, 1H) ; 5,20 (s, 2H) ; 4,35 (large d, 2H) ; 3,10 (m, 2H) ; 2,80 (m, 5H) ; 1,75 (large d, 2H) ; 1,60 (m, 1H) ; 1,40 (m, 2H) ; 1,15 (m, 2H).

### Exemple 4 (Composé N°19)

### 2-[5'-(4-Fluoro-phényl)-3,4,5,6-tétrahydro-2H-[1,2']bipyridinyl-4-yl]-éthylcarbamate de 3-carbamoyl-isoxazol-5-ylméthyle

On chauffe dans un tube scellé à 90°C pendant 12 heures, une solution de 0,25 g (0,54 mmole) de 2-[5'-(4-fluoro-phényl)-3,4,5,6-tétrahydro-2H-[1,2']bipyridinyl-4-yl]-éthylcarbamate de 4-nitro-phényle, préparé à l'étape 1.5. , de 0,139 g (1,08 mmoles) de N,N-diisopropyléthylamine, de 0,033 g (0,27 mmole) de N,N-diméthylaminopyridine et de 0,084 g (0,59 mmole) de 3-carbamoyl-isoxazol-5-ylméthanol dans 5 ml de 1,2-dichloroéthane.

On laisse revenir à température ambiante. Le précipité formé dans le milieu réactionnel est filtré sur frité puis rincé abondamment à l'éther et à l'eau. Le solide est ensuite séché sous vide à environ 80°C pendant la nuit.

On obtient ainsi 0,202 g de produit pur sous forme de poudre blanche.
PF(°C) : 202-204°C, LC-MS : M+H = 468
RMN ¹H (DMSO) δ (ppm) : 8,45 (s, 1H) ; 8,15 (large s, 1H) ; 7,85 (m, 2H) ; 7,70 (dd, 2H) ; 7,45 (large t, 1H) ; 7,30 (t, 2H) ; 6,90 (d, 1H) ; 6,8 (s, 1H) ; 5,20 (s, 2H) ; 4,40 (large d, 2H) ; 3,10 (m, 2H) ; 2,85 (large t, 2H) ; 1,80 (large d, 2H) ; 1,60 (m, 1H) ; 1,40 (m, 2H) ; 1,15 (m, 2H).

### Exemple 5 (Composé N°21)

### 2-[5'-(4-Fluoro-phényl)-3,4,5,6-tétrahydro-2H-[1,2']bipyridinyl-4-yl]-éthylcarbamate de 4-carbamoyl-oxazol-2-ylméthyle

### 5.1. 2-Acétoxyméthyl-oxazole-4-carboxylate de méthyle

A une solution de 1,2 g (4,20 mmoles) de 2-bromométhyl-oxazole-4-carboxylate de méthyle (US2005215577) dans 42 ml d'acétonitrile, on ajoute à température ambiante 0,453 g (4,62 mmoles) d'acétate de potassium puis on poursuit l'agitation à température ambiante pendant 12 heures.

Après concentration sous pression réduite, le résidu est repris dans de dichlorométhane et de l'eau. On sépare la phase aqueuse puis on l'extrait 2 fois avec du dichlorométhane. On réunit les phases organiques et on les lave avec une solution aqueuse saturée en chlorure de sodium. On sèche la phase organique sur sulfate de sodium et on concentre le filtrat sous pression réduite.

On obtient ainsi 1,1 g du produit attendu sous forme d'huile utilisé tel quel dans l'étape suivante.

### 5.2. 4-Carbamoyl-oxazol-2-ylméthanol

Dans un ballon contenant 0,60 g (3,01 mmoles) de 2-acétoxyméthyl-oxazole-4-carboxylate de méthyle, préparé à l'étape 5.1., est additionné 20 ml (352 mmoles) d'ammoniaque aqueux à 28% puis le milieu réactionnel est agité à température ambiante pendant 24 heures.

Après concentration sous pression réduite, le résidu obtenu est purifié par chromatographie sur gel de silice en éluant avec un mélange 90/10/01 de dichlorométhane, de méthanol et d'ammoniaque à 28%.

On obtient ainsi 0,230 g de produit pur sous forme de poudre blanche.
PF(°C) : 148-150°C

### 5.3. 2-[5'-(4-Fluoro-phényl)-3,4,5,6-tétrahydro-2H-[1,2']bipyridinyl-4-yl]-éthylcarbamate de 4-carbamoyl-oxazol-2-ylméthyle

On procède suivant le mode opératoire décrit dans l'exemple 4. A partir de 0,25 g (0,54 mmole) de 2-[5'-(4-fluoro-phényl)-3,4,5,6-tétrahydro-2*H*-[1,2']bipyridinyl-4-yl]-éthylcarbamate de 4-nitro-phényle, décrit dans l'exemple 1 (étape 1.5.), de 0,139 g (1,08 mmoles) de *N,N-*diisopropyléthylamine, de 0,033 g (0,27 mmole) de *N,N-*diméthylaminopyridine, de 0,084 g (0,59 mmole) de 4-carbamoyl-oxazol-2-ylméthanol, préparé à l'étape 5.2., on obtient 0,162 g de produit pur sous forme de poudre blanche.
PF(°C) : 206-208°C, LC-MS : M+H = 468
RMN ¹H (DMSO) δ (ppm) : 8,60 (s, 1H) ; 8,40 (s, 1H) ; 7,80 (dd, 1H) ; 7,65 (m, 3H) ; 7,45 (m, 2H) ; 7,25 (t, 2H) ; 6,85 (d, 1H) ; 5,10 (s, 2H) ; 4,30 (large d, 2H) ; 3,10 (m, 2H) ; 2,75 (large t, 2H) ; 1,75 (large d, 2H) ; 1,55 (m, 1H) ; 1,35 (m, 2H) ; 1,10 (m, 2H).

### Exemple 6 (Composé N°1)

### 2-[1-(6-Chloro-quinolin-2-yl)-pipéridin-4-yl]-éthylcarbamate de 3-carbamoyl-isoxazol-5-ylméthyle

### 6.1. Méthanesulfonate de 2-[1-(6-chloro-quinolin-2-yl)-pipéridin-4-yl]-éthyle

A une solution de 4,00 g (13,76 mmoles) de 2-[1-(6-chloro-quinolin-2-yl)-pipéridin-4-yl]-éthanol (WO2004/099176), de 3,55 g (27,51 mmoles) de *N,N*-diisopropyléthylamine et de 0,84 g (6,88 mmoles) de *N,N*-diméthylaminopyridine dans 30 ml de dichlorométhane, refroidie à environ 0°C, on ajoute goutte à goutte, sous atmosphère inerte, une solution de 2,36 g (20,63 mmoles) de chlorure de mésyle dans 3 ml de dichlorométhane. On poursuit l'agitation à 0°C pendant deux heures puis à température ambiante pendant une heure.

On ajoute de l'eau au milieu réactionnel, on sépare la phase aqueuse, on l'extrait plusieurs fois avec du dichlorométhane, on lave les phases organiques réunies avec une solution aqueuse saturée en chlorure de sodium, on les sèche sur sulfate de sodium et on concentre le filtrat sous pression réduite.

On obtient ainsi 5,1 g de produit sous forme d'huile utilisé tel quel dans l'étape suivante.

### 6.2. 2-[4-(2-Azido-éthyl)-pipéridin-1-yl]-6-chloro-quinoline

On porte au reflux pendant 4 heures, sous atmosphère inerte, une solution de 5 g (13,55 mmoles) de méthanesulfonate de 2-[1-(6-chloro-quinolin-2-yl)-pipéridin-4-yl]-éthyle, préparé à l'étape 6.1. et de 1,76 g (27,11 mmoles) d'azoture de sodium dans 30 ml de N,N-diméthylformamide.

On laisse revenir à température ambiante puis on concentre sous pression réduite. On reprend le résidu par du dichlorométhane et de l'eau, on sépare la phase aqueuse, on l'extrait deux fois avec du dichlorométhane, on lave les phases organiques réunies avec une solution aqueuse saturée en chlorure de sodium et on les sèche sur sulfate de sodium. Après évaporation du solvant, on obtient 3,8 g de produit sous forme d'huile utilisé tel quel dans l'étape suivante.

### 6.3. 2-[1-(6-Chloro-quinolin-2-yl)-pipéridin-4-yl]-éthylamine

A une solution de 3,50 g (11,08 mmoles) de 2-[4-(2-azido-éthyl)-pipéridin-1-yl]-6-chloro-quinoline, obtenu à l'étape 6.2. dans 100 ml de THF/Eau (1/1), on ajoute par petite portion, à température ambiante, 4,36 g (16,62 mmoles) de triphénylphosphine. On poursuit l'agitation à température ambiante pendant dix heures.

On concentre sous pression réduite. On ajoute de l'acétate d'éthyle, on sépare la phase aqueuse, on l'extrait trois fois avec de l'acétate d'éthyle, on lave les phases organiques réunies avec une solution aqueuse saturée en chlorure de sodium, on les sèche sur sulfate de sodium et on concentre le filtrat sous pression réduite. Après chromatographie sur gel de silice en éluant avec un mélange 90/10/1 de dichlorométhane, de méthanol et d'ammoniaque à 28%, on obtient 1,77 g de produit pur sous forme d'huile qui cristallise à température ambiante.
PF (°C) : 68-70°C
RMN ¹H (CDCl₃) δ (ppm) : 7,70 (d, 1H) ; 7,50 (m, 2H) ; 7,35 (m, 1H) ; 6,95 (d, 1H) ; 4,45 (large d, 2H) ; 2,90 (large td, 2H) ; 2,70 (t, 2H) ; 1,70 (m, 2H) ; 1,60-1,10 (m, 5H).

### 6.4. 2-[1-(6-Chloro-quinolin-2-yl)-pipéridin-4-yl]-éthylcarbamate de 4-nitro-phényle

On procède suivant la méthode décrite dans l'exemple 1 (étape 1.5.). A partir de 5,00 g (17,25 mmoles) de 2-[1-(6-chloro-quinolin-2-yl)-pipéridin-4-yl]-éthylamine, préparé à l'étape 6.3., de 3,825 g (18,98 mmoles) de chloroformiate de 4-nitrophényle, de 4,46 g (34,51 mmoles) de *N,N-*diisopropyléthylamine, de 0,105 g (0,86 mmole) de *N,N-*diméthylaminopyridine, et après trituration dans un mélange de diisopropyléther et d'hexane , on obtient 7,8 g de produit pur sous forme de poudre blanche.
PF(°C) : 80-84 °C

### 6.5. 2-[1-(6-Chloro-quinolin-2-yl)-pipéridin-4-yl]-éthylcarbamate de 3-carbamoyl-isoxazol-5-ylméthyle

On procède suivant le mode opératoire décrit dans l'exemple 4. A partir de 0,50 g (1,10 mmoles) de 2-[1-(6-chloro-quinolin-2-yl)-pipéridin-4-yl]-éthylcarbamate de 4-nitro-phényle, obtenu à l'étape 6.4., de 0,284 g (2,2 mmoles) de N,N-diisopropyléthylamine, de 0,067 g (0,55 mmole) de *N,N-*diméthylaminopyridine, de 0,156 g (1,1 mmole) de 3-carbamoyl-isoxazol-5-ylméthanol, on obtient 0,250 g de produit pur sous forme de poudre blanche.
PF(°C) : 220-222°C ; LC-MS : M+H = 468
RMN ¹H (DMSO) δ (ppm) : 8,15 (large s, 1H) ; 8,0 (d, 1H) ; 7,85 (large s, 1H) ; 7,75 (d, 1H) ; 7,50 (q, 2H) ; 7,45 (large t, 1H) ; 7,30 (d, 1H) ; 6,80 (s, 1H) ; 5,20 (s, 2H) ; 4,50 (large d, 2H) ; 3,10 (m, 2H) ; 2,90 (large t, 2H) ; 1,80 (large d, 2H) ; 1,60 (m, 1H) ; 1,40 (m, 2H) ; 1,15 (m, 2H).

### Exemple 7 (Composé N°20)

### 2-[1-(6-Chloro-quinolin-2-yl)-pipéridin-4-yl]-éthylcarbamate de 3-méthylcarbamoyl-isoxazol-5-ylméthyle

### 7.1. 5-{2-[1-(6-Chloro-quinolin-2-yl)-pipéridin-4-yléthylcarbamoyloxyméthyl}-isoxazole-3-carboxylate d'éthyle

On procède suivant le mode opératoire décrit dans l'exemple 1 (étape 1.7.). A partir de 0,5 g (1,1 mmoles) de 2-[1-(6-chloro-quinolin-2-yl)-pipéridin-4-yl]-éthylcarbamate de 4-nitro-phényle, décrit dans l'exemple 6 (étape 6.4.), de 0,311 g (2,2 mmoles) de *N,N*-diisopropyléthylamine, de 0,067 g (0,55 mmole) de *N,N*-diméthylaminopyridine, de 0,188 g (1,1 mmoles) de 5-hydroxyméthyl-isoxazole-3-carboxylate d'éthyle, et après chromatographie sur gel de silice en éluant avec un mélange 98/2 de dichlorométhane et de méthanol, on obtient 0,4 g de produit pur sous forme d'une poudre blanche.
PF(°C) : 113-115°C
RMN ¹H (CDCl₃) δ (ppm) : 7,70 (d, 1H) ; 7,50 (m, 1H) ; 7,45 (m, 1H) ; 7,35 (m, 1H) ; 6,90 (d, 1H) ; 6,65 (s, 1H) ; 5,20 (s, 2H) ; 4,70 (m, 2H) ; 4,50-4,30 (m, 5H) ; 3,20 (m, 2H) ; 2,90 (large t, 2H) ; 1,80 (large d, 2H) ; 1,60-1,20 (m, 6H).

### 7.2. 2-[1-(6-Chloro-quinolin-2-yl)-pipéridin-4-yl]-éthylcarbamate de 3-méthylcarbamoyl-isoxazol-5-ylméthyle

A une solution de 0,17 g (0,35 mmole) de 5-{2-[1-(6-chloro-quinolin-2-yl)-pipéridin-4-yléthylcarbamoyloxyméthyl}-isoxazole-3-carboxylate d'éthyle, préparé à l'étape 7.1., dans 5 ml d'un mélange 5/1 de méthanol et de dichlorométhane, on ajoute à température ambiante, 1 ml (6,98 mmoles) d'une solution de méthylamine (7M) dans le tétrahydrofurane. On poursuit l'agitation à environ 50°C pendant 2 heures.

On laisse revenir à température ambiante puis on refroidit avec un bain de glace. Le précipité ainsi formé est filtré puis rincé abondamment à l'éther. Après séchage sous vide à environ 70°C, on obtient 0,12 g de produit pur sous forme de poudre blanche.
PF(°C) : 200-202°C, LC-MS : M+H = 472
RMN ¹H (DMSO) δ (ppm) : 8,70 (large s, 1H) ; 8,0 (d, 1H) ; 7,80 (s, 1H) ; 7,55 (q, 2H) ; 7,45 (large t, 1H) ; 7,30 (d, 1H) ; 6,80 (s, 1H) ; 5,20 (s, 2H) ; 4,50 (large d, 2H) ; 3,10 (m, 2H) ; 2,90 (large t, 2H) ; 2,80 (d, 3H) ; 1,80 (large d, 2H) ; 1,60 (m, 1H) ; 1,40 (m, 2H) ; 1,15 (m, 2H).

### Exemple 8 (Composé N°23)

### 2-[1-(6-Fluoro-quinolin-2-yl)-pipéridin-4-yl]-éthylcarbamate de 3-carbamoyl-isoxazol-5-ylméthyle

### 8.1. 2-[1-(6-Fluoro-quinolin-2-yl)-pipéridin-4-yl]-éthylcarbamate de tert-butyle

Dans un tube scellé, on introduit 2,18 g (9,64 mmoles) de 2-bromo-6-fluoro-quinoline, 2,00 g (8,76 mmoles) de 2-pipéridin-4-yl-éthylcarbamate de *tert*-butyle, 2,08 g (26,28 mmoles) de pyridine et 15 mL d'acétonitrile. On chauffe ensuite à 80°C pendant 12 heures.

On laisse revenir à température ambiante puis on refroidit avec un bain de glace. Le précipité ainsi formé est filtré puis rincé abondamment à l'éther. Après séchage sous vide à environ 50°C, on obtient 2,00 g de produit pur sous forme de poudre blanche.
PF(°C) : 127-129°C

### 8.2. Chlorhydrate de 2-[1-(6-fluoro-quinolin-2-yl)-pipéridin-4-yl]-éthylamine

A une solution de 1,9 g (5,09 mmoles) de 2-[1-(6-fluoro-quinolin-2-yl)-pipéridin-4-yl]-éthylcarbamate de *tert*-butyle, obtenu à l'étape 8.1., dans 6 ml de dichlorométhane, refroidie par un bain de glace/eau, on ajoute lentement 10 ml (40 mmoles) d'une solution d'acide chlorhydrique 4N dans le dioxane. On poursuit l'agitation à température ambiante pendant 2 heures.

Après évaporation sous pression réduite, on obtient 0,9 g de produit sous forme de chlorhydrate utilisé tel quel dans l'étape 8.4., ci-dessous.

### 8.3. 4-Nitro-phényl-carbonate de 3-carbamoyl-isoxazol-5-ylméthyle

A une solution de 2,00 g (14,07 mmoles) de 3-carbamoyl-isoxazol-5-ylméthanol, de 1,71 ml (21,11 mmoles) de pyridine et de 0,172 g (1,41 mmoles) de *N,N*-diméthylaminopyridine dans 15 ml de dichlorométhane, refroidie à environ 0°C, on ajoute par petites portions 2,84 g (14,07 mmoles) de chloroformiate de 4-nitrophényle. On poursuit l'agitation à 0°C pendant 1 heure puis à température ambiante pendant 1 heure.

Le précipité ainsi formé est filtré puis rincé abondamment avec le diisopropyléther. Après séchage sous vide à environ 60°C, on obtient 3,12 g de produit sous forme de solide blanc utilisé tel quel dans l'étape suivante.
PF(°C) : 143-145°C
RMN ¹H (DMSO) δ (ppm) : 8,40 (d, 2H) ; 8,25 (large s, 1H) ; 7,90 (large s, 1H) ; 7,65 (d, 2H) ; 7,0 (s, 1H) ; 5,50 (s, 2H).

### 8.4. 2-[1-(6-Fluoro-quinolin-2-yl)-pipéridin-4-yl]-éthylcarbamate de 3-carbamoyl-isoxazol-5-ylméthyle

On procède suivant le mode opératoire décrit dans l'exemple 1 à l'étape 1.6. A partir de 0,30 g (0,87 mmole) de chlorhydrate de 2-[1-(6-fluoro-quinolin-2-yl)-pipéridin-4-yl]-éthylamine, obtenu à l'étape 8.2., de 0,266 g (0,87 mmole) de 4-nitro-phényl-carbonate de 3-carbamoyl-isoxazol-5-ylméthyle, obtenu à l'étape 8.3., de 0,367 g (2,6 mmoles) de N,N-diisopropyléthylamine et de 0,053 g (0,43 mmole) de *N,N-*diméthylaminopyridine, on obtient 0,260 g de produit pur sous forme de poudre blanche.
PF(°C) : 200-202°C, LC-MS : M+H = 442
RMN ¹H (DMSO) δ (ppm) : 8,15 (large s, 1H) ; 8,05 (d, 1H) ; 7,80 (large s, 1H) ; 7,55 (dd, 1H) ; 7,50 (dd, 1H) ; 7,40 (m, 2H) ; 7,30 (d, 1H) ; 6,75 (s, 1H) ; 5,20 (s, 2H) ; 4,50 (large d, 2H) ; 3,10 (m, 2H) ; 2,90 (large t, 2H) ; 1,75 (large d, 2H) ; 1,60 (m, 1H) ; 1,40 (m, 2H) ; 1,15 (m, 2H).

### Exemple 9 (Composé N°25)

### 2-[1-(6-Fluoro-quinolin-2-yl)-pipéridin-4-yl]-éthylcarbamate de 3-méthylcarbamoyl-isoxazol-5-ylméthyle

### 9.1. 4-Nitro-phényl-carbonate de 3-méthylcarbamoyl-isoxazol-5-ylméthyle

On procède suivant le mode opératoire décrit dans l'exemple 8 (étape 8.3.). A partir de 2 g (12,81 mmoles) de 3-méthylcarbamoyl-isoxazol-5-ylméthanol, de 2,58 g (12,81 mmoles) de chloroformiate de 4-nitrophényle, de 1,52 g (19,21 mmoles) de pyridine et de 0,157 g (1,28 mmoles) de *N,N*-diméthylaminopyridine, on obtient 2,6 g de produit pur sous forme de poudre blanche.
PF(°C) : 166-168°C
RMN ¹H (CDCl₃) δ (ppm) : 8,40(d, 2H) ; 7,50 (d, 2H) ; 7,0 (s, 1H) ; 6,90 (large s, 1H) ; 5,50 (s, 2H) ; 3,10 (d, 3H).

### 9.2. 2-[1-(6-Fluoro-quinolin-2-yl)-pipéridin-4-yl]-éthylcarbamate de 3-méthylcarbamoyl-isoxazol-5-ylméthyle

On procède suivant le mode opératoire décrit dans l'exemple 1 (étape 1.6.). A partir de 0,310 g (1,13 mmoles) de 2-[1-(6-fluoro-quinolin-2-yl)-pipéridin-4-yl]-éthylamine, obtenu à l'étape 8.2., de 0,383 g (1,19 mmoles) de 4-nitro-phényl-carbonate de 3-méthylcarbamoyl-isoxazol-5-ylméthyle, obtenu à l'étape 9.1. et de 0,32 g (2,27 mmoles) *N, N-*diisopropyléthylamine, on obtient 0,33 g de produit pur sous forme de poudre blanche.
PF(°C) : 180-182°C, LC-MS : M+H = 456
RMN ¹H (DMSO) δ (ppm) : 8,70 (large s, 1H) ; 8,0 (d, 1H) ; 7,55 (dd, 1H) ; 7,50 (dd, 1H) ; 7,40 (m, 2H) ; 7,30 (d, 1H) ; 6,80 (s, 1H) ; 5,20 (s, 2H) ; 4,50 (large d, 2H) ; 3,10 (m, 2H) ; 2,85 (large t, 2H) ; 2,75 (d, 3H) ; 1,75 (large d, 2H) ; 1,60 (m, 1H) ; 1,40 (m, 2H) ; 1,15 (m, 2H).

### Exemple 10 (Composé N°28)

### 2-[5'-(3-Fluoro-phényl)-3,4,5,6-tétrahydro-2H-[1,2']bipyridinyl-4-yl]-éthylcarbamate de 3-carbamoyl-isoxazol-5-ylméthyle

### 10.1. 2-(5'-Bromo-3,4,5,6-tétrahydro-2H-[1,2']bipyridinyl-4-yl)-éthylcarbamate de tert-butyle

Dans un autoclave, on introduit 10,37 g (43,80 mmoles) de 2,5-dibromopyridine, 10,00 g (43,80 mmoles) de 2-pipéridin-4-yl-éthylcarbamate de tert-butyle et 6,05 g (43,80 mmoles) de carbonate de potassium. On chauffe ensuite à 130°C pendant 12 heures.

On laisse revenir à température ambiante puis on reprend le mélange réactionnel par du chloroforme et une solution aqueuse saturée en hydrogénocarbonate de sodium. On sépare la phase aqueuse, on l'extrait deux fois avec du chloroforme, on lave les phases organiques réunies avec une solution aqueuse saturée en chlorure de sodium, on les sèche sur sulfate de sodium et on concentre le filtrat sous pression réduite. Après chromatographie sur gel de silice en éluant avec un mélange 95/5 de dichlorométhane et de méthanol, on obtient 6,9 g de produit pur sous forme de poudre blanche.
PF(°C) : 108-110°C

### 10.2. 2-(5'-Bromo-3,4,5,6-tétrahydro-2H-[1,2']bipyridinyl-4-yl)-éthylamine

A une solution de 6,90 g (17,95 mmoles) de 2-(5'-bromo-3,4,5,6-tétrahydro-2H-[1,2']bipyridinyl-4-yl)-éthylcarbamate de tert-butyle, obtenu à l'étape 10.1., dans 100 ml de dichlorométhane, refroidie par un bain de glace/eau, on ajoute lentement 20,47 g (179,54 mmoles) d'acide trifluoroacétique. On poursuit l'agitation à température ambiante pendant 2 heures. On verse le mélange réactionnel dans un mélange d'eau glacée et d'ammoniaque à 28 %. On décante, on extrait deux fois la phase aqueuse par du dichlorométhane, on lave les phases organiques réunies avec une solution aqueuse saturée en chlorure de sodium, on les sèche sur sulfate de sodium et on concentre sous pression réduite.

On obtient 4,9 g de produit sous forme d'huile utilisé tel quel dans l'étape suivante.

### 10.3. [2-(5'-Bromo-3,4,5,6-tétrahydro-2H-[1,2']bipyridinyl-4-yl)-éthylcarbamate de 4-nitro-phényle

On procède suivant la méthode décrite dans l'exemple 1 (étape 1.5.). A partir de 3,00 g (10,56 mmoles) de 2-(5'-bromo-3,4,5,6-tétrahydro-2*H*-[1,2']bipyridinyl-4-yl)-éthylamine, préparé à l'étape 10.2., de 2,34 g (11,61 mmoles) de chloroformiate de 4-nitrophényle, de 3,41 g (26,39 mmoles) de N,N-diisopropyléthylamine, de 0,129 g (1,06 mmoles) de *N,N-*diméthylaminopyridine, et après trituration dans le disopropyléther, on obtient 3,27 g de produit sous forme d'un solide amorphe.

### 10.4. 2-(5'-Bromo-3,4,5,6-tétrahydro-2H-[1,2']bipyridinyl-4-yl)-éthylcarbamate de 3-carbamoyl-isoxazol-5-ylméthyle

On procède suivant le mode opératoire décrit dans l'exemple 4. A partir de 1,00 g (2,23 mmoles) de [2-(5'-bromo-3,4,5,6-tétrahydro-2*H*-[1,2']bipyridinyl-4-yl)-éthylcarbamate de 4-nitro-phényle, obtenu à l'étape 10.3., de 0,575 g (4,45 mmoles) de *N,N*-diisopropyléthylamine, de 0,136 g (1,11 mmoles) de *N,N*-diméthylaminopyridine et de 0,381 g (2,67 mmoles) de 5-hydroxyméthyl-isoxazole-3-carboxamide, et après trituration avec de l'éther éthylique, on obtient 0,740 g de produit pur sous forme de poudre blanche.
PF(°C) : 164-166°C

### 10.5. 2-[5'-(3-Fluoro-phényl)-3,4,5,6-tétrahydro-2H-[1,2']bipyridinyl-4-yl]-éthylcarbamate de carbamoyl-isoxazol-5-ylméthyle

Sous atmosphère inerte, on introduit 0,735 g (1,63 mmoles) de 2-(5'-bromo-3,4,5,6-tétrahydro-2*H*-[1,2']bipyridinyl-4-yl)-éthylcarbamate de 3-carbamoyl-isoxazol-5-ylméthyle, obtenu à l'étape 10.4., 0,256 g (1,83 mmoles) d'acide 3-fluorophénylboronique, 1,493 g (4,58 mmoles) de carbonate de césium en suspension dans 8 ml d'un mélange 9/1 de tétrahydrofurane et d'eau. On ajoute ensuite 0,125 g (0,15 mmole) de PdCl₂dppf.CH₂Cl₂. On chauffe ensuite à environ 80°C pendant 18 heures.

On laisse revenir à température ambiante, on sépare les sels par filtration sur célite, puis on reprend le filtrat avec de l'acétate d'éthyle et de l'eau, on sépare la phase aqueuse, on l'extrait deux fois avec de l'acétate d'éthyle, on lave les phases organiques réunies avec une solution aqueuse saturée en chlorure de sodium et on les sèche sur sulfate de sodium. Après évaporation du solvant, la gomme marron obtenue est triturée dans le diisopropyléther. Le solide vert obtenu est ensuite filtré puis séché sous vide à environ 80°C.

On obtient 0,651 g de produit.
PF(°C) : 172-176°C, LC-MS : M+H = 468
RMN ¹H (DMSO) δ (ppm) : 8,50 (s, 1H) ; 8,15 (large s, 1H) ; 7,90 (dd, 1H) ; 7,80 (large s, 1H) ; 7,70-7.40 (m, 4H) ; 7,15 (m, 1H) ; 6,90 (d, 1H) ; 6,80 (s, 1H) ; 5,20 (s, 2H) ; 4,35 (large d, 2H) ; 3,10 (m, 2H) ; 2,85 (large t, 2H) ; 1,80 (large d, 2H) ; 1,60 (m, 1H) ; 1,40 (m, 2H) ; 1,15 (m, 2H).

### Exemple 11 (Composé N°53)

### 2-[1-(6-Chloro-quinolin-2-yl)-pipéridin-4-yl]-éthylcarbamoyloxyméthyl}-isoxazole-3- acide carboxylique

### 11.1. {2-[1-(6-Chloro-quinolin-2-yl)-pipéridin-4-yl]-éthyl}-carbamate de tert-butyle

Dans un tube scellé, on introduit 2,00 g (8,76 mmoles) de 2-pipéridin-4-yl-éthyl)-carbamate de tert-butyle (commerciale), 1,73 g (8,76 mmoles) de 2,6-dichloro-quinoline (commerciale) et 1,27 g (36,79 mmoles) de carbonate de potassium dans 11 ml de DMSO. On chauffe ensuite à 130°C pendant 12 heures. On laisse revenir à température ambiante puis on reprend le mélange réactionnel par du dichlorométhane et de l'eau. On sépare la phase aqueuse, on l'extrait deux fois avec du dichlorométhane, on lave les phases organiques réunies avec une solution aqueuse saturée en chlorure d'ammonium, on les sèche sur sulfate de sodium et on concentre le filtrat sous pression réduite. Après évaporation du solvant, on purifie le résidu obtenu par chromatographie sur gel de silice en éluant avec un mélange 98/2/0,2 de dichlorométhane, de méthanol et d'ammoniaque à 28%. On obtient ainsi 3,40 g de produit pur sous forme de poudre.
LC-MS : M+H = 390
PF(°C) : 120-122°C
RMN ¹H (CDCl₃) δ (ppm) : 7,80 (d, 1H) ; 7,65 (d, 1H) ; 7,60 (s, 1H) ; 7,40 (d, 1H) ; 7,00 (d, 1H) ; 4,50 (large d, 3H) ; 3,25 (m, 2H); 2,90 (m, 2H) ; 1,90 (d, 2H) ; 1,65 (m, 1H) ; 1,45 (m, 11H) ; 1,25 (m, 2H).

### 11.2. 2-[1-(6-Chloro-quinolin-2-yl)-pipéridin-4-yl]-éthylamine

A une solution de 10,95 g (28,08 mmoles) de {2-[1-(6-chloro-quinolin-2-yl)-pipéridin-4-yl]-éthyl}-carbamate de *tert-*butyle, obtenu à l'étape 11.1., dans 10 ml de dioxane, refroidie par un bain de glace/eau, on ajoute lentement 28 ml (112,00 mmoles) d'une solution d'acide chlorhydrique 4N dans le dioxane. On poursuit l'agitation à température ambiante pendant 3 heures. Après filtration sur fritté, on obtient le produit sous forme de chlorhydrate puis on basifie par un traitement avec de la soude 35%. Après extraction au dichlorométhane puis séchage sur sulfate de sodium et évaporation à sec, on obtient 8,13 g d'une poudre blanche.
LC-MS : M+H = 290
PF(°C) : 118-120°C
RMN ¹H (CDCl₃) δ (ppm) : 7,80 (d, 1H) ; 7,65 (d, 1H) ; 7,60 (s, 1H) ; 7,45 (d, 1H) ; 7,00 (d, 1H) ; 4,50 (large d, 2H) ; 3,00 (m, 2H); 2,80 (t, 2H) ; 1,85 (d, 2H) ; 1,65 (m, 1H) ; 1,50 (m, 2H) ; 1,30-1,10 (m, 4H).

### 11.3. 5-{2-[1-(6-Chloro-quinolin-2-yl)-pipéridin-4-yl]-éthylcarbamoyloxyméthyl}-isoxazole-3-carboxylate d'éthyle

A une solution de 3,54 g (20,70 mmoles) de 5-hydroxyméthyl-isoxazole-3-carboxylate d'éthyle (commerciale), 7,88 mL (41,41 mmoles) de *N,N*-diisopropyléthylamine et 1,26 g (10,35 mmoles) de *N,N*-diméthylaminopyridine dans 120 mL de dichlorométhane et refroidit à 0°C, on ajoute par portions 4,17 g (20,70 mmoles) de chloroformate de *p*-nitrophényle. Le mélange est agité à 10°C pendant 2 heures puis évaporé à sec. Le résidu obtenu est repris dans 120 mL de 1,2-dichloroéthane puis on ajoute 6,00 g (20,70 mmoles) de 2-[1-(6-Chloro-quinolin-2-yl)-pipéridin-4-yl]-éthylamine, obtenu à l'étape 11.2. et 5 mL (26,27 mmoles) de *N,N-*diisopropyléthylamine. Le mélange est chauffé à 70°C pendant 12 heures.

Après retour à température ambiante, on filtre l'insoluble et on ajoute une solution aqueuse 1N de soude au filtrat. Le produit est ensuite extrait avec du dichlorométhane. Les phases organiques réunies sont successivement lavées par une solution aqueuse saturée de chlorure d'ammonium, puis par une solution aqueuse saturée de chlorure de sodium. Après avoir séché les phases organiques sur sulfate de sodium, on concentre sous pression réduite. Après purification sur colonne de gel de silice en éluant avec un mélange 98/2/0,2 de dichlorométhane, de méthanol et d'ammoniaque 28%, on triture dans le diisopropyléther pour obtenir 5,10 g de produit attendu sous forme d'un solide blanc.
PF(°C) : 113-115°C
RMN ¹H (CDCl₃) δ (ppm) : 7,70 (d, 1H) ; 7,50 (m, 1H) ; 7,45 (m, 1H) ; 7,35 (m, 1H) ; 6,90 (d, 1H) ; 6,65 (s, 1H) ; 5,20 (s, 2H) ; 4,70 (m, 2H) ; 4,50-4,30 (m, 5H) ; 3,20 (m, 2H) ; 2,90 (large t, 2H) ; 1,80 (large d, 2H) ; 1,60-1,20 (m, 6H).

### 11.4. 5-{2-[1-(6-Chloro-quinolin-2-yl)-pipéridin-4-yl]-éthylcarbamoyloxyméthyl}-isoxazole-3-acide carboxylique

A une solution de 1,00 g (2,05 mmoles) de 5-{2-[1-(6-chloro-quinolin-2-yl)-pipéridin-4-yl]-éthylcarbamoyloxyméthyl}-isoxazole-3-carboxylate d'éthyle, obtenu à l'étape 11.3. dans 51 mL d'éthanol, on ajoute 10,27 mL (10,27 mmoles) d'une solution aqueuse de soude (1N). Le mélange est agité à température ambiante pendant 2 heures. Après évaporation à sec, le résidu est repris dans un minimum d'eau puis on ajoute, à 0°C, une solution aqueuse d'acide chlorhydrique 1N jusqu'à pH 4-5. Après décantation, l'huile obtenue est triturée dans l'acétone pour obtenir 0,45 g de produit attendu sous forme d'un solide blanc.
PF(°C) : 180-182°C, LC-MS : M+H = 459
RMN ¹H (DMSO) δ (ppm) : 8,40 (large s, 1H) ; 8,00 (large s, 2H) ; 7,85 (large s, 1H) ; 7,75 (d, 1H) ; 7,55 (d, 1H) ; 6,85 (s, 1H) ; 5,20 (s, 2H) ; 4,50 (large d, 2H) ; 3,55 (m, 2H) ; 3,10 (m, 2H) ; 1,90 (m, 2H) ; 1,70 (m, 1H) ; 1,40 (m, 2H) ; 1,25 (m, 2H).

### Exemple 12 (Composé N°60)

### {2-[3-Diméthylamino-1-(4-trifluorométhyl-pyrimidin-2-yl)-azétidin-3-yl]-éthyl}-carbamate de 3-méthylcarbamoyl-isoxazol-5-ylméthyle

### 12.1. 3-Cyanométhyl-3-diméthylamino-azétidine-1-carboxylate de tert-butyle

Dans un tube scellé, 1,20 g (6,18 mmoles) 3-cyanométhylène-azétidine-1-carboxylate de *tert*-butyle (WO2009/064835), est mis en solution dans 15 mL de méthanol. On ajoute 6,18 mL (12,36 mmoles) d'une solution de diméthylamine dans le méthanol et le milieu réactionnel est agité à 80°C pendant 3 heures.

On laisse revenir à température ambiante puis on évapore à sec. Le résidu obtenu est chromatographié sur colonne de gel de silice en éluant avec un mélange 97/3/0,3 de dichlorométhane, de méthanol et d'ammoniaque 28% et on obtient 1,32 g de produit attendu sous forme d'huile.
LC-MS : M+H = 240
RMN ¹H (DMSO) δ (ppm) : 3,75 (m, 4H) ; 2,90 (s, 2H) ; 2,15 (s, 6H) ; 1,40 (s, 9H).

### 12.2. 3-(2-Amino-éthyl)-3-diméthylamino-azétidine-1-carboxylate de tert-butyle

A une solution de 1,30 g (5,43 mmoles) de 3-cyanométhyl-3-diméthylamino-azétidine-1-carboxylate de *tert*-butyle obtenu à l'étape précédente, dans 27 mL d'une solution de méthanol, on ajoute 1,59 g (27,16 mmoles) de Nickel de Raney. Le milieu réactionnel est placé dans un appareil de Parr sous atmosphère d'hydrogène (70 psi) à 50°C pendant 6 heures. On filtre sur büchner puis on concentre le filtrat sous pression réduite. On obtient ainsi 1,28 g de produit attendu sous forme d'une huile incolore.
RMN ¹H (CDCl₃) δ (ppm) : 3,90 (d, 2H) ; 3,60 (d, 2H) ; 2,90 (m, 2H) ; 2,25 (s, 6H) ; 2,10 (m, 2H) ; 1,90 (m, 2H) ; 1,40 (s, 9H).

### 12.3. 3-Diméthylamino-3-[2-(3-méthylcarbamoyl-isoxazol-5-ylméthoxycarbonylamino)-éthyl]-azétidine-1-carboxylate de tert-butyle

On chauffe à 80°C pendant 3 heures, une solution contenant 0,60 g (2,47 mmoles) de 3-(2-amino-éthyl)-3-diméthylamino-azétidine-1-carboxylate de *tert*-butyle obtenu à l'étape 12.2., 0,87 g (2,71 mmole) de 4-nitro-phényl-carbonate de 3-(méthylcarbamoyl)-isoxazol-5-ylméthyle obtenu à l'étape 9.2., 860 µL (4,93 mmoles) de N,N-diisopropyléthylamine et de 0,15 g (1,23 mmole) de N,N-diméthylaminopyridine dans 12 mL de 1,2-dichloroéthane.

On ajoute de l'eau au milieu réactionnel, on sépare la phase aqueuse, on l'extrait plusieurs fois avec du dichlorométhane, on lave les phases organiques réunies avec une solution aqueuse de soude (1N) puis avec une solution aqueuse saturée en chlorure d'ammonium. On les sèche sur sulfate de sodium et on concentre le filtrat sous pression réduite.

Après évaporation du solvant, on purifie le résidu obtenu par chromatographie sur gel de silice en éluant avec un mélange 97/3/0,3 de dichlorométhane, de méthanol et d'ammoniaque 28% et on obtient 0,64 g de produit attendu sous forme de cire.
LC-MS : M+H = 426
RMN ¹H (DMSO) δ (ppm): 8,70(large s, 1H) ; 7,40 (large s, 1H) ; 6,80 (s, 1H) ; 5,20 (s, 2H) ; 3,80 (m, 2H) ; 3,60 (m, 2H) ; 3,10 (m, 2H) ; 2,80 (s, 3H) ; 2,15 (s, 6H) ; 1,80 (m, 2H) ; 1,40 (s, 9H).

### 12.4. Chlorhydrate de [2-(3-Diméthylamino-azétidin-3-yl)-éthyl]-carbamate de 3-methylcarbamoyl-isoxazol-5-ylméthyle (1:2)

On procède suivant le mode opératoire décrit dans l'exemple 11 (étape 11.2.). A partir de 0,58 g (1,36 mmoles) de 3-diméthylamino-3-[2-(3-méthylcarbamoyl-isoxazol-5-ylméthoxycarbonylamino)-éthyl]-azétidine-1-carboxylate de *tert*-butyle, obtenu à l'étape 12.3., 4 mL (16 mmoles) d'une solution d'acide chlorhydrique 4N dans le dioxane, et après trituration à l'éther, on obtient 1,27 g du produit attendu sous forme d'une poudre blanche.
PF(°C) : 210-212°C
RMN ¹H (DMSO) δ (ppm) : 12, 50 (large s, 1H) ; 9, 80 (large s, 1H) ; 9,20 (large s, 1H) ; 8,80(large s, 1H) ; 7,80 (large s, 1H) ; 6,80 (s, 1H) ; 5,20 (s, 2H) ; 4,50 (m, 2H) ; 4,10 (m, 2H) ; 3,40 (m, 2H) ; 2,80 (s, 3H) ; 2,55 (s, 6H) ; 2,10 (t, 2H).

### 12.5. {2-[3-Diméthylamino-1-(4-trifluorométhyl-pyrimidin-2-yl)-azétidin-3-yl]-éthyl}-carbamate de 3-méthylcarbamoyl-isoxazol-5-ylméthyle

Dans un réacteur micro-onde (BIOTAGE modèle Initiator™ 2.0), 0,40 g (1,00 mmole) de chlorhydrate de [2-(3-diméthylamino-azétidin-3-yl)-éthyl]-carbamate de 3-méthylcarbamoyl-isoxazol-5-ylméthyle)(1:2) préparé à l'étape précédente, 0,20 g (1,10 mmole) de 2-chloro-4-trifluorométhyl-pyrimidine et 700 µL (4,02 mmoles) de N,N-diisopropyléthylamine sont mis en solution dans 5 mL d'acétonitrile. Le milieu réactionnel est soumis aux radiations micro-ondes pendant 10 minutes à 130°C.

On laisse revenir à température ambiante puis on ajoute de l'eau au milieu réactionnel. On sépare la phase aqueuse, on l'extrait plusieurs fois avec du dichlorométhane, on lave les phases organiques réunies avec une solution aqueuse saturée en chlorure d'ammonium. On les sèche sur sulfate de sodium et on concentre le filtrat sous pression réduite.

On purifie le résidu obtenu par chromatographie sur gel de silice en éluant avec un mélange 97/3/0,3 de dichlorométhane, de méthanol et d'ammoniaque 28% et après trituration dans l'éther diisopropylique et filtration, on obtient 0,38 g de produit attendu sous forme de poudre blanche.
PF(°C) : 154-156°C, LC-MS : M+H = 472
RMN ¹H (DMSO) δ (ppm): 8,70 (large s, 2H) ; 7,45 (t, 1H) ; 7,10 (m, 1H) ; 6,80 (s, 1H) ; 5,20 (s, 2H) ; 4,10 (d, 2H) ; 3,80 (d, 2H) ; 3,10 (m, 2H) ; 2,80 (s, 3H) ; 2,20 (s, 6H) ; 1,90 (m, 2H).

### Exemple 13 (Composé N°63)

### {2-[1-(4-Chloro-phthalazin-1-yl)-3-diméthylamino-azétidin-3-yl]-éthyl}-carbamate de 2,2,2-trifluoro-1-(1-méthyl-1H-imidazol-2-yl)-éthyle

### 13.1. 3-Diméthylamino-3-{2-[2,2,2-trifluoro-1-(1-méthyl-1H-imidazol-2-yl)-éthoxycarbonylamino]-éthyl}-azétidine-1-carboxylate de tert-butyle

A une solution de 0,60 g (2,47 mmoles) de 3-(2-amino-éthyl)-3-diméthylamino-azétidine-1-carboxylate de *tert*-butyle, préparé à l'étape 12.2., de 0,95 g (7,40 mmoles) de *N,N-*diisopropyléthylamine et de 0,06 g (0,49 mmole) de *N,N-*diméthylaminopyridine dans 12 mL de 1,2-dichloroéthane, refroidie à environ 0°C, on ajoute par petites portions 0,497 g (2,47 mmoles) de chloroformiate de 4-nitrophényle dissout dans 3 mL de 1,2-dichloroéthane. On poursuit l'agitation à température ambiante pendant 1 heure. On ajoute ensuite 0,488 g (2,71 mmoles) de 2,2,2-trifluoro-1-(1-méthyl-1H-imidazol-2-yl)-éthanol (commercial) et 0,47 g (3,70 mmoles) de N,N-diisopropyléthylamine. Le mélange est chauffé à 80°C pendant 12 heures.

Après retour à température ambiante, on ajoute de l'eau au milieu réactionnel, on sépare la phase aqueuse, on l'extrait plusieurs fois avec du dichlorométhane, on lave les phases organiques réunies avec une solution aqueuse de soude (1N) puis avec une solution aqueuse saturée en chlorure d'ammonium. On les sèche sur sulfate de sodium et on concentre le filtrat sous pression réduite.

Après purification sur colonne de gel de silice en éluant avec un mélange 97/3/0,3 de dichlorométhane, de méthanol et d'ammoniaque 28%, on obtient 0,71 g de produit attendu sous forme de cire.
LC-MS : M+H = 450
RMN ¹H (DMSO) δ (ppm) : 7,85(t, 1H) ; 7,25 (s, 1H) ; 7,00 (s, 1H) ; 6,45 (m, 1H) ; 3,75 (m, 5H) ; 3,50 (m, 2H) ; 3,10 (m, 2H) ; 2,15 (s, 6H) ; 1,85 (t, 2H) ; 1,40 (s, 9H).

### 13.2. Chlorhydrate de [2-(3-diméthylamino-azétidin-3-yl)-éthyl]-carbamate de 2,2,2-trifluoro-1-(1-méthyl-1H-imidazol-2-yl)-éthyle (1:2)

On procède suivant le mode opératoire décrit dans l'exemple 11 (étape 11.2.). A partir de 0,71 g (1,58 mmoles) de 3-diméthylamino-3-{2-[2,2,2-trifluoro-1-(1-méthyl-1H-imidazol-2-yl)-éthoxycarbonylamino]-éthyl}-azétidine-1-carboxylate de *tert*-butyle, obtenu à l'étape 13.1., de 3,90 mL (15,75 mmoles) d'une solution d'acide chlorhydrique 4N dans le dioxane, et après trituration à l'éther, on obtient 0,84 g du produit attendu sous forme d'un solide amorphe.
RMN ¹H (DMSO) δ (ppm) : 12,50 (large s, 1H) ; 10,00 (large s, 1H) ; 9,20 (large s, 1H) ; 8,30(t, 1H) ; 7,60 (s, 1H) ; 7,30 (s, 1H) ; 6,80 (m, 1H) ; 4,50 (m, 2H) ; 4,10 (m, 2H) ; 3,90 (s, 3H) ; 3,40 (m, 2H) ; 2,70 (s, 6H) ; 2,15 (m, 2H).

### 13.3. {2-[1-(4-Chloro-phthalazin-1-yl)-3-diméthylamino-azétidin-3-yl]-éthyl}-carbamate de 2,2,2-trifluoro-1-(1-méthyl-1H-imidazol-2-yl)-éthyle

On procède suivant le mode opératoire décrit dans l'exemple 12 (étape 12.5.). A partir de 0,50 g (1,09 mmole) de chlorhydrate de [2-(3-diméthylamino-azétidin-3-yl)-éthyl]-carbamate de 2,2,2-trifluoro-1-(1-méthyl-1H-imidazol-2-yl)-éthyle (1:2) obtenu dans l'étape précédente 13.2., de 0,26 g (1,31 mmole) de 1,4-dichloro-phthalazine (commercial) et de 0,70 g (5,45 mmoles) de N,N-diisopropyléthylamine dans 5,45 mL d'acétonitrile, on obtient 0,185 g du produit attendu sous forme de poudre.
PF(°C) : 168-170°C, LC-MS : M+H = 512,
RMN ¹H (DMSO) δ (ppm): 8,20 (m, 2H) ; 8,05 (m, 1H) ; 7,90 (m, 1H) ; 7,80 (large s, 1H) ; 7,20 (m, 1H) ; 6,90 (s, 1H) ; 6,40 (m, 1H) ; 4,40 (large s, 2H) ; 4,20 (large s, 2H) ; 3,70 (s, 3H) ; 3,20 (m, 2H) ; 2,30 (m, 6H) ; 1,95 (m, 2H).

### Exemple 14 (Composé N°56)

### 5-[1-(6-Chloro-quinolin-2-yl)-4-fluoro-pipéridin-4-ylméthylcarbamoyloxyméthyl]-isoxazole-3-carboxylate d'éthyle

### 14.1. 5-(4-Nitro-phénoxycarbonyloxyméthyl)-isoxazole-3-carboxylate d'éthyle

On procède suivant le mode opératoire décrit dans l'exemple 8 (étape 8.3.). A partir de 3,00 g (17,53 mmoles) de 5-hydroxyéthyl-isoxazole-3-carboxylate d'éthyle, de 3,71 g (18,40 mmoles) de chloroformate de 4-nitrophényle, de 2,07 g (26,29 mmoles) de pyridine et de 0,214 g (1,75 mmoles) de *N,N*-diméthylaminopyridine, on obtient 3,80 g de produit attendu sous forme de poudre blanche.
PF(°C) : 85-87°C, LC-MS : M+H = 337
RMN ¹H (DMSO) δ (ppm) : 8,40(d, 2H) ; 7,60 (d, 2H) ; 7,10 (s, 1H) ; 5,55 (s, 2H) ; 4,40 (q, 2H) ; 1,40 (t, 3H).

### 14.2. 4-[(3-Ethoxycarbonyl-isoxazol-5-ylméthoxycarbonylamino)-méthyl]-4-fluoro-pipéridine-1-carboxylate de tert-butyle

On procède suivant le mode opératoire décrit dans l'exemple 1 (étape 1.6.). A partir de 0,70 g (3,01 mmoles) de 4-aminométhyl-4-fluoro-pipéridine-1-carboxylate de *tert*-butyle (commercial), de 1,11 g (3,31 mmoles) de 5-(4-Nitro-phénoxycarbonyloxyméthyl)-isoxazole-3-carboxylate d'éthyle, obtenu à l'étape 14.1., on obtient 0,33 g de produit pur sous forme d'une huile orange.
RMN ¹H (DMSO) δ (ppm) : 7,75 (large t, 1H) ; 6,90 (s, 1H) ; 5,25 (s, 2H) ; 4,40 (q, 2H) ; 3,80 (m, 2H) ; 3,00 (m, 2H) ; 3,25 (m, 2H) ; 1,75-1,45 (m, 4H) ; 1,40 (s, 9H) ; 1,30 (t, 3H).

### 14.3. Trifluoroacétate de 5-(4-Fluoro-pipéridin-4-ylméthylcarbamoyloxyméthyl)-isoxazole-3-carboxylate d'éthyle

A une solution de 0,90 g (2,10 mmoles) de 4-[(3-éthoxycarbonyl-isoxazol-5-ylméthoxycarbonylamino)-méthyl]-4-fluoro-pipéridine-1-carboxylate de *tert*-butyle, obtenu à l'étape 14.2., dans 10 ml de dichlorométhane, refroidie par un bain de glace/eau, on ajoute lentement 1,06 mL (12,57 mmoles) d'une solution d'acide trifluoroacétique. On poursuit l'agitation à température ambiante pendant 3 heures.

Après évaporation sous pression réduite, on obtient 0,46 g de produit sous forme de trifluoroacétate utilisé tel quel dans l'étape 14.4., ci-dessous.

### 14.4. 5-[1-(6-Chloro-quinolin-2-yl)-4-fluoro-pipéridin-4-ylméthylcarbamoyloxyméthyl]-isoxazole-3-carboxylate d'éthyle

Dans un tube scellé, on introduit 0,465 g (1,05 mmole) de trifluoroacétate de 5-(4-fluoro-pipéridin-4-ylméthylcarbamoyloxyméthyl)-isoxazole-3-carboxylate d'éthyle, obtenu à l'étape 14.3., 0,23 g (1,15 mmole) de 2,6-dichloro-quinoline et 730 µL (4,20 mmoles) de *N,N-*diisopropyléthylamine dans 5 mL d'acétonitrile. On chauffe ensuite à 120°C pendant 12 heures. On laisse revenir à température ambiante puis on reprend le milieu réactionnel avec de l'acétate d'éthyle, on sépare la phase aqueuse, on l'extrait deux fois avec de l'acétate d'éthyle, on lave les phases organiques réunies avec une solution aqueuse saturée en chlorure d'ammonium et on les sèche sur sulfate de sodium. Après évaporation du solvant, on purifie le résidu obtenu par chromatographie sur colonne de gel de silice en éluant avec un mélange 99/1 de dichlorométhane et de méthanol et on obtient ainsi 0,07 g de produit pur sous forme de poudre blanche.
PF(°C) : 132-134°C, LC-MS : M+H = 491,
RMN ¹H (DMSO) δ (ppm) : 8,05 (d, 1H) ; 7,90 - 7,70 (m, 2H) ; 7,60 - 7,50 (m, 2H) ; 7,35 (d, 1H) ; 6,90 (s, 1H) ; 5,25 (m, 2H) ; 4, 45 - 4,30 (m, 4H) ; 3, 40 - 3,20 (m, 4H) ; 1,90 - 1,60 (m, 4H) ; 1,35 (t, 3H).

### Exemple 15 (composé N°57)

### [4-Fluoro-1-(4-trifluorométhyl-pyrimidin-2-yl)-pipéridin-4-yl]-méthylcarbamate de 3-méthylcarbamoyl-isoxazol-5-ylméthyle

### 15.1. 5-[4-Fluoro-1-(4-trifluorométhyl-pyrimidin-2-yl)-pipéridin-4-ylméthylcarbamoyloxyméthyl]-isoxazole-3-carboxylate d'éthyle

On procède suivant le mode opératoire décrit dans l'exemple 14 (étape 14.4.). A partir de 0,46 g (1,05 mmole) de trifluoroacétate de 5-(4-Fluoro-pipéridin-4-ylméthylcarbamoyloxyméthyl)-isoxazole-3-carboxylate d'éthyle, obtenu à l'étape 14.3., 0,21 g (1,15 mmole) de 2-chloro-4-trifluorométhyl-pyrimidine et 730 µL (4,20 mmoles) de *N,N-*diisopropyléthylamine dans 5 mL d'acétonitrile on obtient ainsi 0,22 g de produit pur sous forme de poudre blanche.
PF(°C) : 136-138°C, LC-MS : M+H = 476
RMN ¹H (DMSO) δ (ppm) : 8,70 (d, 1H) ; 7,75 (t, 1H) ; 7,00 (d, 1H); 6,90 (s, 1H); 5,25 (s, 2H) ; 4,50-4,30 (m, 4H) ; 3,40-3,15 (m, 4H) ; 1,90-1,50 (m, 4H) ; 1,30 (t, 3H).

### 15.2. [4-Fluoro-1-(4-trifluorométhyl-pyrimidin-2-yl)-pipéridin-4-yl]-méthylcarbamate de 3-méthylcarbamoyl-isoxazol-5-ylméthyle

Dans un tube scellé, une solution de 0,14 g (0,29 mmole) de 5-[4-fluoro-1-(4-trifluorométhyl-pyrimidin-2-yl)-pipéridin-4-ylméthylcarbamoyloxyméthyl]-isoxazole-3-carboxylate d'éthyle, préparé à l'étape 15.1., dans 1,10 mL (8,83 mmoles) d'une solution de méthylamine dans l'éthanol, est agitée à température ambiante pendant 1 heure et 30 minutes. On évapore à sec. Le résidu obtenu est trituré dans l'éther et filtré. Après séchage sous vide à environ 60°C, on obtient 0,12 g de produit pur sous forme de poudre blanche.
PF(°C) : 187-189°C, LC-MS : M+H = 461
RMN ¹H (DMSO) δ (ppm) : 8,70 (m, 2H) ; 7,75 (m, 1H) ; 7,00 (m, 1H) ; 6,80 (s, 1H) ; 5,20 (m, 2H) ; 4,45 (m, 2H) ; 3,40 - 3,20 (m, 4H) ; 2,80 (m, 3H) ; 1,90 - 1,55 (m, 4H).

### Exemple 16 (composé N°43)

### [2-(5'-Isobutyl-3,4,5,6-tétrahydro-2H-[1,2']bipyridinyl-4-yl]-éthylcarbamate de 3-carbamoyl-isoxazol-5-ylméthyle

### 16.1. [2-(5'-Bromo-3,4,5,6-tétrahydro-2H-[1,2']bipyridinyl-4-yl)-éthyl]-carbamate d'éthyle

On procède suivant la méthode décrite dans l'exemple 1 (étape 1.5.). A partir de 4,52 g (15,90 mmoles) de 2-(5'-bromo-3,4,5,6-tétrahydro-2*H*-[1,2']bipyridinyl-4-yl)-éthylamine, préparé à l'étape 10.2., de 1,89 g (17,49 mmoles) de chloroformate d'éthyle, de 5,13 g (39,76 mmoles) de *N,N-*diisopropyléthylamine, de 0,19 g (1,59 mmoles) de *N,N-*diméthylaminopyridine, et après purification sur colonne de gel de silice en éluant avec un mélange 99/1/0,1 de dichlorométhane, de méthanol et d'ammoniaque 28%, on obtient 3,87 g de produit attendu sous forme de poudre.
PF(°C) : 87-89°C
RMN ¹H (DMSO) δ (ppm) : 8,00 (m, 1H) ; 7,30 (m, 1H) ; 6,40 (d, 1H) ; 4,45 (large s, 1H) ; 4,15-3,90 (m, 4H) ; 3,10 (m, 2H) ; 2,60 (m, 2H) ; 1,65 (m, 2H) ; 1,55-1,25 (m, 3H) ; 1,20-0,95 (m, 5H).

### 16.2. {2-[5'-(2-Méthyl-propényl)-3,4,5,6-tétrahydro-2H-[1,2']bipyridinyl-4-yl]-éthyl}-carbamate d'éthyle

On procède suivant la méthode décrite dans l'exemple 10 (étape 10.5.). A partir de 1,00 g (2,81 mmoles) de [2-(5'-bromo-3,4,5,6-tétrahydro-2H-[1,2']bipyridinyl-4-yl)-éthyl]-carbamate d'éthyle, préparé à l'étape précédente, de 0,61 g (3,37 mmoles) de 2-méthyl-1-propénylboronate de pinacol (commercial), de 2,74 g (8,42 mmoles) de carbonate de césium, en suspension dans 18 mL d'un mélange 9/1 de tétrahydrofurane et d'eau, de 0,23 g (0,28 mmole) de PdCl₂dppf.CH₂Cl₂ et après purification sur colonne de gel de silice en éluant avec un mélange 99/1/0,1 de dichlorométhane, de méthanol et d'ammoniaque 28%, on obtient 0,75 g de produit attendu sous forme de cire.
RMN ¹H (CDCl₃) δ (ppm) : 8,10 (m, 1H) ; 7,40 (m, 1H) ; 6,65 (d, 1H) ; 6,10 (m, 1H) ; 4,65 (large s, 1H) ; 4,40-4,10 (m, 4H) ; 3,25 (m, 2H) ; 2,85 (m, 2H) ; 1,90-1,75 (m, 8H) ; 1,70-1,40 (m, 4H) ; 1,35-1,20 (m, 4H).

### 16.3. [2-(5'-Isobutyl-3,4,5,6-tétrahydro-2H-[1,2']bipyridinyl-4-yl)-éthyl]-carbamate d'éthyle

A une solution de 0,74 g (2,23 mmoles) de {2-[5'-(2-Méthyl-propényl)-3,4,5,6-tétrahydro-2H-[1,2']bipyridinyl-4-yl]-éthyl}-carbamate d'éthyle obtenu à l'étape précédente, dans 30 mL de méthanol, on ajoute 0,10 g (0,94 mmole) de Palladium sur charbon. Le milieu réactionnel est placé dans un appareil de Parr sous atmosphère d'hydrogène (10 psi) à température ambiante pendant 1 heure et 30 minutes. On filtre sur büchner puis on concentre le filtrat sous pression réduite. On obtient ainsi 0,74 g de produit attendu sous forme d'une poudre.
PF(°C) : 78-80°C
RMN ¹H (CDCl₃) δ (ppm) : 8,00 (m, 1H) ; 7,30 (m, 1H) ; 6,65 (d, 1H) ; 4,65 (large s, 1H) ; 4, 35-4, 05 (m, 4H) ; 3,30 (m, 2H) ; 2,80 (m, 2H) ; 2,35 (d, 2H) ; 1,90-1,75 (m, 2H) ; 1,70-1,45 (m, 2H); 1,35-1,20 (m, 3H); 0,90 (d, 6H).

### 16.4. 2-(5'-Isobutyl-3,4,5,6-tétrahydro-2H-[1,2']bipyridinyl-4-yl)-éthylamine

A une solution de 0,64 g (1,93 mmole) de [2-(5'-isobutyl-3,4,5,6-tétrahydro-2H-[1,2']bipyridinyl-4-yl)-éthyl]-carbamate d'éthyle, obtenu à l'étape 16.3., dans 9,70 mL de éthanol/eau (1/1), on ajoute, à température ambiante, 2,17 g (38,68 mmoles) d'hydroxyde de potassium. On chauffe ensuite à 110°C pendant 12 heures. On ajoute 2,17 g (38,68 mmoles) d'hydroxyde de potassium et on laisse agiter 4 heures. On laisse revenir à température ambiante puis on concentre sous pression réduite. On reprend le milieu réactionnel avec du dichlorométhane, on sépare la phase aqueuse, on l'extrait deux fois avec du dichlorométhane, on lave les phases organiques réunies avec une solution aqueuse saturée en chlorure de sodium et on les sèche sur sulfate de sodium. Après évaporation sous pression réduite, on obtient 0,37g de produit attendu sous forme de cire de couleur jaune.
LC-MS : M+H = 262
RMN ¹H (CDCl₃) δ (ppm) : 8,00 (m, 1H) ; 7,30 (m, 1H) ; 6,65 (d, 1H) ; 4,25 (m, 2H) ; 2,80 (m, 4H) ; 2,35 (d, 2H) ; 1,80 (m, 3H) ; 1,60 (m, 1H) ; 1,45 (m, 2H) ; 1,30 (m, 4H); 0,90 (d, 6H).

### 16.5. [2-(5'-Isobutyl-3,4,5,6-tétrahydro-2H-[1,2']bipyridinyl-4-yl]-éthylcarbamate de 3-carbamoyl-isoxazol-5-ylméthyle

On procède suivant le mode opératoire décrit dans l'exemple 1 à l'étape 1.6. A partir de 0,37 g (1,42 mmole) de 2-(5'-isobutyl-3,4,5,6-tétrahydro-2H-[1,2']bipyridinyl-4-yl)-éthylamine, obtenu à l'étape 16.4., de 0,52 g (1,70 mmole) de 4-nitro-phényl-carbonate de 3-carbamoyl-isoxazol-5-ylméthyle, obtenu à l'étape 8.3., de 0,62 mL g (3,54 mmoles) de *N,N-*diisopropyléthylamine et de 0,087 g (0,71 mmole) de *N,N-*diméthylaminopyridine, dans 14 mL de 1,2-dichloroéthane, on obtient 0,42 g de produit pur sous forme de poudre blanche.
PF(°C) : 168-170°C, LC-MS : M+H = 430
RMN ¹H (DMSO) δ (ppm) : 8,10 (m, 1H) ; 7,95 - 7,75 (m, 2H) ; 7,50 - 7,25 (m, 2H) ; 6,75 (m, 2H) ; 5,20 (m, 2H) ; 4,20 (m, 2H) ; 3,10 (m, 2H) ; 2,70 (m, 2H) ; 2,30 (d, 2H); 1,85 - 1,65 (m, 3H) ; 1, 60 - 1,30 (m, 3H) ; 1,10 (m, 2H) ; 0,85 (m, 6H).

### Exemple 17 (composé N°72)

### {2-[4-Méthyl-1-(4-trifluorométhyl-pyrimidin-2-yl)-pipéridin-4-yl]-éthyl}-carbamate de 3-méthylcarbamoyl-isoxazol-5-ylméthyle

### 17.1. 4-(2-Amino-éthyl)-4-méthyl-pipéridine-1-carboxylate de tert-butyle

On procède suivant le mode opératoire décrit dans l'exemple 12 (étape 12.2.). A partir de 1,40 g (5,87 mmoles) de 4-cyanométhyl-4-méthyl-pipéridine-1-carboxylate de *tert*-butyle (WO 2006/001752) et de 1,72 g (29,37 mmoles) de Nickel de Raney dans 20 mL de méthanol sous atmosphère d'hydrogène (70 psi) à 45°C, on obtient 1,35 g de produit attendu sous forme de cire.
LC-MS : M+H = 243
RMN ¹H (CDCl₃) δ (ppm) : 3,60-3,40 (m, 2H) ; 3,30-3,10 (m, 2H) ; 2,75 (m, 2H) ; 1,50 (s, 9H) ; 1,40-1,25 (m, 6H) ; 0,95 (s, 3H).

### 17.2. 4-Méthyl-4-[2-(3-méthylcarbamoyl-isoxazol-5-ylméthoxycarbonylamino)-éthyl]-pipéridine-1-carboxylate de tert-butyle

On procède suivant le mode opératoire décrit dans l'exemple 12 (étape 12.3.). A partir de 0,54 g (2,22 mmoles) de 4-(2-amino-éthyl)-4-méthyl-pipéridine-1-carboxylate de *tert*-butyle et de 0,78 g (2,45 mmoles) de 4-nitro-phényl-carbonate de 3-(méthylcarbamoyl)-isoxazol-5-ylméthyle obtenu à l'étape 9.2., de 580 µL (3,34 mmoles) de N,N-diisopropyléthylamine et de 0,13 g (1,11 mmole) de N,N-diméthylaminopyridine dans 22 mL de 1,2-dichloroéthane, on obtient 0,94 g de produit attendu sous forme de cire.
LC-MS : M+H = 425
RMN ¹H (DMSO) δ (ppm) : 8,70 (large s, 1H) ; 7,40 (large s, 1H) ; 6,75 (s, 1H) ; 5,20 (s, 2H) ; 3,40 (m, 2H) ; 3,20 (m, 2H) ; 3,05 (m, 2H) ; 2,80 (d, 3H) ; 1,40 (m, 11H) ; 1,25 (m, 4H) ; 0,95 (s, 3H).

### 17.3. Chlorhydrate de [2-(4-méthyl-pipéridin-4-yl)-éthyl]-carbamate de 3-méthylcarbamoyl-isoxazol-5-ylméthyle

On procède suivant le mode opératoire décrit dans l'exemple 11 (étape 11.2.). A partir de 0,97 g (2,30 mmoles) de 4-méthyl-4-[2-(3-méthylcarbamoyl-isoxazol-5-ylméthoxycarbonylamino)-éthyl]-pipéridine-1-carboxylate de tert-butyle, obtenu à l'étape 17.2. et 5,74 mL (22,97 mmoles) d'une solution d'acide chlorhydrique 4N dans le dioxane, et après trituration à l'éther, on obtient 0,73 g du produit attendu sous forme d'une poudre blanche.
PF(°C) : 188-190, LC-MS : M+H = 361
RMN ¹H (DMSO) δ (ppm) : 8,70 (large s, 1H) ; 8,50 (large s, 1H) ; 7,45 (large s, 1H) ; 6,80 (s, 1H) ; 5,20 (s, 2H) ; 3,00 (m, 6H) ; 2,75 (d, 3H) ; 1,60-1,40 (m, 6H) ; 0,95 (m, 3H).

### 17.4. {2-[4-Méthyl-1-(4-trifluorométhyl-pyrimidin-2-yl)-pipéridin-4-yl]-éthyl}-carbamate de 3-méthylcarbamoyl-isoxazol-5-ylméthyle

On procède suivant le mode opératoire décrit dans l'exemple 12 (étape 12.5.). A partir de 0,30 g (0,83 mmole) de chlorhydrate de [2-(4-méthyl-pipéridin-4-yl)-éthyl]-carbamate de 3-méthylcarbamoyl-isoxazol-5-ylméthyle, de 0,23 g (1,25 mmole) de 2-chloro-4-trifluorométhyl-pyrimidine et 430 µL (2,49 mmoles) de N,N -diisopropyléthylamine, en solution dans 2,77 mL d'acétonitrile, on obtient 0.29 g de produit attendu sous forme de poudre.
PF(°C) : 160-162°C, LC-MS : M+H = 471
RMN ¹H (DMSO) δ (ppm) : 8,80 - 8,60 (m, 2H) ; 7,45 (m, 1H) ; 6,95 (m, 1H) ; 6,80 (s, 1H) ; 5,20 (s, 2H) ; 3,95 (m, 2H) ; 3,60 (m, 2H) ; 3,10 (m, 2H) ; 2,80 (s, 3H) ; 1,50 (m, 2H) ; 1,40 (m, 4H) ; 1,00 (s, 3H).

### Exemple 18 (composé N°64)

### {2-[1-(6-Chloro-quinoxalin-2-yl)-4-éthyl-pipéridin-4- yl]-éthylcarbamate de 3-méthylcarbamoyl-isoxazol-5-ylméthyle

### 18.1. 4-(Cyano-éthoxycarbonyl-méthyl)-4-éthyl-pipéridine-1-carboxylate de tert-butyle

A une solution de 2,00 g (6,79 mmoles) de 4-(cyano-éthoxycarbonyl-méthylène)-pipéridine-1-carboxylate de *tert-*butyle ester (WO 2006/001752) dans 33 mL de tétrahydrofurane, on ajoute au goutte à goutte et à -5°C sous argon, 4,53 mL (13,59 mmoles) d'une solution 3M dans l'éther de bromure d'éthyle magnésium. Le milieu réactionnel est ensuite agité à température ambiante pendant 12 heures. On ajoute de l'acétate d'éthyle et le milieu est refroidit par un bain de glace/eau puis on ajoute une solution saturée en chlorure d'ammonium. On sépare la phase aqueuse, on l'extrait trois fois avec de l'acétate d'éthyle, on lave les phases organiques réunies avec une solution aqueuse saturée en chlorure de sodium, on les sèche sur sulfate de sodium et on concentre le filtrat sous pression réduite. Le résidu est chromatographié sur gel de silice en éluant avec un mélange 95/5 de cyclohexane et d'acétate d'éthyle et on obtient 1,63 g de produit pur sous forme de poudre blanche.
LC-MS : M+H = 325
RMN ¹H (CDCl₃) δ (ppm) : 4,25 (s, 1H) ; 4,20 (q, 2H) ; 3,50-3,15 (t, 4H) ; 1,60 (q, 2H) ; 2,50-1,55 (t, 2H) ; 1,40 (s, 9H) ; 1,20 (t, 3H) ; 0,85 (t, 3H).

### 18.2. 4-Cyanométhyl-4-éthyl-pipéridine-1-carboxylate de tert-butyle

A une solution de 1,30 g (4,01 mmoles) de 4-(cyano-éthoxycarbonyl-méthyl)-4-éthyl-pipéridine-1-carboxylate de *tert*-butyle obtenu à l'étape précédente, dans 14 mL de diméthylsulfoxyde, on ajoute 0,09 g (1,60 mmole) de chlorure de sodium et 0,14 g (8,01 mmoles) d'eau. Le milieu réactionnel est agité à 150°C pendant 1 heure. On ajoute de l'éther diéthylique et de l'eau. On sépare la phase aqueuse, on l'extrait trois fois avec de l'éther diéthylique, on lave les phases organiques réunies avec une solution aqueuse saturée en chlorure de sodium, on les sèche sur sulfate de sodium et on concentre le filtrat sous pression réduite. Le résidu est chromatographié sur gel de silice en éluant avec un mélange 95/5 de cyclohexane et d'acétate d'éthyle et on obtient 0,96 g de produit pur sous forme de cire.
LC-MS : M+H = 253
RMN ¹H (CDCl₃) δ (ppm) : 3,30 (t, 4H) ; 2,60 (s, 2H) ; 1,45 (q, 2H) ; 1,40 (t, 4H) ; 1,35 (s, 9H) ; 0,80 (t, 3H).

### 18.3. 4-(2-Amino-éthyl)-4-éthyl-pipéridine-1-carboxylate de tert-butyle

On procède suivant le mode opératoire décrit dans l'exemple 12 (étape 12.2.). A partir de 0,96 g (3,83 mmole) de 4-cyanométhyl-4-éthyl-pipéridine-1-carboxylate de *tert*-butyle et de 1,12 g (19,15 mmoles) de Nickel de Raney dans 50 mL de méthanol sous atmosphère d'hydrogène (70 psi) à 50°C et après chromatographie sur colonne de gel de silice en éluant avec un mélange 95/5/0,5 de dichlorométhane, de méthanol et d'ammoniaque 28%, on obtient 0,71 g de produit attendu sous forme de cire.
LC-MS : M+H = 257
RMN ¹H (CDCl3) δ (ppm) : 3,30 (m, 4H) ; 2,50 (m, 2H) ; 1,35 (t, 2H) ; 1,40 (s, 9H) ; 1,30 (q, 2H) ; 1,25 (m, 4H) ; 0,80 (t, 3H).

### 18.4. 4-Ethyl-4-[2-(3-méthylcarbamoyl-isoxazol-5-ylmethoxycarbonylamino)-éthyl]-pipéridine-1-carboxylate de tert-butyle

On procède suivant le mode opératoire décrit dans l'exemple 12 (étape 12.3.). A partir de 0.71 g (2,78 mmoles) de 4-(2-amino-éthyl)-4-éthyl-pipéridine-1-carboxylate de *tert*-butyle, de 0,98 g (3,06 mmoles) de 4-nitro-phényl-carbonate de 3-(méthylcarbamoyl)-isoxazol-5-ylméthyle obtenu à l'étape 9.1., de 730 µL (4,17 mmoles) de N,N-diisopropyléthylamine et de 0,17 g (1,39 mmole) de N,N-diméthylaminopyridine dans 27 mL de 1,2-dichloroéthane, on obtient 0,94 g de produit attendu sous forme poudre.
LC-MS : M+H = 439
RMN ¹H (CDCl3) δ (ppm) : 8,70 (d, 1H) ; 7,40 (t, 1H) ; 6,75 (s, 1H) ; 5,20 (s, 2H) ; 3,30 (q, 2H) ; 3,25 (m, 4H) ; 2,80 (d, 3H) ; 1, 50-1, 20 (m, 8H) ; 1,10 (s, 9H) ; 0,80 (t, 3H).

### 18.5. Chlorhydrate de [2-(4-éthyl-pipéridin-4-yl)-éthyl]-carbamate de 3-méthylcarbamoyl-isoxazol-5-ylméthyle

On procède suivant le mode opératoire décrit dans l'exemple 11 (étape 11.2.). A partir de 0,94 g (2,14 mmoles) de 4-éhyl-4-[2-(3-méthylcarbamoyl-isoxazol-5-ylméthoxycarbonylamino)-éthyl]-pipéridine-1-carboxylate de *tert*-butyle, obtenu à l'étape 18.4., de 2,68 mL (10,72 mmoles) d'une solution d'acide chlorhydrique 4N dans le dioxane, et après trituration dans l'éther, on obtient 0,76 g du produit attendu sous forme d'une poudre blanche.
PF(°C) : 222-224, LC-MS : M+H = 339
RMN ¹H (DMSO) δ (ppm) : 8,70 (large s, 2H) ; 7,40 (t, 1H) ; 6,80 (s, 1H) ; 5,20 (s, 2H) ; 2,95 (m, 6H) ; 2,75 (d, 3H) ; 1,55 (q, 2H) ; 1,45 (t, 2H) ; 1,35 (m, 4H) ; 0,80 (t, 3H).

### 18.6. {2-[1-(6-Chloro-quinoxalin-2-yl)-4-éthyl-pipéridin-4-yl]-éthylcarbamate de 3-méthylcarbamoyl-isoxazol-5-ylméthyle

On procède suivant le mode opératoire décrit dans l'exemple 12 (étape 12.5.). A partir de 0,30 g (0,89 mmole) de chlorhydrate de [2-(4-éthyl-pipéridin-4-yl)-éthyl]-carbamate de 3-méthylcarbamoyl-isoxazol-5-ylméthyle, de 0,26 g (1,33 mmole) de 2,6-dichloro-quinoxaline et 460 µL (2,66 mmoles) de N,N-diisopropyléthylamine en solution dans 3 mL d'acétonitrile, on obtient 0,29 g de produit attendu sous forme de poudre jaune.
PF(°C) : 158-160°C, LC-MS : M+H = 501
RMN ¹H (DMSO) δ (ppm) : 8,80 (s, 1H) ; 8,70 (m, 1H) ; 7,90 (s, 1H) ; 7,60 (s, 2H) ; 7,40 (m, 1H) ; 6,80 (s, 1H) ; 5,20 (s, 2H) ; 3,90-3,60 (m, 4H) ; 3,00 (m, 2H) ; 2,80 (m, 3H) ; 1,50 -1,30 (m, 8H) ; 0,80 (t, 3H).

### Exemple 19 (composé N°65)

### {2-[1-(6-Chloro-quinoxalin-2-yl)-4-isobutyl-pipéridin-4- yl]-éthyl}-carbamate de 3-méthylcarbamoyl-isoxazol-5-ylméthyle

### 19.1. 4-(Cyano-éthoxycarbonyl-méthyl)-4-isobutyl-pipéridine-1-carboxylate de tert-butyle

On procède suivant le mode opératoire décrit dans l'exemple 18 (étape 18.1.). A partir de 5,00 g (16,99 mmoles) de 4-(cyano-éthoxycarbonyl-méthylène)-pipéridine-1-carboxylate de *tert*-butyle ester (WO2006/001752) dans 56 mL de tétrahydrofurane, et de 16,99 mL (33,97 mmoles) d'une solution 2M dans l'éther de bromure d'isobutyle magnésium, on obtient 1,46 g de produit pur sous forme de cire.
LC-MS : M+H = 353
RMN ¹H (CDCl3) δ (ppm) : 4,25 (q, 2H) ; 3,80 (s, 1H) ; 3,45 (m, 2H) ; 3,30 (m, 2H) ; 1,75 (m, 5H) ; 1,60 (m, 2H) ; 1,40 (s, 9H) ; 1,30 (t, 3H) ; 0,90 (d, 6H).

### 19.2. 4-Cyanométhyl-4-isobutyl-pipéridine-1-carboxylate de tert-butyle

On procède suivant le mode opératoire décrit dans l'exemple 18 (étape 18.2.). A partir de 1,46 g (4,16 mmoles) de 4-(cyano-éthoxycarbonyl-méthyl)-4-isobutyl-pipéridine-1-carboxylate de *tert*-butyle obtenu à l'étape précédente, dans 14 mL de diméthylsulfoxyde, de 0,097 g (1,66 mmole) de chlorure de sodium et de 0,15 g (8,31 mmoles) d'eau, on obtient 1,10 g de produit pur sous forme de cire.
LC-MS : M+H = 281
RMN ¹H (CDCl3) δ (ppm) : 3,50-3,30 (m, 4H) ; 2,45 (m, 2H) ; 2,40 (s, 2H) ; 1,70 (m, 2H) ; 1,60 (m, 3H) ; 1,40 (s, 9H) ; 0,90 (d, 6H).

### 19.3. 4-(2-Amino-éthyl)-4-isobutyl-pipéridine-1-carboxylate de tert-butyle

On procède suivant le mode opératoire décrit dans l'exemple 12 (étape 12.2.). A partir de 1,10 g (3,95 mmoles) de 4-cyanométhyl-4-isobutyl-pipéridine-1-carboxylate de *tert-*butyle et de 1,16 g (19,75 mmoles) de Nickel de Raney dans 13 mL de méthanol sous atmosphère d'hydrogène (70 psi) à 45°C et après chromatographie sur colonne de gel de silice en éluant avec un mélange 96/4/0,4 de dichlorométhane, de méthanol et d'ammoniaque 28%, on obtient 0,50 g de produit attendu sous forme d'huile.
LC-MS : M+H = 285
RMN ¹H (CDCl3) δ (ppm) : 3,50-3,30 (m, 4H) ; 2,45 (m, 2H) ; 1,70 (m, 1H) ; 1,60 (m, 4H) ; 1,40 (s, 9H) ; 1,35 (m, 4H) ; 1,25 (m, 2H) ; 0,90 (d, 6H).

### 19.4. 4-Isobutyl-4-[2-(3-méthylcarbamoyl-isoxazol-5-ylméthoxycarbonylamino)-éthyl]-pipéridine-1-carboxylate de tert-butyle

On procède suivant le mode opératoire décrit dans l'exemple 12 (étape 12.3.). A partir de 0.49 g (1,73 mmoles) de 4-(2-amino-éthyl)-4-isobutyl-pipéridine-1-carboxylate de *tert-*butyle, de 0,61 g (1,91 mmoles) de 4-nitro-phényl-carbonate de 3-(méthylcarbamoyl)-isoxazol-5-ylméthyle obtenu à l'étape 9.2., de 450 µL (2,60 mmoles) de N,N-diisopropyléthylamine et de 0,10 g (0,87 mmole) de N,N-diméthylaminopyridine dans 17 mL de 1,2-dichloroéthane, on obtient 0,73 g de produit attendu sous forme de cire.
LC-MS : M+H = 467
RMN ¹H (DMSO) δ (ppm) : 8,70 (large s, 1H) ; 7,40 (m, 1H) ; 6,80 (s, 1H) ; 5,20 (s, 2H) ; 3,40-3,15 (m, 4H) ; 3,00 (m, 2H) ; 2,80 (d, 3H) ; 1,70 (m, 1H) ; 1,50 (m, 2H) ; 1,40 (s, 9H) ; 1,30 (m, 4H) ; 1,20 (m, 2H) ; 0,90 (d, 6H).

### 19.5. [2-(4-Isobutyl-pipéridin-4-yl)-ethyl]-carbamate de 3-méthylcarbamoyl-isoxazol-5-ylméthyle

On procède suivant le mode opératoire décrit dans l'exemple 11 (étape 11.2.). A partir de 0,71 g (1,53 mmoles) de 4-éthyl-4-[2-(3-méthylcarbamoyl-isoxazol-5-ylméthoxycarbonylamino)-isobutyl]-pipéridine-1-carboxylate de *tert*-butyle, obtenu à l'étape 19.4., de 3,83 mL (15,30 mmoles) d'une solution d'acide chlorhydrique 4N dans le dioxane, et après trituration à l'éther, on obtient 0,60 g du produit attendu sous forme d'huile.
LC-MS : M+H = 367
RMN ¹H (DMSO) δ (ppm) : 8,70 (large s, 1H); 8,50 (large s, 1H) ; 7,40 (m, 1H) ; 6,80 (s, 1H) ; 5,20 (s, 2H) ; 3,00 (m, 6H) ; 2,75 (d, 3H) ; 1,70 (m, 1H) ; 1,50 (m, 6H) ; 1,30 (m, 2H) ; 0,90 (d, 6H).

### 19.6. {2-[1-(6-Chloro-quinoxalin-2-yl)-4-isobutyl-pipéridin-4-yl]-éthyl}-carbamate de 3-méthylcarbamoyl-isoxazol-5-ylméthyle

On procède suivant le mode opératoire décrit dans l'exemple 12 (étape 12.5.). A partir de 0,30 g (0,74 mmole) de chlorhydrate de [2-(4-éthyl-pipéridin-4-yl)-isobutyl]-carbamate de 3-méthylcarbamoyl-isoxazol-5-ylméthyle obtenu à l'étape précédente, de 0,22 g (1,12 mmole) de 2,6-dichloro-quinoxaline et 390 µL (2,23 mmoles) de N,N-diisopropyléthylamine en solution dans 2,50 mL d'acétonitrile, on obtient 0,29 g de produit attendu sous forme de poudre jaune.
PF(°C) : 144-146°C, LC-MS : M+H = 530
RMN ¹H (DMSO) δ (ppm) : 8,80 (s, 1H) ; 8,70 (m, 1H) ; 7,90 (s, 1H) ; 7,60 (s, 2H) ; 7,40 (m, 1H) ; 6,80 (s, 1H) ; 5,20 (s, 2H) ; 3,90 (m, 2H) ; 3,70 (m, 2H) ; 3,10 (m, 2H) ; 2,80 (m, 3H) ; 1,80 (m, 1H) ; 1,60 -1,40 (m, 6H) ; 1,30 (m, 2H) ; 0,90 (d, 6H).

### Exemple 20 (composé N°62)

### {2-[4-Hydroxy-1-(4-trifluorométhyl-pyrimidin-2-yl)-pipéridin-4-yl]-éthyl}-carbamate de 3-méthylcarbamoyl-isoxazol-5-ylméthyle

### 20.1. 4-Ethoxycarbonylméthyl-4-hydroxy-pipéridine-1-carboxylate de benzyle

A une solution de 5,00 g (56,75 mmoles) d'acétate d'éthyle dans 140 mL de diéthyl éther, on ajoute lentement et à -78°C sous argon, 28,38 mL (56,75 mmoles) d'une solution de diisopropylamidure de lithium (2N). Après 30 minutes d'agitation, on ajoute au goutte à goutte et à -78°C sous argon, 12,57 g (53,91 mmoles) de 4-oxo-pipéridine-1-carboxylate de benzyle en solution dans 140 mL de diéthyl éther. Le milieu réactionnel est ensuite agité à température ambiante pendant 2 heures. On ajoute de l'acétate d'éthyle et le milieu est refroidit par un bain de glace/eau puis on ajoute une solution saturée en chlorure d'ammonium. On sépare la phase aqueuse, on l'extrait trois fois avec de l'acétate d'éthyle, on lave les phases organiques réunies avec une solution aqueuse saturée en chlorure de sodium, on les sèche sur sulfate de sodium et on concentre le filtrat sous pression réduite. Le résidu est chromatographié sur gel de silice en éluant avec un mélange 85/15 de cyclohexane et d'acétate d'éthyle et on obtient 13,00 g de produit pur sous forme d'huile.
LC-MS : M+H = 322
RMN ¹H (DMSO) δ (ppm) : 7,50-7,30 (m, 5H) ; 5,10 (s, 2H) ; 4,70 (s, 1H) ; 4,10 (q, 2H) ; 3,70 (m, 2H) ; 3,20 (large s, 2H) ; 2,40 (s, 2H) ; 1,60 (m, 4H) ; 1,20 (t, 3H).

### 20.2. Bromhydrate de (4-Hydroxy-pipéridin-4-yl)-acétate d'éthyle

A une solution de 2,00g (6,22 mmoles) de 4-éthoxycarbonylméthyl-4-hydroxy-pipéridine-1-carboxylate de benzyle, obtenu à l'étape précédente, dans 31 mL de dichlorométhane, refroidie par un bain de glace/eau, on ajoute lentement 5,46 mL (31,12 mmoles) d'une solution d'acide bromhydrique 5,7N dans l'acide acétique. On poursuit l'agitation à température ambiante pendant 2 heures. On ajoute 50 mL de toluène et on évapore à sec. Le résidu est trituré dans l'éther. Après filtration sur fritté, on obtient 1,50 g du produit sous forme de bromhydrate.
RMN ¹H (DMSO) δ (ppm) : 8,45 (large s, 2H) ; 4,10 (q, 2H) ; 3,40 (large s, 1H) ; 3,10 (m, 4H) ; 2,40 (m, 2H) ; 1,80 (m, 4H) ; 1,20 (t, 3H).

### 20.3. [4-Hydroxy-1-(4-trifluorométhyl-pyrimidin-2-yl)-pipéridin-4-yl]-acétate d'éthyle

On procède suivant le mode opératoire décrit dans l'exemple 12 (étape 12.5.). A partir de 1,21 g (4,51 mmoles) de bromhydrate de (4-hydroxy-pipéridin-4-yl)-acétate d'éthyle, obtenu à l'étape précédente, de 0,90 g (4,96 mmoles) de 2-chloro-4-trifluoromethyl-pyrimidine et 1,65 mL (9,48 mmoles) de N,N-diisopropyléthylamine en solution dans 10 mL d'acétonitrile, et après purification par chromatographie sur gel de silice en éluant avec un mélange 99/1/0,1 de dichlorométhane, de méthanol et d'ammoniaque 28%, on obtient 1,33 g de produit attendu sous forme de cire.
RMN ¹H (CDCl3) δ (ppm) : 8,40 (d, 1H) ; 6,60 (d, 2H) ; 4,45 (m, 2H) ; 4,10 (q, 2H) ; 3,65 (large s, 1H) ; 3,40-3,20 (m, 2H) ; 2,40 (s, 2H) ; 1,75 (m, 2H) ; 1,60-1,30 (m, 2H) ; 1,20 (t, 3H).

### 20.4. 4-(2-Hydroxy-éthyl)-1-(4-trifluorométhyl-pyrimidin-2-yl)-pipéridin-4-ol

A une solution de 1,30 g de [4-hydroxy-1-(4-trifluorométhyl-pyrimidin-2-yl)-pipéridin-4-yl]-acétate d'éthyle, obtenu à l'étape précédente, dans 39 mL de tétrahydrofurane, on additionne par portions et à -10°C, 0,15 g d'hydrure de lithium et d'aluminium. On laisse ensuite agiter à température ambiante pendant 1 heure. On refroidit le milieu réactionnel à environ 0°C puis on additionne lentement 15 mL d'une solution aqueuse d'hydroxyde de sodium (1M). On laisse sous agitation à température ambiante pendant 30 minutes puis on additionne par petites portions du sulfate de sodium humide. On sépare les sels par filtration sur célite puis on laisse décanter. On extrait la phase aqueuse avec de l'acétate d'éthyle, on sèche les phases organiques réunies sur sulfate de sodium et on concentre le filtrat sous pression réduite. On purifie le résidu ainsi obtenu par chromatographie sur gel de silice en éluant avec un mélange 97/3/0,3 de dichlorométhane, de méthanol et d'ammoniaque 28%. On obtient 0,64 g de produit attendu sous forme de cire.
RMN ¹H (CDCl3) δ (ppm) : 8,40 (d, 1H) ; 6,60 (d, 1H) ; 4,45 (m, 2H) ; 4,00 (q, 2H) ; 3,60-3,30 (m, 2H) ; 3,20 (large s, 1H) ; 2,50 (large s, 1H) ; 1,80 (m, 4H) ; 1, 65-1, 40 (m, 2H).

### 20.5. 4,5,6,7-Tétrachloro-2-{2-[4-hydroxy-1-(4-trifluorométhyl-pyrimidin-2-yl)-pipéridin-4-yl]-éthyl}-isoindole-1,3-dione

On procède suivant le mode opératoire décrit dans l'exemple 1 (étape 1.3.). A partir de 0,63 g (2, 16 mmoles) de 4-(2-hydroxy-éthyl)-1-(4-trifluorométhyl-pyrimidin-2-yl)-pipéridin-4-ol, obtenu à l'étape précédente, de 0,62 g (2,38 mmoles) de triphénylphosphine, de 0,69 g (2,38 mmoles) de 4,5,6,7-tétrachloro-isoindole-1,3-dione et d'une solution de 0,41 g (2,38 mmoles) de diéthylazodicarboxylate (DEAD) dans 10 mL de tétrahydrofurane, on obtient 0,38 g du produit attendu.
RMN ¹H (CDCl3) δ (ppm) : 8,40 (d, 1H) ; 6,70 (d, 1H) ; 4,50 (m, 2H) ; 3,90 (m, 2H) ; 3,60-3,30 (m, 2H) ; 1,90 (m, 2H) ; 1,70-1,40 (m, 4H).

### 20.6. 4-(2-Amino-éthyl)-1-(4-trifluorométhyl-pyrimidin-2-yl)-pipéridin-4-ol

On procède suivant le mode opératoire décrit dans l'exemple 1 (étape 1.4.). A partir de 0,38 g (0,68 mmoles) de 4,5,6,7-tétrachloro-2-{2-[4-hydroxy-1-(4-trifluorométhyl-pyrimidin-2-yl)-pipéridin-4-yl]-éthyl}-isoindole-1,3-dione), obtenu à l'étape précédente et dissout dans un mélange acétonitrile/tétrahydrofurane/eau (2/1/1) et de 0,18 g (3,06 mmoles) d'éthylène diamine dans 4,50 mL d'éthanol et après chromatographie sur gel de silice en éluant avec un mélange 90/10/1 de dichlorométhane, de méthanol et d'ammoniaque 28%, on obtient 0,15 g de produit attendu sous forme de cire.
RMN ¹H (CDCl₃) δ (ppm) : 8,40 (d, 1H) ; 6,60 (d, 1H) ; 4,50 (m, 2H) ; 3,50-3,20 (m, 2H) ; 3,20 (m, 2H) ; 2,90-2,60 (large s, 2H) ; 1,70 (m, 2H) ; 1,50-1,30 (m, 4H).

### 20.7. {2-[4-Hydroxy-1-(4-trifluorométhyl-pyrimidin-2-yl)-pipéridin-4-yl]-éthyl}-carbamate de 3-méthylcarbamoyl-isoxazol-5-ylméthyle

On procède suivant le mode opératoire décrit dans l'exemple 12 (étape 12.3.). A partir de 0,13 g (0,45 mmole) de 4-(2-amino-éthyl)-1-(4-trifluorométhyl-pyrimidin-2-yl)-pipéridin-4-ol, obtenu à l'étape précédente, de 0,187 g (0,58 mmole) de 4-nitro-phényl-carbonate de 3-(méthylcarbamoyl)-isoxazol-5-ylméthyle obtenu à l'étape 9.1., de 200 µL (1,12 mmole) de N,N-diisopropyléthylamine et de 0,027 g (0,22 mmole) de N,N-diméthylaminopyridine dans 2,20 mL de 1,2-dichloroéthane, on obtient 0,165 g de produit attendu sous forme poudre.
PF(°C) : 138-140°C, LC-MS : M+H = 473
RMN ¹H (DMSO) δ (ppm) : 8,70 (m, 2H) ; 7,35 (t, 1H) ; 6,95 (m, 1H) ; 6,80 (s, 1H) ; 5,20 (m, 2H) ; 4,50 (s, 1H) ; 4,30 (m, 2H) ; 3,40 (m, 2H) ; 3,20 (m, 2H) ; 2,80 (m, 3H) ; 1,90 (m, 4H) ; 1,45 (m, 2H).

### Exemple 21 (composé N°29)

### {2-[1-(4'-Fluoro-biphényl-4-yl)-pipéridin-4-yl]-éthyl}-carbamate de 3-méthylcarbamoyl-isoxazol-5-ylméthyle

### 21.1. {2-[1-(4-Bromo-phényl)-pipéridin-4-yl]-éthyl}-carbamate de tert-butyle

Sous atmosphère inerte, on introduit 6,19 g (21,90 mmoles) de 1-bromo-4-iodo-benzene, 5,00 g (21,90 mmoles) de (2-pipéridin-4-yl-éthyl)-carbamate de *tert*-butyle, 9,98 g (60,66 mmoles) de carbonate de césium et 0,54 g (0,88 mmole) de BINAP (2,2'-bis(diphénylphosphino)-1,1'-binaphthyle) en suspension dans 100 mL de toluène. On ajoute ensuite 0,098 g (0,44 mmole) de diacétate de palladium. On porte ensuite le mélange réactionnel au reflux pendant 6 heures. On ajoute 0,045 g (0,20 mmole) de diacétate de palladium et 0,25 g (0,40 mmole) de BINAP et on laisse agiter au reflux pendant 12 heures. On laisse refroidir le milieu réactionnel et on ajoute de l'acétate d'éthyle et de l'eau. On sépare la phase aqueuse, on l'extrait deux fois avec de l'acétate d'éthyle, on lave les phases organiques réunies avec une solution aqueuse saturée en chlorure de sodium et on sèche sur sulfate de sodium. Après évaporation du solvant, le résidu obtenu est purifié par chromatographie sur gel de silice en éluant avec un mélange 98/2/0,2 de dichlorométhane, de méthanol et d'ammoniaque 28% et on obtient 1,66 g de produit attendu sous forme de solide blanc.
PF(°C) : 120-122°C, LC-MS : M+H = 384
RMN ¹H (DMSO) δ (ppm): 7,30 (d, 2H) ; 6,90 (d, 2H) ; 6,70 (large s, 1H) ; 3,70 (m, 2H) ; 2,95 (m, 2H) ; 2,60 (m, 2H) ; 1,70 (m, 2H) ; 1,40 (s, 9H) ; 1,35 (m, 3H) ; 1,20 (m, 2H).

### 21.2. {2-[1-(4'-Fluoro-biphényl-4-yl)-pipéridin-4-yl]-éthyl}-carbamate de tert-butyle

On procède suivant le mode opératoire décrit dans l'exemple 10 (étape 10.5.). A partir de 1,50 g (3,91 mmoles) de {2-[1-(4-bromo-phényl)-pipéridin-4-yl]-éthyl}-carbamate de *tert-*butyle, obtenu à l'étape précédente, de 0,66 g (4,70 mmoles) d'acide 4-fluorophénylboronique, de 3,82 g (11,74 mmoles) de carbonate de césium dans 40 mL d'un mélange 9/1 de tétrahydrofurane et d'eau et de 0,32 g (0,39 mmole) de PdCl₂dppf.CH₂Cl₂, et après purification par chromatographie sur gel de silice en éluant avec un mélange 98/2/0,2 de dichlorométhane, de méthanol et d'ammoniaque 28%, on obtient 1,23 g de produit attendu sous forme de solide blanc.
PF(°C) : 173-175°C, LC-MS : M+H = 399
RMN ¹H (DMSO) δ (ppm) : 7,60 (m, 2H) ; 7,50 (d, 2H) ; 7,20 (m, 2H) ; 6,95 (d, 2H) ; 6,75 (large s, 1H) ; 3,70 (m, 2H) ; 2,95 (m, 2H) ; 2,70 (m, 2H) ; 1,70 (m, 2H) ; 1,40 (s, 9H) ; 1,35 (m, 3H) ; 1,20 (m, 2H).

### 21.3. 2-[1-(4'-Fluoro-biphényl-4-yl)-pipéridin-4-yl]-éthylamine

On procède suivant le mode opératoire décrit dans l'exemple 14 (étape 14.3.). A partir de 1, 10 g (2,76 mmoles) de {2-[1-(4'-fluoro-biphényl-4-yl)-pipéridin-4-yl]-éthyl}-carbamate de *tert*-butyle, obtenu à l'étape précédente, de 2,11 mL (25,09 mmoles) d'acide trifluoroacétique dans 20 mL de dichlorométhane et après basification par un traitement à la soude 35%, extraction au dichlorométhane, séchage sur sulfate de sodium et évaporation à sec, on obtient 0,74 g de produit attendu sous forme d'un solide amorphe de couleur orange.
LC-MS : M+H = 299
RMN ¹H (DMSO) δ (ppm): 7,60 (m, 2H) ; 7,50 (d, 2H) ; 7,25 (m, 2H) ; 7,00 (d, 2H) ; 3,75 (m, 2H) ; 2,85 (m, 2H) ; 2,75 (m, 2H) ; 1,70 (m, 2H) ; 1,50 (m, 1H) ; 1,35 (m, 2H) ; 1,25 (m, 2H).

### 21.4. {2-[1-(4'-Fluoro-biphényl-4-yl)-pipéridin-4-yl]-éthyl}-carbamate de 3-méthylcarbamoyl-isoxazol-5-ylméthyle

On procède suivant le mode opératoire décrit dans l'exemple 12 (étape 12.3.). A partir de 0,27 g (0,90 mmole) de 2-[1-(4'-fluoro-biphényl-4-yl)-pipéridin-4-yl]-éthylamine, obtenu à l'étape précédente, de 0,29 g (0,90 mmole) de 4-nitro-phényl-carbonate de 3-(méthylcarbamoyl)-isoxazol-5-ylméthyle obtenu à l'étape 9.1., de 320 µL (1,81 mmole) de N,N-diisopropyléthylamine dans 10 mL de 1,2-dichloroéthane et après trituration dans l'éther, on obtient 0,26 g de produit attendu sous forme poudre blanche.
PF(°C) : 212-215°C, LC-MS : M+H = 481
RMN ¹H (DMSO) δ (ppm) : 8,70 (large s, 1H) ; 7,60 (m, 2H) ; 7,50 (m, 2H) ; 7,45 (large s, 1H) ; 7,25 (m, 2H) ; 7,00 (m, 2H) ; 6,90 (s, 1H) ; 5,20 (s, 2H) ; 3,75 (m, 2H) ; 3,10 (m, 2H) ; 2,80 (s, 3H) ; 2,70 (m, 2H) ; 1,75 (m, 2H) ; 1,45 (m, 3H) ; 1,25 (m, 2H).

### Exemple 22 (composé N°79)

### (+/-)-[1-(4-Chloro-phthalazin-1-yl)-pyrrolidin-3-ylméthyl]-carbamate de 3-méthylcarbamoyl-isoxazol-5-ylméthyle

### 22.1 (+/-)-3-[(3-Méthylcarbamoyl-isoxazol-5-ylméthoxycarbonylamino)-méthyl]-pyrrolidine-1-carboxylate de tert-butyle

On procède suivant le mode opératoire décrit dans l'exemple 1 (étape 1.6.). A partir de 2,00 g (9,99 mmoles) de **(+/-)-**1-boc-3-(aminométhyl) pyrrolidine (commerciale), 3,52 g (10,98 mmoles) de 4-nitro-phényl-carbonate de 3-(méthylcarbamoyl)-isoxazol-5-ylméthyle obtenu à l'étape 9.2., de 2,61 mL (14,98 mmoles) de N,N-diisopropyléthylamine et 0,61 g (4,99 mmoles) de dimétylaminopyridine dans 100 mL de 1,2-dichloroéthane et après purification par chromatographie sur gel de silice en éluant avec un mélange de 100/0/0 à 98/2/0,2 de dichlorométhane, de méthanol et d'ammoniaque 28%, on obtient 1,32 g de produit attendu sous forme de cire.
LC-MS : M+H = 383
RMN ¹H (DMSO) δ (ppm) : 8,70 (sl, 1H); 7,00 (sl, 1H); 6,80 (s, 1H) ; 5,25 (s, 2H) ; 3,40 (m, 3H) ; 3,00 (m, 3H) ; 2,80 (s, 3H) ; 2,20 (m, 1H) ; 1,90 (m, 1H) ; 1,60 (m, 1H) ; 1,40 (s, 9H).

### 22.2 (+/-)-Chlorydrate de pyrrolidin-3-ylméthyl-carbamate de 3-méthylcarbamoyl-isoxazol-5-ylméthyle

On procède suivant le mode opératoire décrit dans l'exemple 11 (étape 11.2.). A partir de 1,30 g (3,40 mmoles) de 3-[(3-méthylcarbamoyl-isoxazol-5-ylméthoxycarbonylamino)-méthyl]-pyrrolidine-1-carboxylate de tert-butyle, 4,25 mL d'acide chlorydrique en solution à 4N dans le dioxane et après trituration dans l'éther diéthylique, on obtient 0.82 g d'une poudre blanche.
PF(°C) : 187-189°C, LC-MS : M+H = 283
RMN ¹H (DMSO) δ (ppm) : 8,70 (sl, 1H) ; 8,00 (m, 2H) ; 6,80 (m, 1H); 5,25 (s, 2H); 3,60 (m, 1H); 3,45 (m, 1H); 3,30 (m, 1H); 3,10 (m, 1H); 2,90 (m, 2H); 2,80 (s, 3H); 2,50 (m, 1H); 2,05 (m, 1H); 1,70 (m, 1H).

### 22.3 (+/-)-[1-(4-Chloro-phthalazin-1-yl)-pyrrolidin-3-ylméthyl]-carbamate de 3-méthylcarbamoyl-isoxazol-5-ylméthyle

On procède suivant le mode opératoire décrit dans l'exemple 12 (étape 12.5.). A partir de 0,19 g (0,60 mmole) de chlorydrate de pyrrolidin-3-ylméthyl-carbamate de 3-méthylcarbamoyl-isoxazol-5-ylméthyle, 0,17 g (0,89 mmmole) de 1,4-dichloro-phthalazine et 0,21 mL (0,15 mmole) de N,N-diisopropyléthylamine (15 min, 150°C) et après purification par chromatographie sur gel de silice en éluant avec un mélange de 100/0/0 à 98/2/0,2 de dichlorométhane, de méthanol et d'ammoniaque 28%, on obtient 0,09 g de produit attendu sous forme de poudre blanche.
PF(°C) : 159-161°C, LC-MS : M+H = 445
RMN ¹H (DMSO) δ (ppm) : 8,80 (m, 1H) ; 8,40 (m, 1H) ; 8,15 (m, 1H) ; 8,05 (m, 2H) ; 7,80 (m, 1H) ; 6,80 (s, 1H) ; 5,25 (s, 2H) ; 3,65 (m, 1H) ; 3,50 (m, 2H) ; 3,30 (m, 1H) ; 3,20 (m, 2H) ; 2,80 (m, 4H) ; 2,05 (m, 1H) ; 1,70 (m, 1H).

### Exemple 23 (composé N°82)

### {2-[1-(4-Trifluorométhyl-pyrimidin-2-yl)-azétidin-3-yl]-éthyl}-carbamate de 3-carbamoyl-isoxazol-5-ylméthyle

### 23.1 {2-[1-(4-Trifluorométhyl-pyrimidin-2-yl)-azétidin-3-yl]-éthyl}-carbamate de tert-butyle

On procède suivant le mode opératoire décrit dans l'exemple 14 (étape 14.4.). A partir de 3,00 g (12,57 mmoles) de (2-azétidin-3-yl-éthyl)-carbamate de *tert*-butyle(commercial), de 3,00 g (16,47 mmoles) de 2-chloro-4-trifluorométhyl-pyrimidine et 7,73 mL (44,35 mmoles), on obtient 4,00 g de produit sous forme de poudre orange après purification par chromatographie sur gel de silice en éluant avec un mélange de 100/0/0 à 98/2/0,2 de dichlorométhane, de méthanol et d'ammoniaque 28%.
PF(°C) : 95-97°C

### 23.2 2-[1-(4-Trifluorométhyl-pyrimidin-2-yl)-azétidin-3-yl]-éthylamine

On procède suivant le mode opératoire décrit dans l'exemple 11 (étape 11.2.). A partir de 3,67 g (10,60 mmoles) de {2-[1-(4-trifluorométhyl-pyrimidin-2-yl)-azétidin-3-yl]-éthyl}-carbamate de *tert*-butyle et 10,60 mL (42,38 mmoles) d'une solution d'acide chlorydrique à 4N dans le dioxane, on obtient 1,75 g de produit sous forme d'huile jaune après purification par chromatographie sur gel de silice en éluant avec un mélange de 100/0/0 à 90/10/1 de dichlorométhane, de méthanol et d'ammoniaque 28%.
LC-MS : M+H = 247
RMN ¹H (CDCl₃) δ (ppm) : 8,50 (d, 1H) ; 6,80 (d, 1H) ; 4,30 (m, 2H); 3,90 (m, 2H); 2,85 (m, 1H); 2,75 (m, 2H); 1,85 (m, 2H); 1,30 (sl, 2H).

### 23.3 {2-[1-(4-Trifluorométhyl-pyrimidin-2-yl)-azetidin-3-yl]-éthyl}-carbamate de 3-carbamoyl-isoxazol-5-ylméthyle

On procède suivant le mode opératoire décrit dans l'exemple 1 (étape 1.6.). A partir de 0,30 g (1,22 mmole) de 2-[1-(4-trifluorométhyl-pyrimidin-2-yl)-azétidin-3-yl]-éthylamine, de 0,45 g (1,46 mmole) de 4-nitro-phényl-carbonate de 3-carbamoyl-isoxazol-5-ylméthyle décrit dans l'exemple 8 (étape 8.3.), de 0,53 mL (3,05 mmoles) de diisopropyléthylamine et de 0,07 g (0,61 mmole) de diméthylaminopyridine, on obtient 0,41 g de produit sous forme de poudre blanche après purification par chromatographie sur gel de silice en éluant avec un mélange de 100/0/0 à 98/2/0,2 de dichlorométhane, de méthanol et d'ammoniaque 28%.
PF(°C) : 163-165°C, LC-MS : M+H = 415
RMN ¹H (DMSO) δ (ppm) : 8,65 (d, 1H); 8,15 (sl, 1H); 7,85 (sl, 1H); 7,50 (tl, 1H); 7,05 (d, 1H); 6,80 (s, 1H); 5,20 (s, 2H); 4,20 (s, 2H); 3,70 (m, 2H); 3,10 (m, 2H); 2,75 (m, 1H); 1,80 (m, 2H).

### Exemple 24 (composé N°84)

### (-)-[1-(4-Chloro-pyrimidin-2-yl)-pyrrolidin-3-ylméthyl]-carbamate de 3-méthylcarbamoyl-isoxazol-5-ylméthyle

0.32 g de (+/-)-[1-(4-chloro-pyrimidin-2-yl)-pyrrolidin-3-ylméthyl]-carbamate de 3-méthylcarbamoyl-isoxazol-5-ylméthyle a été séparé par chromatographie préparative chirale HPLC (Chiralpak AD_20µm 50x220 mm) en éluant avec un mélange propanol/n-heptane dans les proportions 25/75 pour donner 0.070 g de produit ontenu sous forme de base.
t_{R} : 45 min.
PF(°C) : 114,4-118,3°C, LC-MS : M+H = 429
[α]^{20°C} - 9,88 (c = 0.333, DMSO, 589nm)
RMN ¹H (DMSO) δ (ppm) : 8,70 (sl, 1H) ; 8,60 (sl, 1H) ; 8,05 (m, 1H) ; 7,00 (m, 1H) ; 6,80 (s, 1H) ; 5,25 (s, 2H) ; 3,45 (m, 2H) ; 3,30 (m, 3H) ; 3,10 (m, 1H) ; 2,80 (m, 3H) ; 2,50 (m, 1H); 2,00 (m, 1H) ; 1,70 (m, 1H).

### Exemple 25 (composé N°85)

### (+)-[1-(4-Chloro-pyrimidin-2-yl)-pyrrolidin-3-ylméthyl]-carbamate de 3-méthylcarbamoyl-isoxazol-5-ylméthyle

0.32 g de (+/-)-[1-(4-chloro-pyrimidin-2-yl)-pyrrolidin-3-ylméthyl]-carbamate de 3-méthylcarbamoyl-isoxazol-5-ylméthyle a été séparé par chromatographie préparative chirale HPLC (Chiralpak AD_20µm 50x220 mm) en éluant avec un mélange propanol/n-heptane dans les proportions 25/75 pour donner 0.090 g de produit ontenu sous forme de base.
t_{R} : 52 min.
PF(°C) : 114.4-118.3°C, LC-MS : M+H = 429
[**α**]^{20°C} + 9,55(c = 0.222, DMSO, 589nm)
RMN ¹H (DMSO) δ (ppm) : 8,70 (sl, 1H) ; 8,60 (sl, 1H) ; 8,05 (m, 1H) ; 7,00 (m, 1H) ; 6,80 (s, 1H) ; 5,25 (s, 2H) ; 3,45 (m, 2H) ; 3,30 (m, 3H) ; 3,10 (m, 1H) ; 2,80 (m, 3H) ; 2,50 (m, 1H); 2,00 (m, 1H) ; 1,70 (m, 1H).

Le tableau 1 qui suit illustre les structures chimiques et les propriétés physiques de quelques composés selon l'invention.

Dans ce tableau :
- tous les composés sont sous forme de base ;
- la colonne « PF (°C) » renseigne les points de fusion des produits en degrés Celsius (°C) ;
- le terme « Rot. » indique la propriété lévogyre ou déxtrogyre du composé.
- La liaison en pointillé «----» représente la liaison reliant le substitutant au reste de la molécule.

**Tableau 1**

| | | | | | | | | |
|---|---|---|---|---|---|---|---|---|
| 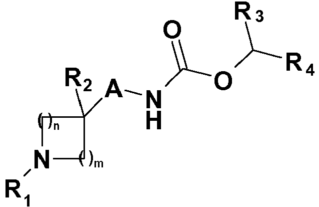 | | | | | | | | |

| **N°** | **R₁** | **m** | **n** | **A** | **R₂** | **R₃** | **R₄** | **PF (°C**) |
|---|---|---|---|---|---|---|---|---|
| **1.** | 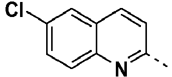 | **2** | **2** | **(CH₂)₂** | **H** | **H** | 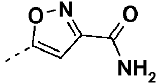 | 220-222°C |
| **2.** | 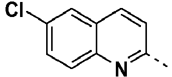 | **2** | **2** | **(CH₂)₂** | **H** | **H** | 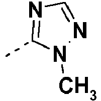 | 122-124°C |
| **3.** | 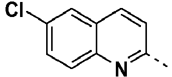 | **2** | **2** | **(CH₂)₂** | **H** | **H** | 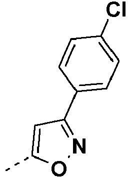 | 139-140°C |
| **4.** | 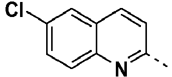 | **2** | **2** | **(CH₂)₂** | **H** | **H** | 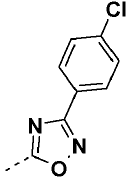 | 134-136°C |
| **5.** | 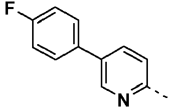 | **2** | **2** | **(CH₂)₂** | **H** | **H** | 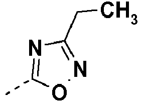 | 110-112°C |
| **6.** | 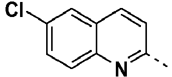 | **2** | **2** | **(CH₂)₂** | **H** | **H** | 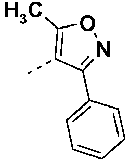 | 110-112°C |
| **7.** | 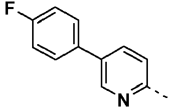 | **2** | **2** | **(CH₂)₂** | **H** | **H** | 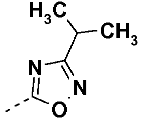 | 96-98°C |
| **8.** | 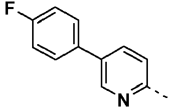 | **2** | **2** | **(CH₂)₂** | **H** | **H** | 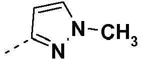 | 154-156°C |
| **9.** | 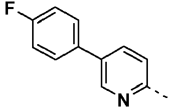 | **2** | **2** | **(CH₂)₂** | **H** | **H** | 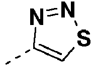 | 139-141°C |
| **10.** | 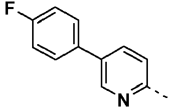 | **2** | **2** | **(CH₂)₂** | **H** | **H** | 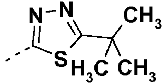 | 139-140°C |
| **11.** | 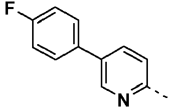 | **2** | **2** | **(CH₂)₂** | **H** | **H** | 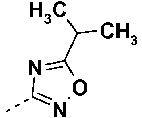 | 112-113°C |
| **12.** | 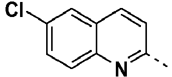 | **2** | **2** | **(CH₂)₂** | **H** | **H** | 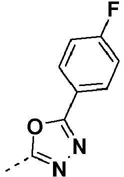 | 169-170°C |
| **13.** | 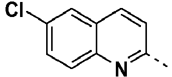 | **2** | **2** | **(CH₂)₂** | **H** | **H** | 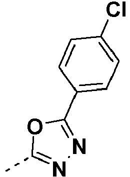 | 152-153°C |
| **14.** | 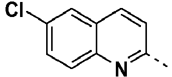 | **2** | **2** | **(CH₂)₂** | **H** | **H** | 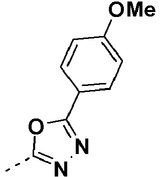 | 154-156°C |
| **15.** | 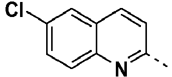 | **2** | **2** | **(CH₂)₂** | **H** | **H** | 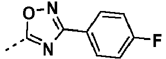 | 160-162°C |
| **16.** | 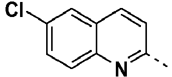 | **2** | **2** | **(CH₂)₂** | **H** | **H** | 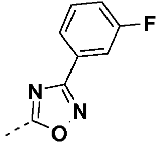 | 134-136°C |
| **17.** | 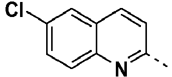 | **2** | **2** | **(CH₂)₂** | **H** | **H** | 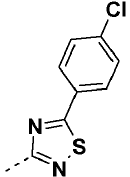 | 191-193°C |
| **18.** | 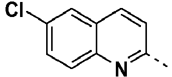 | **2** | **2** | **(CH₂)₂** | **H** | **H** | 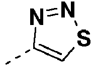 | 146-148°C |
| **19.** | 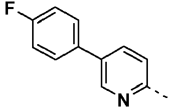 | **2** | **2** | **(CH₂)₂** | **H** | **H** | 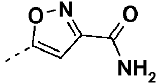 | 202-204°C |
| **20.** | 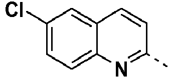 | **2** | **2** | **(CH₂)₂** | **H** | **H** | 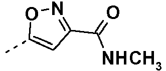 | 200-202°C |
| **21**. | 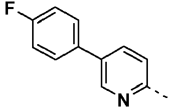 | **2** | **2** | **(CH₂)₂** | **H** | **H** | 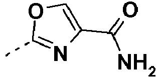 | 206-208°C |
| **22.** | 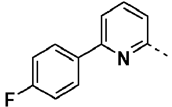 | **2** | **2** | **(CH₂)₂** | **H** | **H** | 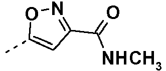 | 155,5-157,5 °C |
| **23.** | 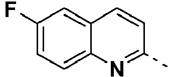 | **2** | **2** | **(CH₂)₂** | **H** | **H** | 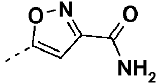 | 200-202°C |
| **24.** | 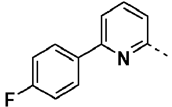 | **2** | **2** | **(CH₂)₂** | **H** | **H** | 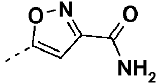 | 172-174°C |
| **25.** | 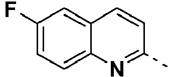 | **2** | **2** | **(CH₂)₂** | **H** | **H** | 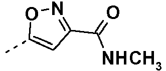 | 180-182°C |
| **26.** | 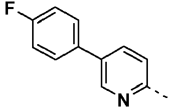 | **2** | **2** | **(CH₂)₂** | **H** | **H** | 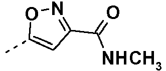 | 191-193°C |
| **27.** | 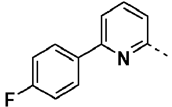 | **2** | **2** | **(CH₂)₂** | **H** | **H** | 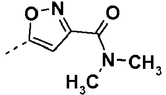 | 81 - 83 °C |
| **28.** | 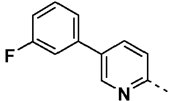 | **2** | **2** | **(CH₂)₂** | **H** | **H** | 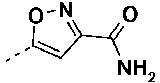 | 172-176°C |
| **29.** | 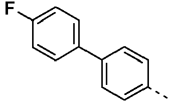 | **2** | **2** | **(CH₂)₂** | **H** | **H** | 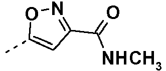 | 212-215°C |
| **30.** | 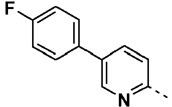 | **2** | **2** | **(CH₂)₂** | **H** | **H** | 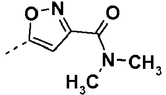 | 149-151°C |
| **31**. | 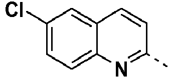 | **2** | **2** | **(CH₂)₂** | **H** | **H** | 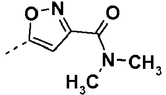 | 114-116°C |
| **32.** | 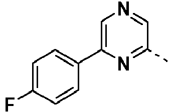 | **2** | **2** | **(CH₂)₂** | **H** | **H** | 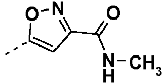 | 150-152°C |
| **33.** | 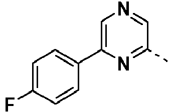 | **2** | **2** | **(CH₂)₂** | **H** | **H** | 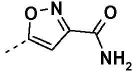 | 165-167°C |
| **34.** | 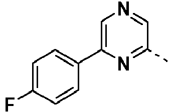 | **2** | **2** | **(CH₂)₂** | **H** | **H** | 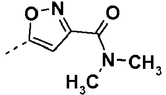 | 100-102°C |
| **35.** | 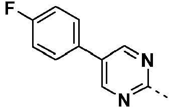 | **2** | **2** | **(CH₂)₂** | **H** | **H** | 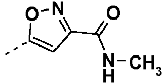 | 209-211°C |
| **36.** | 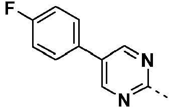 | **2** | **2** | **(CH₂)₂** | **H** | **H** | 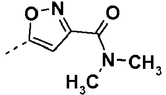 | 147-149°C |
| **37.** | 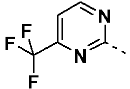 | **2** | **2** | **(CH₂)₂** | **H** | **H** | 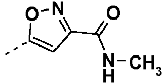 | 164-166°C |
| **38.** | 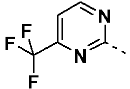 | **2** | **2** | **(CH₂)₂** | **H** | **H** | 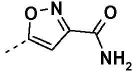 | 165-167°C |
| **39.** | 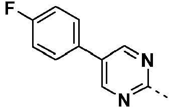 | **2** | **2** | **(CH₂)₂** | **H** | **H** | 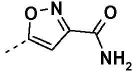 | 209-211°C |
| **40.** | 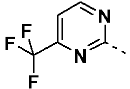 | **2** | **2** | **(CH₂)₂** | **H** | **H** | 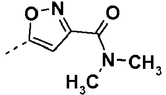 | 100-102°C |
| **41.** | 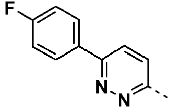 | **2** | **2** | **(CH₂)₂** | **H** | **H** | 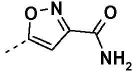 | 198-200°C |
| **42.** | 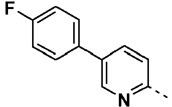 | **2** | **2** | **(CH₂)₂** | **H** | **H** | 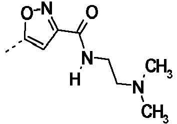 | 143-145°C |
| **43.** | 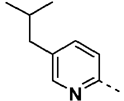 | **2** | **2** | **(CH₂)₂** | **H** | **H** | 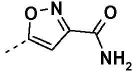 | 168-170°C |
| **44.** | 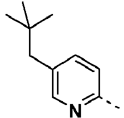 | **2** | **2** | **(CH₂)₂** | **H** | **H** | 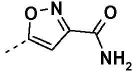 | 173-175°C |
| **45.** | 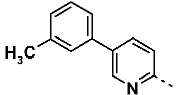 | **2** | **2** | **(CH₂)₂** | **H** | **H** | 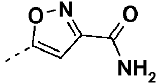 | 179-181°C |
| **46.** | 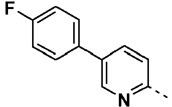 | **2** | **2** | **(CH₂)₂** | **H** | **H** | 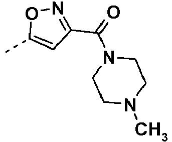 | 180-182°C |
| **47.** | 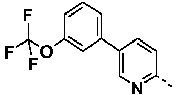 | **2** | **2** | **(CH₂)₂** | **H** | **H** | 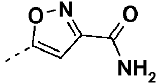 | 177-179°C |
| **48.** | 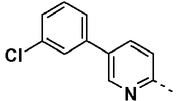 | **2** | **2** | **(CH₂)₂** | **H** | **H** | 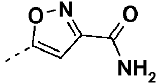 | 179-181°C |
| **49.** | 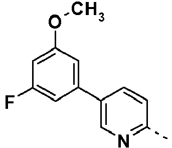 | **2** | **2** | **(CH₂)₂** | **H** | **H** | 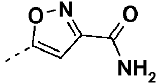 | 175-177°C |
| **50.** | 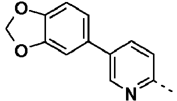 | **2** | **2** | **(CH₂)₂** | **H** | **H** | 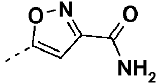 | 206-208°C |
| **51.** | 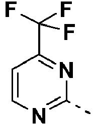 | **2** | **2** | **CH₂** | **F** | **H** | 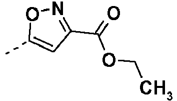 | 136-138°C |
| **52.** | 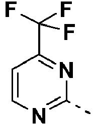 | **2** | **2** | **(CH₂)₃** | **H** | **H** | 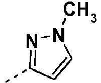 | 72-74°C |
| **53.** | 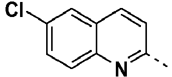 | **2** | **2** | **(CH₂)₂** | **H** | **H** | 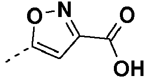 | 180-182°C |
| **54.** | 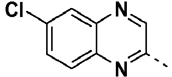 | **2** | **2** | **CH₂** | **H** | **H** | 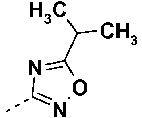 | 136-138°C |
| **55.** | 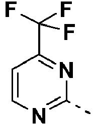 | **2** | **2** | **CH₂** | **H** | **H** | 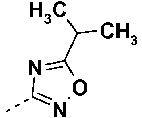 | 104-106°C |
| **56.** | 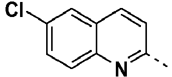 | **2** | **2** | **CH₂** | **F** | **H** | 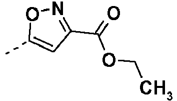 | 132-134°C |
| **57.** | 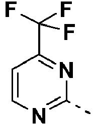 | **2** | **2** | **CH₂** | **F** | **H** | 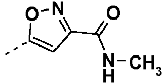 | 187-189°C |
| **58.** | 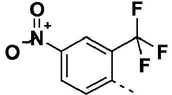 | **2** | **2** | **CH₂** | **H** | **H** | 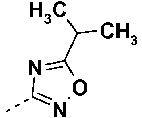 | Cire |
| **59.** | 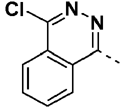 | **2** | **2** | **CH₂** | **H** | **H** | 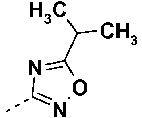 | 139-141°C |
| **60.** | 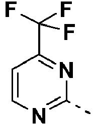 | **1** | **1** | **(CH₂)₂** | **N(CH₃)₂** | **H** | 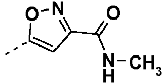 | 154-156°C |
| **61.** | 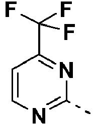 | **2** | **2** | **(CH₂)₂** | **CH₂CH₃** | **H** | 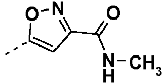 | 152-154°C |
| **62.** | 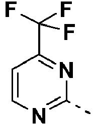 | **2** | **2** | **(CH₂)₂** | **OH** | **H** | 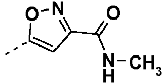 | 138-140°C |
| **63.** | 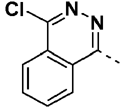 | **1** | **1** | **(CH₂)₂** | **N(CH₃)₂** | **CF₃** | 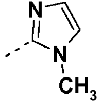 | 168-170°C Rot. (+/-) |
| **64.** | 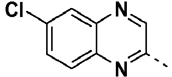 | **2** | **2** | **(CH₂)₂** | **CH₂CH₃** | **H** | 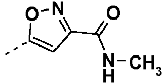 | 158-160°C |
| **65.** | 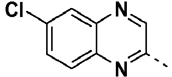 | **2** | **2** | **(CH₂)₂** | **CH₂CH(CH₃)₂** | **H** | 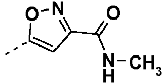 | 144-146°C |
| **66.** | 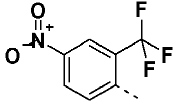 | **2** | **2** | **(CH₂)₂** | **CH₂CH(CH₃)₂** | **H** | 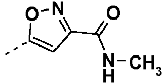 | 145-147°C |
| **67.** | 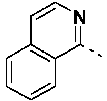 | **2** | **2** | **CH₂** | **H** | **H** | 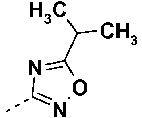 | 95-97°C |
| **68.** | 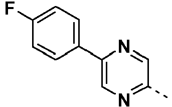 | **2** | **2** | **(CH₂)₂** | **H** | **H** | 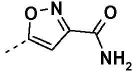 | 187-189°C |
| **69.** | 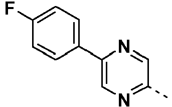 | **2** | **2** | **(CH₂)₂** | **H** | **H** | 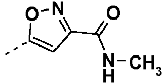 | 181,5-183,5°C |
| **70.** | 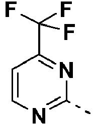 | **2** | **2** | **liaison** | **H** | **H** | 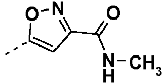 | 197-199°C |
| **71.** | 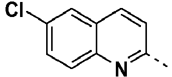 | **2** | **2** | **(CH₂)₂** | **H** | **H** | 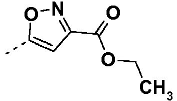 | 117-119°C |
| **72.** | 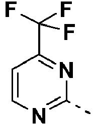 | **2** | **2** | **(CH₂)₂** | **CH₃** | **H** | 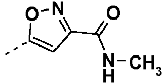 | 160-162°C |
| **73.** | 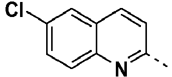 | **2** | **2** | **(CH₂)₂** | **H** | **CF₃** | 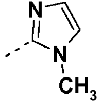 | 196-198°C Rot. (+/-) |
| **74.** | 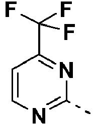 | **2** | **3** | **liaison** | **H** | **H** | 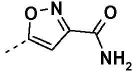 | 171-173°C Rot. (+/-) |
| **75.** | 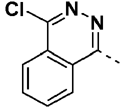 | **2** | **2** | **liaison** | **H** | **H** | 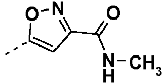 | 208-210°C |
| **76.** | 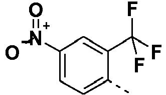 | **2** | **2** | **liaison** | **H** | **H** | 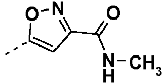 | 156-158°C |
| **77.** | 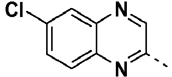 | **2** | **2** | **liaison** | **H** | **H** | 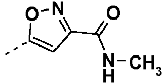 | 232-234°C |
| **78.** | 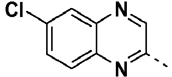 | **2** | **2** | **(CH₂)₂** | **CH₃** | **H** | 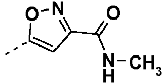 | 167-169°C |
| **79.** | 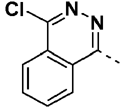 | **2** | **1** | **CH₂** | **H** | **H** | 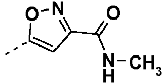 | 159-161°C Rot. (+/-) |
| **80.** | 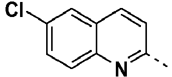 | **2** | **2** | **(CH₂)₂** | **H** | 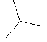 | 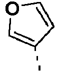 | 97-99°C Rot. (+/-) |
| **81.** | 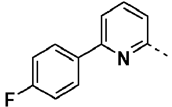 | **2** | **2** | **(CH₂)₂** | **H** | 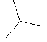 | 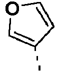 | 101-103°C Rot. (+/-) |
| **82.** | 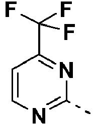 | **1** | **1** | **(CH₂)₂** | **H** | **H** | 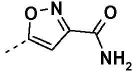 | 163-165°C |
| **83.** | 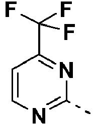 | **1** | **1** | **(CH₂)₂** | **H** | **H** | 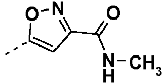 | 155-157°C |
| **84.** | 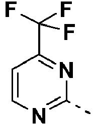 | **2** | **1** | **CH₂** | **H** | **H** | 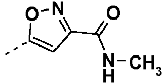 | 114,4-118,3°C Rot. (-) |
| **85.** | 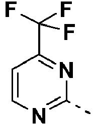 | **2** | **1** | **CH₂** | **H** | **H** | 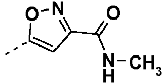 | 114,4-118,3°C Rot. (+) |

Le tableau 2 qui suit donne les résultats des analyses RMN ¹H, ainsi que les résultats des analyses LC-MS pour les composés du tableau 1. Dans ce tableau, la colonne TR indique le temps de rétention.

**Tableau 2**

| **N°** | **RMN ¹H** | **LC-MS** | | |
|---|---|---|---|---|
| | | **M+H** | **TR** | **Methode** |
| **1** | (DMSO) δ (ppm) : 8,15 (large s, 1H) ; 8,0 (d, 1H) ; 7,85 (large s, 1H) ; 7,75 (d, 1H) ; 7,50 (q, 2H) ; 7,45 (large t, 1H) ; 7,30 (d, 1H) ; 6,80 (s, 1H) ; 5,20 (s, 2H) ; 4,50 (large d, 2H) ; 3,10 (m, 2H) ; 2,90 (large t, 2H) ; 1,80 (large d, 2H) ; 1,60 (m, 1H) ; 1,40 (m, 2H) ; 1,15 (m, 2H). | 458 | 0.79 | A |
| **2** | (DMSO) δ (ppm) : 8,0 (d, 1H) ; 7,9 (s, 1H) ; 7,80 (s, 1H) ; 7,50 (q, 2H) ; 7,40 (large t, 1H); 7,30 (d, 1 H) ; 5,15 (s, 2H) ; 4,55 (large d, 2H) ; 4,10 (s, 3H) ; 3,10 (m, 2H) ; 2,90 (large t, 2H) ; 1,80 (large d, 2H) ; 1,60 (m, 1H) ; 1,40 (m, 2H) ; 1,15 (m, 2H). | 429 | 0.75 | A |
| **3** | (DMSO) δ (ppm) : 8,0 (d, 1H) ; 7,90 (d, 2H) ; 7,80 (s, 1H) ; 7,60 (q, 2H) ; 7,50 (q, 2H) ; 7,40 (large t, 1H) ; 7,25 (d, 1H) ; 7,10 (s, 1H) ; 5,2 (s, 2H) ; 4,50 (large d, 2H) ; 3,10 (m, 2H) ; 2,85 (large t, 2H) ; 1,80 (large d, 2H) ; 1,65 (m, 1H) ; 1,4 (m, 2H) ; 1,15 (m, 2H). | 525 | 1.12 | A |
| **4** | (DMSO) δ (ppm) : 8,05 (m, 3H) ; 7,80 (s, 1H) ; 7,75 (m, 3H) ; 7,50 (q, 2H) ; 7,25 (d, 1H) ; 5,4 (s, 2H) ; 4,50 (large d, 2H) ; 3,15 (m, 2H) ; 2,85 (large t, 2H) ; 1,80 (large d, 2H) ; 1,65 (m, 1H) ; 1,4 (m, 2H) ; 1,15 (m, 2H). | 526 | 5.87 | B |
| **5** | (DMSO) δ (ppm) : 8,40 (s, 1H) ; 7,8 (d, 1H) ; 7,7 (dd, 2H) ; 7,65 (large t, 1H) ; 7,30 (t, 2H) ; 6,90 (d, 1H) ; 5,30 (s, 2H) ; 4,35 (large d, 2H) ; 3,1 (m, 2H) ; 2,85 (large t, 2H) ; 2,75 (q, 2H) ; 1,75 (large d, 2H) ; 1,60 (m, 1H) ; 1,45 (m, 2H) ; 1,25 (t, 3H) ; 1,15 (m, 2H). | 454 | 0.93 | A |
| **6** | (DMSO) δ (ppm) : 8,0 (d, 1H) ; 7,75 (m, 3H) ; 7,55 (m, 5H) ; 7,30 (m, 2H) ; 5,0 (s, 2H) ; 4,55 (large d, 2H) ; 3,10 (m, 2H) ; 2,90 (large t, 2H) ; 2,5 (s, 3H) ; 1,75 (large d, 2H) ; 1,60 (m, 1H) ; 1,4 (m, 2H) ; 1,15 (m, 2H). | 505 | 1.04 | A |
| **7** | (DMSO) δ (ppm) : 8,40 (s, 1H) ; 7,8 (d, 1H) ; 7,65 (dd, 2H) ; 7,55 (large t, 1H) ; 7,25 (t, 2H) ; 6,90 (d, 1H) ; 5,30 (s, 2H) ; 4,30 (large d, 2H) ; 3,10 (m, 3H) ; 2,80 (large t, 2H) ; 1,75 (large d, 2H); 1,60 (m, 1H) ; 1,4 (m, 2H) ; 1,25 (d, 6H) ; 1,15 (m, 2H). | 468 | 0.98 | A |
| **8** | (DMSO) δ (ppm) : 8,40 (s, 1H) ; 7,8 (d, 1H) ; 7,65 (m, 3H) ; 7,25 (t, 2H) ; 7,15 (large t, 1H) ; 6,90 (d, 1H) ; 6,2 (s, 1H) ; 4,95 (s, 2H) ; 4,30 (large d, 2H) ; 3,80 (s, 3H) ; 3,05 (m, 2H) ; 2,80 (large t, 2H) ; 1,75 (large d, 2H) ; 1,60 (m, 1H) ; 1,4 (m, 2H) ; 1,15 (m, 2H). | 438 | 0.85 | A |
| **9** | (DMSO) δ (ppm) : 9,15 (s, 1H) ; 8,4 (s, 1H) ; 7,8 (d, 1H) ; 7,7 (dd, 2H) ; 7,40 (large t, 1H) ; 7,25 (t, 2H) ; 6,90 (d, 1H) ; 5,5 (s, 2H) ; 4,35 (large d, 2H) ; 3,1 (m, 2H) ; 2,85 (large t, 2H) ; 1,75 (large d, 2H) ; 1,55 (m, 1H) ; 1,4 (m, 2H) ; 1,15 (m, 2H). | 442 | 0.87 | A |
| **10** | (DMSO) δ (ppm) : 8,40 (s, 1H) ; 7,80 (d, 1H) ; 7,70 (dd, 2H) ; 7,50 (large t, 1H) ; 7,30 (t, 2H) ; 6,90 (d, 1H) ; 5,40 (s, 2H) ; 4,35 (large d, 2H) ; 3,10 (m, 2H) ; 2,80 (large t, 2H) ; 1,75 (large d, 2H) ; 1,55 (m, 1H) ; 1,45 (m, 11 H) ; 1,15 (m, 2H). | 498 | 0.99 | A |
| **11** | (DMSO) δ (ppm) : 8,40 (s, 1H) ; 7,80 (d, 1H) ; 7,65 (dd, 2H) ; 7,45 (large t, 1H) ; 7,25 (t, 2H) ; 6,90 (d, 1H) ; 5,15 (s, 2H) ; 4,30 (large d, 2H) ; 3,30 (m, 1H) ; 3,10 (m, 2H) ; 2,80 (large t, 2H) ; 1,75 (large d, 2H) ; 1,60 (m, 1H) ; 1,40 (m, 2H) ; 1,30 (d, 6H) ; 1,10 (m, 2H). | 468 | 0.97 | A |
| **12** | (DMSO) δ (ppm) : 8,10 (dd, 2H) ; 8,0 (d, 1H) ; 7,80 (s, 1H) ; 7,60-7,40 (m, 5H) ; 7,25 (d, 1H) ; 5,30 (s, 2H) ; 4,50 (large d, 2H) ; 3,10 (m, 2H) ; 2,85 (large t, 2H) ; 1,80 (large d, 2H) ; 1,60 (m, 1H) ; 1,40 (m, 2H) ; 1,15 (m, 2H). | 510 | 0.97 | A |
| **13** | (DMSO) δ (ppm) : 8,05 (m, 3H) ; 7,80 (d, 1H) ; 7,70 (d, 2H) ; 7,65-7,45 (m, 3H) ; 7,25 (d, 1H) ; 5,30 (s, 2H) ; 4,50 (large d, 2H) ; 3,10 (m, 2H) ; 2,85 (large t, 2H) ; 1,75 (large d, 2H) ; 1,60 (m, 1H) ; 1,40 (m, 2H) ; 1,15 (m, 2H). | 526 | 1.02 | A |
| **14** | (DMSO) δ (ppm) : 8,0 (d, 1H) ; 7,95 (d, 2H) ; 7,80 (s, 1H) ; 7,55 (large t, 1H) ; 7,50 (q, 2H) ; 7,30 (d, 1H) ; 7,15 (d, 2H) ; 5,30 (s, 2H) ; 4,50 (large d, 2H) ; 3,85 (s, 3H) ; 3,10 (m, 2H) ; 2,85 (large t, 2H) ; 1,80 (large d, 2H) ; 1,60 (m, 1H) ; 1,40 (m, 2H) ; 1,15 (m, 2H). | 522 | 0.95 | A |
| **15** | (DMSO) δ (ppm) : 8,15 (dd, 2H) ; 7,95 (d, 1H) ; 7,75 (s, 1H) ; 7,65 (large t, 1H) ; 7,50 (q, 2H) ; 7,4 (t, 2H) ; 7,25 (d, 1H) ; 5,40 (s, 2H) ; 4,50 (large d, 2H) ; 3,10 (m, 2H) ; 2,85 (large t, 2H) ; 1,75 (large d, 2H) ; 1,60 (m, 1H) ; 1,40 (m, 2H) ; 1,15 (m, 2H). | 510 | 1.06 | A |
| **16** | (DMSO) δ (ppm) : 8,0 (d, 1H) ; 7,85 (d, 1H) ; 7,80 (s, 1H) ; 7,75 (dd, 1H) ; 7,65 (m, 2H) ; 7,50 (q, 2H) ; 7,45 (m, 1H) ; 7,25 (d, 1H) ; 5,40 (s, 2H) ; 4,50 (large d, 2H) ; 3,10 (m, 2H) ; 2,85 (large t, 2H) ; 1,80 (large d, 2H) ; 1,60 (m, 1H) ; 1,40 (m, 2H) ; 1,15 (m, 2H). | 510 | 1.06 | A |
| **17** | (DMSO) δ (ppm) : 8,05 (d, 2H) ; 8,0 (d, 1H) ; 7,80 (s, 1H) ; 7,65 (d, 2H) ; 7,50 (q, 2H) ; 7,45 (large t, 1H) ; 7,25 (d, 1H) ; 5,30 (s, 2H) ; 4,50 (large d, 2H) ; 3,15 (m, 2H) ; 2,85 (large t, 2H) ; 1,80 (large d, 2H) ; 1,60 (m, 1H) ; 1,40 (m, 2H) ; 1,15 (m, 2H). | 542 | 1.12 | A |
| **18** | (DMSO) δ (ppm) : 9,15 (s, 1H) ; 8,0 (d, 1 H) ; 7,75 (s, 1H) ; 7,50 (q, 2H) ; 7,35 (large t, 1H) ; 7,25 (d, 1 H) ; 5,50 (s, 2H) ; 4,50 (large d, 2H) ; 3,10 (m, 2H) ; 2,90 (large t, 2H) ; 1,80 (large d, 2H) ; 1,60 (m, 1H) ; 1,40 (m, 2H) ; 1,15 (m, 2H). | 432 | 0.84 | A |
| **19** | (DMSO) δ (ppm) : 8,45 (s, 1H) ; 8,15 (large s, 1H) ; 7,85 (m, 2H) ; 7,70 (dd, 2H) ; 7,45 (large t, 1 H) ; 7,30 (t, 2H) ; 6,90 (d, 1H) ; 6,8 (s, 1H) ; 5,20 (s, 2H) ; 4,40 (large d, 2H) ; 3,10 (m, 2H) ; 2,85 (large t, 2H) ; 1,80 (large d, 2H) ; 1,60 (m, 1H) ; 1,40 (m, 2H) ; 1,15 (m, 2H). | 468 | 0.82 | A |
| **20** | (DMSO) δ (ppm) : 8,70 (large s, 1H) ; 8,0 (d, 1H) ; 7,80 (s, 1H) ; 7,55 (q, 2H) ; 7,45 (large t, 1H) ; 7,30 (d, 1H) ; 6,80 (s, 1H) ; 5,20 (s, 2H) ; 4,50 (large d, 2H) ; 3,10 (m, 2H) ; 2,90 (large t, 2H) ; 2,80 (d, 3H) ; 1,80 (large d, 2H) ; 1,60 (m, 1H) ; 1,40 (m, 2H) ; 1,15 (m, 2H). | 472 | 0.82 | A |
| **21** | (DMSO) δ (ppm) : 8,60 (s, 1H) ; 8,40 (s, 1H) ; 7,80 (dd, 1H) ; 7,65 (m, 3H) ; 7,45 (m, 2H) ; 7,25 (t, 2H) ; 6,85 (d, 1H) ; 5,10 (s, 2H) ; 4,30 (large d, 2H) ; 3,10 (m, 2H) ; 2,75 (large t, 2H) ; 1,75 (large d, 2H) ; 1,55 (m, 1H) ; 1,35 (m, 2H) ; 1,10 (m, 2H). | 468 | 0.8 | A |
| **22** | (DMSO) δ (ppm) : 8,70 (large s, 1H) ; 8,05 (dd, 2H) ; 7,60 (dd, 1H) ; 7,45 (large t, 1H) ; 7,30 (t, 2H) ; 7,15 (d, 1H) ; 6,80 (s, 1H) ; 6,75 (s, 1H) ; 5,20 (s, 2H) ; 4,40 (large d, 2H) ; 3,10 (m, 2H) ; 2,85 (large t, 2H) ; 2,80 (d, 3H) ; 1,75 (large d, 2H) ; 1,60 (m, 1H) ; 1,40 (m, 2H) ; 1,15 (m, 2H). | 482 | 0.95 | A |
| **23** | (DMSO) δ (ppm) : 8,15 (large s, 1H) ; 8,05 (d, 1H) ; 7,80 (large s, 1H) ; 7,55 (dd, 1H) ; 7,50 (dd, 1H) ; 7,40 (m, 2H) ; 7,30 (d, 1H) ; 6,75 (s, 1H) ; 5,20 (s, 2H) ; 4,50 (large d, 2H) ; 3,10 (m, 2H) ; 2,90 (large t, 2H) ; 1,75 (large d, 2H) ; 1,60 (m, 1H) ; 1,40 (m, 2H) ; 1,15 (m, 2H). | 442 | 0.73 | A |
| **24** | (DMSO) δ (ppm) : 8,10 (large s, 1H) ; 8,05 (dd, 2H) ; 7,80 (large s, 1H) ; 7,60 (dd, 1H) ; 7,45 (large t, 1H) ; 7,30 (dd, 2H) ; 7,15 (d, 1H) ; 6,80 (s, 1H) ; 6,75 (s, 1H) ; 5,20 (s, 2H) ; 4,40 (large d, 2H) ; 3,15 (m, 2H) ; 2,85 (large t, 2H) ; 1,80 (large d, 2H) ; 1,60 (m, 1H) ; 1,40 (m, 2H) ; 1,15 (m, 2H). | 468 | 0.92 | A |
| **25** | (DMSO) δ (ppm) : 8,70 (large s, 1H) ; 8,0 (d, 1H) ; 7,55 (dd, 1H); 7,50 (dd, 1H) ; 7,40 (m, 2H) ; 7,30 (d, 1H) ; 6,80 (s, 1H) ; 5,20 (s, 2H) ; 4,50 (large d, 2H) ; 3,10 (m, 2H) ; 2,85 (large t, 2H) ; 2,75 (d, 3H) ; 1,75 (large d, 2H) ; 1,60 (m, 1H) ; 1,40 (m, 2H) ; 1,15 (m, 2H). | 456 | 0.75 | A |
| **26** | (DMSO) δ (ppm) : 8,75 (large s, 1H) ; 8,40 (s, 1H) ; 7,80 (d, 1H) ; 7,65 (dd, 2H) ; 7,45 (large t, 1H) ; 7,25 (t, 2H) ; 6,90 (d, 1H) ; 6,80 (s, 1H) ; 5,20 (s, 2H) ; 4,40 (large d, 2H) ; 3,10 (m, 2H) ; 2,80 (large t, 2H) ; 2,75 (d, 3H) ; 1,75 (large d, 2H) ; 1,60 (m, 1H) ; 1,40 (m, 2H) ; 1,15 (m, 2H). | 482 | 0.85 | A |
| **27** | (DMSO) δ (ppm) : 8,10 (dd, 2H) ; 7,60 (dd, 1H) ; 7,45 (large t, 1H) ; 7,30 (dd, 2H) ; 7,15 (d, 1H); 6,80 (d, 1H); 6,70 (s, 1H) ; 5,20 (s, 2H) ; 4,40 (large d, 2H) ; 3,10 (m, 2H) ; 3,05 (s, 3H) ; 3,0 (s, 3H) ; 2,80 (large t, 2H) ; 1,80 (large d, 2H) ; 1,60 (m, 1H) ; 1,40 (m, 2H) ; 1,15 (m, 2H). | 496 | 0.99 | A |
| **28** | (DMSO) δ (ppm) : 8,50 (s, 1H) ; 8,15 (large s, 1H) ; 7,90 (dd, 1H) ; 7,80 (large s, 1H) ; 7,70-7.40 (m, 4H) ; 7,15 (m, 1H) ; 6,90 (d, 1H) ; 6,80 (s, 1H) ; 5,20 (s, 2H) ; 4,35 (large d, 2H) ; 3,10 (m, 2H) ; 2,85 (large t, 2H) ; 1,80 (large d, 2H) ; 1,60 (m, 1H) ; 1,40 (m, 2H) ; 1,15 (m, 2H). | 468 | 0.82 | A |
| **29** | (DMSO) δ (ppm) : 8,70 (s, 1H) ; 7,60 (dd, 2H) ; 7,50 (d, 2H) ; 7,40 (large t, 1H) ; 7,25 (t, 2H) ; 7,0 (d, 2H) ; 6,90 (s, 1H) ; 5,20 (s, 2H) ; 3,75 (large d, 2H) ; 3,10 (m, 2H) ; 2,75 (d, 3H) ; 2,65 (large t, 2H) ; 1,75 (large d, 2H) ; 1,45 (m, 3H) ; 1,15 (m, 2H). | 481 | 0.91 | A |
| **30** | (DMSO) δ (ppm) : 8,40 (s, 1H) ; 7,80 (d, 1H) ; 7,65 (dd, 2H) ; 7,45 (large t, 1H) ; 7,30 (t, 2H) ; 6,90 (d, 1H) ; 6,70 (s, 1H) ; 5,20 (s, 2H) ; 4,30 (large d, 2H) ; 3,15 (m, 2H) ; 3,10 (s, 3H) ; 3,05 (s, 3H) ; 2,80 (large t, 2H) ; 1,75 (large d, 2H) ; 1.60 (m, 1H) ; 1,40 (m, 2H) ; 1,15 (m, 2H). | 496 | 0.89 | A |
| **31** | (DMSO) δ (ppm) : 8,0 (d, 1H) ; 7,80 (s, 1H) ; 7,50 (q, 2H) ; 7,45 (large t, 1H) ; 7,30 (d, 1H) ; 6,70 (s, 1H) ; 5,20 (s, 2H) ; 4,55 (large d, 2H) ; 3,15 (m, 2H) ; 3,10 (s, 3H) ; 3,05 (s, 3H) ; 2,90 (large t, 2H) ; 1,80 (large d, 2H) ; 1.60 (m, 1H) ; 1,40 (m, 2H) ; 1,15 (m, 2H). | 486 | 0.86 | A |
| **32** | (DMSO) δ (ppm) : 8,70 (large s, 1H) ; 8,4 (s, 1H) ; 8,3 (s, 1H) ; 8,150 (dd, 2H) ; 7,45 (large t, 1H) ; 7,35 (t, 2H) ; 6,80 (s, 1H) ; 5,20 (s, 2H) ; 4,45 (large d, 2H) ; 3,10 (m, 2H) ; 2,90 (large t, 2H) ; 2,75 (d, 3H) ; 1,80 (large d, 2H) ; 1.60 (m, 1H) ; 1,40 (m, 2H) ; 1,15 (m, 2H). | 483 | 1.09 | A |
| **33** | (DMSO) δ (ppm) : 8,40 (s, 1H) ; 8,30 (s, 1H) ; 8,15 (m, 3H) ; 7,85 (m, 1H) ; 7,45 (m, 1H) ; 7,35 (m, 2H) ; 6,80 (s, 1H) ; 5,25 (m, 2H) ; 4,45 (m, 2H) ; 3,10 (m, 2H) ; 2,80 (m, 2H) ; 1,80 (m, 2H) ; 1,60 (m, 1H) ; 1,40 (m, 2H) ; 1,20 (m, 2H) . | 469 | 1.06 | A |
| **34** | (DMSO) δ (ppm) : 8,40 (s, 1H) ; 8,30 (s, 1H) ; 8,15 (m, 2H) ; 7,45 (m, 1H) ; 7,35 (m, 2H) ; 6,70 (s, 1H) ; 5,25 (m, 2H) ; 4,45 (m, 2H) ; 3,15 - 3,00 (m, 8H) ; 2,90 (m, 2H) ; 1,80 (m, 2H) ; 1,60 (m, 1H) ; 1,40 (m, 2H) ; 1,20 (m, 2H). | 497 | 1.13 | A |
| **35** | (DMSO) δ (ppm) : 8,75 - 8,60 (m, 3H) ; 7,70 (m, 2H) ; 7,45 (m, 1H) ; 7,30 (t, 2H) ; 6,80 (s, 1H) ; 5,20 (m, 2H) ; 4,70 (m, 2H) ; 3,10 (m, 2H) ; 3,90 (m, 2H) ; 2,90 (m, 3H) ; 1,75 (m, 2H) ; 1,60 (m, 1H) ; 1,4 (m, 2H) ; 1,10 (m, 2H). | 483 | 1.13 | A |
| **36** | (DMSO) δ (ppm) : 8,70 (s, 2H) ; 7,70 (m, 2H) ; 7,45 (m, 1H) ; 7,30 (m, 2H) ; 6,70 (s, 1H) ; 5,20 (m, 2H) ; 4,70 (m, 2H) ; 3,15 - 3,00 (m, 8H) ; 3,00 - 2,80 (m, 2H) ; 1,75 (m, 2H) ; 1,65 (m, 1H) ; 1,40 (m, 2H) ; 1,10 (m, 2H). | 497 | 1.16 | A |
| **37** | (DMSO) δ (ppm) : 8,80 - 8,60 (m, 2H) ; 7,45 (m, 1H) ; 7,00 (m, 1H) ; 6,80 (s, 1H) ; 5,20 (m, 2H) ; 4,65 (m, 2H) ; 3,10 (m, 2H) ; 2,95 (m, 2H) ; 2,80 (m, 3H) ; 1,80 (m, 2H) ; 1,60 (m, 1H) ; 1,40 (m, 2H) ; 1,10 (m, 2H). | 457 | 1.44 | A |
| **38** | (DMSO) δ (ppm) : 8,65 (m, 1H) ; 8,15 (m, 1H); 7,85 (m, 1H) 7,45 (m, 1H) ; 6,95 (m, 1H) ; 6,80 (s, 1H) ; 5,20 (m, 2H) ; 4,65 (m, 2H) ; 3,10 (m, 2H) ; 2,95 (m, 2H) ; 1,80 (m, 2H) ; 1,60 (m, 1H); 1,40 (m, 2H) ; 1,10 (m, 2H). | 443 | 1.39 | A |
| **39** | (DMSO) δ (ppm) : 8,70 (s, 2H) ; 8,15 (m, 1H) ; 7,85 (m, 1H) ; 7,70 (m, 2H) ; 7,45 (m, 1H) ; 7,30 (m, 2H) ; 6,75 (s, 1H) ; 5,20 (m, 2H) ; 4,70 (m, 2H) ; 3,10 (m, 2H) ; 2,90 (m, 2H) ; 1,75 (m, 2H) ; 1,60 (m, 1H) ; 1,40 (m, 2H) ; 1,10 (m, 2H). | 469 | 1.33 | A |
| **40** | (DMSO) δ (ppm) : 8,65 (m, 1H) ; 7,45(m, 1H) ; 6,95 (m, 1H) ; 6,70 (s, 1H) ; 5,20 (m, 2H) ; 4,65 (m, 2H) ; 3,20 (m, 5H) ; 3,05 (s, 3H) ; 2,90 (m, 2H) ; 1,80 (m, 2H) ; 1,60 (m, 1H) ; 1,40 (m, 2H) ; 1,10 (m, 2H). | 471 | 1.4 | A |
| **41** | (DMSO) δ (ppm) : 8,25 - 8,00 (m, 3H) ; 7,90 (m, 1H) ; 7,85 (m, 1H) ; 7,55 - 7,25 (m, 4H) ; 6,80 (s, 1H) ; 5,25 (m, 2H) ; 4,45 (m, 2H) ; 3,10 (m, 2H) ; 2,95 (m, 2H) ; 1,80 (m, 2H) ; 1,65 (m, 1H) ; 1,40 (m, 2H) ; 1,20 (m, 2H) . | 469 | 0.84 | A |
| **42** | (DMSO) δ (ppm) : 8,60 (m, 1H) ; 8,40 (s, 1H) ; 7,80 (m, 1H) ; 7,65 (m, 2H) ; 7,45 (m, 1H) ; 7,30 (m, 2H) ; 6,90 (m, 1H) ; 6,80 (s, 1H) ; 5,20 (m, 2H) ; 4,35 (m, 2H) ; 3,30 (m, 2H) ; 3,10 (m, 2H) ; 2,80 (m, 2H) ; 2,40 (m, 2H) ; 2,10 (s, 6H) ; 1,75 (m, 2H) ; 1,60 (m, 1H) ; 1,40 (m, 2H) ; 1,15 (m, 2H). | 539 | 0.81 | A |
| **43** | (DMSO) δ (ppm): 8,10 (m, 1H) ; 7,95-7,75 (m, 2H) ; 7,50-7,25 (m, 2H) ; 6,75 (m, 2H) ; 5,20 (m, 2H) ; 4,20 (m, 2H) ; 3,10 (m, 2H) ; 2,70 (m, 2H) ; 2,30 (d, 2H) ;1,85 - 1,65 (m, 3H) ; 1,60 - 1,30 (m, 3H) ; 1,10 (m, 2H) ; 0,85 (m, 6H) . | 430 | 0.9 | A |
| **44** | (DMSO) δ (ppm) : 8,00 (m, 1H) ; 7,90 - 7,80(m, 2H) ; 7,45 (m, 1H) ; 7,30 (m, 1H) ; 6,75 (m, 2H) ; 5,20 (m, 2H) ; 4,20 (m, 2H) ; 3,10 (m, 2H) ; 2,70 (m, 2H) ; 2,35 (s, 2H) ; 1,75 (m, 2H) ; 1,60 - 1,30 (m, 3H) ; 1,10 (m, 2H ; 0,85 (s, 9H). | 444 | 0.95 | A |
| **45** | (DMSO) δ (ppm) : 8,40 (s, 1H) ; 8,15 (large s, 1H) ; 7,80 (m, 2H) ; 7,40 (m, 2H) ; 7,30 (t, 1H); 7,10 (d, 1H); 6,90 (d, 1H); 6,75 (s, 1H) ; 5.20 (s, 2H) ; 4,30 (d, 2H) ; 3,10 (t, 2H) ; 2,80 ( t, 2H) ; 2,35 (s, 3H) ; 1,75 (d, 2H) ; 1,60 (m, 1H) ; 1,40 (q, 2H) ; 1,10 (q, 2H). | 464 | 0.8 | A |
| **46** | (DMSO) δ (ppm) : 8,25 (s, 1H) ; 8,10 ( large d, 1H) ; 7,70 ( m, 2H) ; 7,40 - 7,30 (m, 3H) ; 6,80 (s, 1H) ; 5,25 (s, 2H) ; 4,30 ( m, 2H) ; 3,65-3,35 (m, 8H) ; 3,20-2,90 (m, 4H) ; 2,85 (s, 3H) ; 1,80 (d, 2H) ; 1.65 (m, 1H) ; 1,40 (m, 2H) ; 1,30-1,10(m,2H). | 551 | 0.82 | A |
| **47** | (DMSO) δ (ppm): 8,50 (s, 1H) ; 8,15 (large s, 1H) ; 7,90 (d, 1H) ;7,85 (large s, 1H) ; 7,70 (d, 1H) ; 7,60 (m, 2H) ; 7,55 (m, 1H) ; 7,30 (d, 1H) ; 6,95 (d, 1H) ; 6,80 (s, 1H) ; 5,20 (s, 2H) ; 4,35 (d, 2H) ; 3,10 (m, 2H) ; 2,85 (t, 2H) ; 1,75 (d, 2H) ; 1,60 (m, 1H) ; 1,40 (m, 2H) ; 1,15 (m, 2H). | 534 | 1.03 | A |
| **48** | (DMSO) δ (ppm): 8,40 (s, 1H) ; 8,15 (s, 1H) ; 7,85 (m, 2H); 7,70 (s, 1H) ; 7,60 (m, 1H) ; 7,50-7,20 (m, 3H) ; 6,85 (d, 1H) ; 6,75 (s, 1H) ; 5,20 (s, 2H) ; 4,30 (d, 2H) ; 3,05 (m, 2H) ; 2,80 (m, 2H); 1,70 (d, 2H) ; 1,55 (m, 1H) ; 1,35 (m, 2H) ; 1,10 (m, 2H). | 484 | 0.96 | A |
| **49** | (DMSO) δ (ppm) : 8,45 (s, 1H) ; 8,15 (large s, 1H) ; 7,85 (m, 2H) ; 7,45 (m, 1H) ; 7,05 (m, 2H) ; 6,90 (d, 1H) ; 6,75 (m, 2H) ; 5,20 (s, 2H) ; 4,35 (m, 2H) ; 3,85 (s, 3H) ; 3,10 ( m, 2H) ; 2,85 (m, 2H) ; 1,75 (m, 2H) ; 1,60 (m, 1H) ; 1,40 (m, 2H) ; 1,10(q,2H). | 498 | 0.93 | A |
| **50** | (DMSO) δ (ppm) : 8,35 (s, 1H) ; 8,15 ( large s, 1H) ; 7,80 ( large s, 1H) ; 7,75 (d, 1H) ; 7,45 (t, 1H) ; 7,20 (s, 1H) ; 7,10 (d, 1H) ; 7,00 (d, 1H) ; 6,90 (d, 1H) ; 6,80 (s, 1H) ; 6,05 (s, 2H) ; 5,20 (s, 2H) ; 4,30 (d, 2H) ; 3,10 (m, 2H) ; 2,80 (t, 2H) ; 1,75 (d, 2H) ; 1,55 (m, 1H) ; 1,40 (m, 2H) ; 1,15 (m, 2H). | 494 | 0.88 | A |
| **51** | (DMSO) δ (ppm) : 8,70 (m, 1H) ; 7,75 (m, 1H) ; 7,00 (m, 1H) ; 6,90 (s, 1H) ; 5,25 (m, 2H) ; 4,50-4,30 (m, 4H) ; 3,40-3,15 (m, 4H) ; 1,90 - 1,50 (m, 4H) ; 1,35 (m, 3H). | 476 | 1.36 | A |
| **52** | (DMSO) δ (ppm) : 8,65 (d, 1H) ; 7,60 (d, 1H) ; 7,15 (m, 1H) ; 6,95 (d, 1H) ; 6,20 (d, 1H) ; 4,90 (m, 2H) ; 4,60 (m, 2H) ; 3,80 (s, 3H) ; 3,05-2,80 (m, 4H) ; 1,80-1,65 (m, 2H) ; 1,60 - 1,35 (m, 3H) ; 1,20 (m, 2H) ; 1,05 (m, 2H). | 427 | 1,33 | A |
| **53** | (DMSO) δ (ppm) : 8,40 (large s, 1H) ; 8,00 (large s, 2H) ; 7,85 (large s, 1H) ; 7,75 (d, 1H) ; 7,55 (d, 1H) ; 6,85 (s, 1 H) ; 5,20 (s, 2H) ; 4,50 (large d, 2H) ; 3,55 (m, 2H) ; 3,10 (m, 2H) ; 1,90 (m, 2H) ; 1,70 (m, 1H) ; 1,40 (m, 2H) ; 1,25 (m, 2H). | 459 | 0,84 | A |
| **54** | (DMSO) δ (ppm) : 8,90 (s, 1H) ; 7,90 (s, 1H) ; 7,65 - 7,50 (m, 3H) ; 5,65 (m, 2H) ; 4,60 (m, 2H) ; 3,30 (m, 1H) ; 3,10-2,90 (m, 4H) ; 1,85 - 1,70 (m, 3H) ; 1,35 (d, 6H) ; 1,20 (m, 2H). | 445 | 1,31 | A |
| **55** | (DMSO) δ (ppm) : 8,70 (m, 1H) ; 7,55 (m, 1H) ; 7,00 (m, 1H) ; 5,15 (m, 2H) ; 4,65 (m, 2H) ; 3,30 (m, 1H) ; 3,00 - 2,90 (m, 4H) ; 1,80 - 1,60 (m, 3H) ; 1,35 (m, 6H) ; 1,10 (m, 2H). | 429 | 1,37 | A |
| **56** | (DMSO) δ (ppm) : 8,05 (d, 1H) ; 7,90 - 7,70 (m, 2H) ; 7,60-7,50 (m, 2H) ; 7,35 (d, 1H) ; 6,90 (s, 1H) ; 5,25 (m, 2H) ; 4,45 - 4,30 (m, 4H) ; 3,40 - 3,20 (m, 4H) ; 1,90 - 1,60 (m, 4H) ; 1,35 (t, 3H). | 491 | 1,00 | A |
| **57** | (DMSO) δ (ppm) : 8,70 (m, 2H) ; 7,75 (m, 1H) ; 7,00 (m, 1H) ; 6,80 (s, 1H) ; 5,20 (m, 2H) ; 4,45 (m, 2H) ; 3,40 - 3,20 (m, 4H) ; 2,80 (m, 3H) ; 1,90 - 1,55 (m, 4H) . | 461 | 1,19 | A |
| **58** | (DMSO) δ (ppm) : 8,40 (m, 2H) ; 7,60 - 7,50 (m, 2H) ; 5,15 (m, 2H) ; 3,40 - 3,25 (m, 3H) ; 3,05 - 2,80 (m, 4H) ; 1,80 (m, 2H) ; 1,65 (m, 1H) ; 1,40 - 1,20 (m, 8H) . | 472 | 1,44 | A |
| **59** | (DMSO) δ (ppm) : 8,20 (m, 1H) ; 8,00 - 8,20 (m, 3H) ; 7,60 (t, 1H) ; 5,15 (s, 2H) ; 3,85 (d, 2H) ; 3,30 (m, 1H) ; 3,00 (m, 4H) ; 1,85 (m, 2H) ; 1,75 (m, 1H) ; 1,60 - 1,40 (m, 2H) ; 1,30 (m, 6H). | 445 | 1.06 | A |
| **60** | (DMSO) δ (ppm) : 8,70 (large s, 2H) ; 7,45 (t, 1H) ; 7,10 (m, 1H) ; 6,80 (s, 1H) ; 5,20 (s, 2H) ; 4,10 (d, 2H) ; 3,80 (d, 2H) ; 3,10 (m, 2H) ; 2,80 (s, 3H) ; 2,20 (s, 6H) ; 1,90 (m, 2H). | 472 | 0.77 | A |
| **61** | (DMSO) δ (ppm) : 8,70 (m, 2H) ; 7,45 (m, 1H) ; 7,00 (m, 1H) ; 6,80 (s, 1H) ; 5,20 (s, 2H) ; 3,90-3,60 (m, 4H) ; 3,10 (m, 2H) ; 2,80 (m, 3H) ;1,50 (m, 2H) ; 1,40 (m, 6H) ; 0,80 (m, 3H). | 485 | 1,39 | A |
| **62** | (DMSO) δ (ppm) : 8,70 (m, 2H) ; 7,35 (t, 1H) ; 6,95 (m, 1H) ; 6,80 (s, 1H ) ; 5,20 (m, 2H) ; 4,50 (s, 1H) ; 4,30 (m, 2H) ; 3,40 (m, 2H) ; 3,20 (m, 2H) ; 2,80 (m, 3H) ; 1,90 (m, 4H) ; 1,45 (m, 2H). | 473 | 1,14 | A |
| **63** | (DMSO) δ (ppm) : 8,20 (m, 2H) ; 8,05 (m, 1H) ; 7,90 (m, 1H) ; 7,80 (large s, 1H) ; 7,20 (m, 1H) ; 6,90 (s, 1H) ; 6,40 (m, 1H) ; 4,40 (large s, 2H) ; 4,20 (large s, 2H) ; 3,70 (s, 3H) ; 3,20 (m, 2H) ; 2,30 (m, 6H) ; 1,95 (m, 2H). | 512 | 0,74 | A |
| **64** | (DMSO) δ (ppm) : 8,80 (s, 1H) ; 8,70 (m, 1H) ; 7,90 (s, 1H) ; 7,60 (s, 2H) ; 7,40 (m, 1H) ; 6,80 (s, 1H) ; 5,20 (s, 2H) ; 3,90-3,60 (m, 4H) ; 3,00 (m, 2H) ; 2,80 (m, 3H) ;1,50 -1,30 (m, 8H) ; 0,80 (t, 3H). | 501 | 1,33 | A |
| **65** | (DMSO) δ (ppm) : 8,80 (s, 1H) ; 8,70 (m, 1H) ; 7,90 (s, 1H) ; 7,60 (s, 2H) ; 7,40 (m, 1H) ; 6,80 (s, 1H) ; 5,20 (s, 2H) ; 3,90 (m, 2H) ; 3,70 (m, 2H) ; 3,10 (m, 2H) ; 2,80 (m, 3H) ;1,80 (m, 1H) ; 1,60 -1,40 (m, 6H) ; 1,30 (m, 2H) ; 0,90 (d, 6H). | 529 | 5.13 | B |
| **66** | (DMSO) δ (ppm) : 8,70 (s, 1H) ; 8,40 (m, 2H) ; 7,60 (d, 1H) ; 7,40 (t, 1H) ; 6,80 (s, 1H) ; 5,20 (s, 2H) ; 3,20-3,00 (m, 6H) ; 2,80 (m, 3H) ; 1,70 (m, 1H) ; 1,60-1,40 (m, 6H) ; 1,30 (m, 2H) ; 0,90 (d, 6H). | 556 | 1,54 | A |
| **67** | (DMSO) δ (ppm) : 8,15 (d, 1H) ; 8,05 (d, 1H) ; 7,90 (d, 1H) ; 7,70 (m, 1H) ; 7,60 (m, 2H) ; 7,35 (d, 1H) ; 5,20 (s, 2H) ; 3,75 (d, 2H) ; 3,30 (m, 1H); 3,10 (m, 2H) ; 2,90 (t, 2H) ; 1,85 (d, 2H) ; 1,70 (m, 1H) ; 1,45 (m, 2H) ; 1,30 (d, 6H). | 410 | 0.92 | A |
| **68** | (DMSO) δ (ppm) : 8,70 (s, 1H) ; 8,40 (s, 1H) ; 8,15 (large s, 1H) ; 8,00 (m, 2H) ; 7,80 (large s, 1H) ; 7,45 (m, 1H) ; 7,30 (m, 2H) ; 6,80 (s, 1H) ; 5,25 (m, 2H) ; 4,40 (m, 2H) ; 3,10 (m, 2H) ; 2,90 (m, 2H) ; 1,80 (m, 2H) ; 1,60 (m, 1H) ; 1,40 (m, 2H) ; 1,20 (m, 2H). | 469 | 1,20 | A |
| **69** | (DMSO) δ (ppm) : 8,75 (large s, 1H) ; 8,70 (s, 1H) ; 8,40 (s, 1H) ; 8,00 (m, 2H) ; 7,45 (large s, 1H) ; 7,30 (t, 2H) ; 6,85 (s, 1H) ; 5,25 (m, 2H) ; 4,40 (m, 2H) ; 3,10 (m, 2H) ; 2,90 (m, 2H) ; 2,75 (s, 3H) ; 1,80 (m, 2H) ; 1,60 (m, 1H) ; 1,40 (m, 2H) ; 1,20 (m, 2H). | 483 | 1,24 | A |
| **70** | (DMSO) δ (ppm) : 8,80 - 8,60 (m, 2H) ; 7,55 (m, 1H) ; 7,00 (m, 1H) ; 6,80 (s, 1H) ; 5,20 (s, 2H) ; 4,50 (m, 2H) ; 3,70 (m, 1H);3,15 (m, 2H) ; 2,80 (s, 3H);1,90 (m, 2H); 1,40 (m, 2H). | 429 | 1,18 | A |
| **71** | (DMSO) δ (ppm) : 8,00 (d, 1H); 7,80 (s, 1H); 7,45-7,55 (m, 3H); 7,30 (d, 1H); 6,90 (s, 1H); 5,20 (s, 2H); 4,55 (m, 2H); 4,40 (q, 2H); 3,10 (m, 2H); 2,90 (m, 2H); 1,80 (m, 2H); 1,60 (m, 1H); 1,40 (m, 2H); 1,30 (t, 3H); 1,15 (m, 2H). | 487 | 1,05 | A |
| **72** | (DMSO) δ (ppm) :8,80 - 8,60 (m, 2H) ; 7,45 (m, 1H) ; 6,95 (m, 1H) ; 6,80 (s, 1H) ; 5,20 (s, 2H) ; 3,95 (m, 2H) ; 3,60 (m, 2H);3,10 (m, 2H) ; 2,80 (s, 3H);1,50 (m, 2H); 1,40 (m, 4H) ;1,00 (s, 3H). | 471 | 4,48 | B |
| **73** | (DMSO) δ (ppm) : 8,10 (d, 1H) ; 7,85 (m, 1H) ; 7,80(s, 1H) ; 7,50 (m, 2H) ; 7,30 (d, 1H) ; 7,25 (s, 1H) ; 6,95 (s, 1H) ; 6,45 (m, 1H) ; 4,40 (d, 2H) ; 3,75 (s, 3H) ; 3,10 (m, 2H) ; 2,90 (m, 2H) ; 1,80 (d, 2H) ; 1,60 (m, 1H) ; 1,40 (m, 2H) ; 1,10 (m, 2H). | 496 | 0.77 | A |
| **74** | (DMSO) δ (ppm) : 8,70 (s, 1H) ; 8,10 (large s, 1H) ; 7,90 (large s, 1H) ; 7,50 (m, 1H) ; 7,00 (m, 1H) ; 6,80 (s, 1H) ; 5,20 (s, 2H) ; 3,90 (m, 1H) ; 3,75 (m, 2H);3,55 (m, 2H) ; 2,00-1,85 (m, 2H);1,80-1,65 (m, 3H); 1,40 (m, 1H). | 429 | 1.1 | A |
| **75** | (DMSO) δ (ppm) : 8,70 (m, 1H); 8,20 (m, 1H); 8,10 (m, 3H); 7,65 (m, 1H); 6,80 (s, 1H); 5,25 (s, 2H); 3,80 (m, 2H); 3,60 (m, 1H); 3,15 (m, 2H); 2,80 (s, 3H); 2,00 (m, 2H); 1,75 (m, 2H). | 445 | 3,52 | B |
| **76** | (DMSO) δ (ppm) : 8,70 (m, 1H); 8,40 (m, 2H); 7,60 (m, 2H); 6,80 (s, 1H); 5,20 (s, 2H); 3,30 (m, 2H); 3,60 (m, 1H); 3,05 (m, 2H); 2,80 (s, 3H); 1,90 (m, 2H); 1,60 (m, 2H). | 472 | 4,30 | B |
| **77** | (DMSO) δ (ppm) : 8,90 (s, 1H); 8,70 (m, 1H); 7,90 (s, 1H); 7,60 (m, 2H); 7,55 (large d, 1H); 6,80 (s, 1H); 5,20 (m, 2H); 4,50 (m, 2H); 3,70 (s, 1H); 3,20 (m, 2H); 2,80 (s, 3H); 1,90 (m, 2H); 1,50 (m, 2H). | 445 | 4,02 | B |
| **78** | (DMSO) δ (ppm) : 8,90 (s, 1H) ; 8,70 (large s, 1H) ; 7,90 (s, 1H) ; 7,60 (s, 2H) ; 7,40 (m, 1H) ; 6,80 (s, 1H) ; 5,20 (s, 2H) ; 3,95 (m, 2H) ; 3,60 (m, 2H) ; 3,10 (m, 2H) ; 2,80 (m, 3H) ; 1,60 - 1,40 (m, 6H) ; 1,00 (s, 3H). | 487 | 1,24 | A |
| **79** | (DMSO) δ (ppm) : 8,80 (m, 1H) ; 8,40 (m, 1H) ; 8,15 (m, 1H) ; 8,05 (m, 2H) ; 7,80 (m, 1H) ; 6,80 (s, 1H) ; 5,25 (s, 2H) ; 3,65 (m, 1H) ; 3,50 (m, 2H) ; 3,30 (m, 1H) ; 3,20(m, 2H) ; 2,80(m, 4H) ; 2,05 (m, 1H) ; 1,70 (m, 1H). | 445 | 0,82 | A |
| **80** | (DMSO) δ (ppm): 8,00 (d, 1H); 7,80 (s, 1H); 7,65 (m, 2H); 7,50 (m, 2H); 7,30 (d, 1H); 7,10 (m, 1H); 6,50 (m, 1H); 5,65 (m, 1H); 4,50 (m, 2H); 3,05 (m, 2H); 2,85 (m, 2H); 1,75 (m, 3H); 1,60 (m, 3H); 1,35 (m, 2H); 1,10 (m, 2H); 0,90 (d, 6H). | 470 | 1,16 | A |
| **81** | (DMSO) δ (ppm): 8,10 (m, 2H); 7,60 (m, 3H); 7,30 (m, 2H); 7,20 (d, 1H); 7,10 (tl, 1H); 6,80 (d, 1H); 6,45 (m, 1H); 5,65 (m, 1H); 4,40 (m, 2H); 3,05 (m, 2H); 2,80 (m, 2H); 1,80 (m, 3H); 1,55 (m, 3H); 1,35 (m, 2H); 1,15 (m, 2H); 0,90 (d, 6H). | 480 | 1,25 | A |
| **82** | (DMSO) δ (ppm): 8,65 (d, 1H); 8,15 (sl, 1H); 7,85 (sl, 1H); 7,50 (tl, 1H); 7,05 (d, 1H); 6,80 (s, 1H); 5,20 (s, 2H); 4,20 (s, 2H); 3,70 (m, 2H); 3,10 (m, 2H); 2,75 (m, 1H); 1,80 (m, 2H). | 415 | 0,94 | A |
| **83** | (DMSO) δ (ppm): 8,70 (sl, 1H); 8,65 (d, 1H); 7,50 (tl, 1H); 7,05 (d, 1H); 6,80 (s, 1H); 5,20 (s, 2H); 4,20 (s, 2H); 3,70 (m, 2H); 3,05 (m, 2H); 2,80 (s, 3H); 2,70 (m, 1H); 1,80 (m, 2H ). | 429 | 0,98 | A |
| **84** | (DMSO) δ (ppm) : 8,70 (sl, 1H) ; 8,60 (sl, 1H) ; 8,05 (m, 1H) ; 7,00 (m, 1H) ; 6,80 (s, 1H) ; 5,25 (s, 2H) ; 3,45 (m, 2H) ; 3,30 (m, 3H) ; 3,10 (m, 1H) ; 2,50 (m, 1H); 2,80 (m, 3H) ; 2,00 (m, 1H) ; 1,70 (m, 1H). | 429 | 1,00 | A |
| **85** | (DMSO) δ (ppm) : 8,70 (sl, 1H) ; 8,60 (sl, 1H) ; 8,05 (m, 1H) ; 7,00 (m, 1H) ; 6,80 (s, 1H) ; 5,25 (s, 2H) ; 3,45 (m, 2H) ; 3,30 (m, 3H) ; 3,10 (m, 1H) ; 2,80 (m, 3H) ; 2,50 (m, 1H); 2,00 (m, 1H) ; 1,70 (m, 1H). | 429 | 1,01 | A |

Les composés de l'invention ont fait l'objet d'essais pharmacologiques permettant de déterminer leur effet inhibiteur de l'enzyme FAAH (Fatty Acid Amide Hydrolase).

Protocole 1: L'activité inhibitrice a été mise en évidence dans un test radioenzymatique basé sur la mesure du produit d'hydrolyse de l'anandamide [éthanolamine 1-³H] par la FAAH *(*Life Sciences (1995), 56, 1999-2005 et Journal of Biochemical and Biophysical Methods (2004), 60(2), 171-177). Ainsi, les cerveaux de souris (moins le cervelet) sont prélevés et conservés à -80°C. Les homogénats membranaires sont préparés extemporanément par homogénéisation des tissus à l'aide d'un appareil Precellys® dans le tampon de réaction (Tris-HCl 10 mM pH=8, NaCl 150 mM et acide éthylène-diamine-tétraacétique (EDTA) 1 mM). La réaction enzymatique est conduite dans des plaques de filtration Multiscreen 96puits dans un volume final de 70µl. Du tampon de réaction supplémenté avec de l'albumine de sérum bovin sans acides gras (BSA, 1 mg/ml) est utilisé pour la réaction enzymatique, la dilution des composés et de l'anandamide [éthanolamine 1-³H]. Sont ajoutés successivement dans les puits, du tampon de réaction contenant la BSA (43µl/puits), les composés dilués testés à différentes concentrations (7µl/puits contenant 1% de DMSO) et la préparation membranaire (10µl/puits soit 200 µg de tissu par essai). Après 20 minutes de pré-incubation des composés avec l'enzyme à 25°C, la réaction est démarrée par l'addition d'anandamide [éthanolamine 1-³H] (activité spécifique de 15-20 Ci/mmol) diluée avec de l'anandamide froide (10µl/puits, concentration finale de 10 µM, 0,01µCi par essai). Après 20 minutes d'incubation à 25°C, la réaction enzymatique est arrêtée par addition d'une solution de charbon actif 5M préparée dans un tampon NaCl 1,5M et HCl 0,5M (50µl/puits). Le mélange est agité 10 minutes puis la phase aqueuse contenant l'éthanolamine [1-³H] est récupérée par filtration sous-vide et comptée par scintillation liquide.

Protocole 2: L'activité inhibitrice a été mise en évidence par technique fluorescente dans un test enzymatique basé sur la mesure du produit d'hydrolyse fluorescent de l'arachidonoyl 7-amino 4-méthyl coumarin amide (AAMC) par la FAAH (Analytical Biochemistry (2005), 343:143-151, J. of Biomolecular Screening (2006), 11(5): 519-527 et J. of Neurosciences Methods (2007), 161: 47-54). Ainsi, les cerveaux de souris (moins le cervelet) sont prélevés et conservés à -80°C. Les homogénats de cerveaux sont préparés extemporanément par homogénéisation des tissus à l'aide d'un appareil Precellys® dans le tampon de réaction (Tris-HCl 10 mM pH=8, NaCl 150 mM et acide éthylène-diamine-tétraacétique (EDTA) 1 mM). La réaction enzymatique est conduite dans des plaques 384 puits noires en polystyrène dans un volume final de 50µL. Du tampon de réaction supplémenté avec de l'albumine de sérum bovin sans acides gras (BSA, 1 mg/ml) est utilisé pour la réaction enzymatique, la dilution des composés et la dilution de l'AAMC. Sont ajoutés successivement dans les puits, du tampon de réaction contenant la BSA (25µl/puits), les composés dilués testés à différentes concentrations (5µl/puits contenant 1% de DMSO) et la préparation membranaire (10µl/puits soit 200 µg de tissu par essai). Après 20 minutes de pré-incubation des composés avec l'enzyme à 25°C, la réaction est démarrée par l'addition de 10µL de substrat par puits (AAMC, concentration finale de 10 µM). Après 40 minutes d'incubation à 37°C, l'aminométhyl coumarin (AMC) produit est mesuré par comptage fluorescent (lecteur de plaque Envision).

Dans ces conditions, les composés les plus actifs de l'invention présentent des CI₅₀ (concentration inhibant de 50 % l'activité enzymatique contrôle de la FAAH) comprises entre 0,001 et 1 µM. Certains composés présentent des CI₅₀ inférieures à 50nM et plus particulièrement inférieures à 15 nM.

Le tableau 3 donne des exemples de CI₅₀ obtenues avec les composés de l'invention.

**Tableau 3**

| N° composé | CI₅₀ (nM) | Protocole utilisé |
|---|---|---|
| 1 | 1 | 1 |
| 2 | 40 | 1 |
| 5 | 2 | 1 |
| 9 | 2 | 1 |
| 12 | 47 | 1 |
| 19 | 1 | 1 |
| 29 | 0,7 | 2 |
| 38 | 2 | 1 |
| 41 | 0,6 | 1 |
| 46 | 16 | 1 |
| 52 | 28 | 1 |
| 64 | 3 | 1 |

Il apparaît donc que les composés selon l'invention ont une activité inhibitrice sur l'enzyme FAAH.

L'activité *in vivo* des composés de l'invention a été évaluée dans un test d'analgésie.

Ainsi, l'administration intrapéritonéale (i.p.) de PBQ (phénylbenzoquinone, 2 mg/kg dans une solution de chlorure de sodium à 0,9 % contenant 5 % d'éthanol) chez des souris mâles OF1 de 25 à 30 g, provoque des étirements abdominaux, en moyenne 30 torsions ou contractions pendant la période de 5 à 15 minutes après injection. Les composés testés sont administrés par voie orale (p.o.) ou par voie intrapéritonéale (i.p.) en suspension dans du Tween 80 à 0,5 %, 60 minutes ou 120 minutes avant l'administration de PBQ. Dans ces conditions, les composés les plus puissants de l'invention réduisent de 30 à 80 % le nombre d'étirements induits par le PBQ, dans une gamme de doses comprise entre 1 et 30 mg/kg.

Par exemple, les composés n°1 et n°19 du tableau réduisent, respectivement, de 55% et de 30% le nombre d'étirements induits par le PBQ, à la dose de 30 mg/kg p.o. à 120 minutes.

L'enzyme FAAH *(*Chemistry and Physics of Lipids, (2000), 108, 107-121) catalyse l'hydrolyse des dérivés endogènes d'amides et d'esters de différents acides gras tels que la *N*-arachidonoyléthanolamine (anandamide), la *N*-palmitoyl-éthanolamine, la *N*-oléoyléthanolamine, l'oléamide ou le 2-arachidonoylglycérol. Ces dérivés exercent différentes activités pharmacologiques en interagissant, entre autres, avec les récepteurs cannabinoïdes et vanilloïdes.

Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoïdes endogènes et/ou tous autres substrats métabolisés par l'enzyme FAAH, sont impliqués. On peut par exemple citer les maladies et les affections suivantes :
la douleur notamment les douleurs aiguës ou chroniques de type neurogène : migraine, douleurs neuropathiques incluant les formes associées au virus de l'herpès et au diabète et à la chimiothérapie, les douleurs aiguës ou chroniques associées aux maladies inflammatoires : arthrite, arthrite rhumatoïde, ostéoarthrite, spondylite, goutte, vascularite, maladie de Crohn, syndrome du colon irritable, les douleurs aiguës ou chroniques périphériques, les vertiges, les vomissements, les nausées en particulier celles consécutives à une chimiothérapie, les troubles du comportement alimentaire en particulier les anorexies et cachexies de diverses natures , les pathologies neurologiques et psychiatriques : tremblements, dyskinésies, dystonies, spasticité, comportements compulsifs et obsessionnels, syndrome de Tourette, toutes les formes de dépression et d'anxiété de toute nature et origine, troubles de l'humeur, psychoses, les maladies neuro-dégénératives aiguës et chroniques : maladie de Parkinson, maladie d'Alzheimer, démence sénile, chorée de Huntington, lésions liées à l'ischémie cérébrale et aux traumatismes crâniens et médullaires, l'épilepsie, les troubles du sommeil incluant les apnées du sommeil, les maladies cardiovasculaires eh particulier hypertension, arythmies cardiaques, artériosclérose, crise cardiaque, ischémies cardiaques, l'ischémie rénale, les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaire, astrocytomes, astroblastomes, épendyomes, oligodendrogliomes, tumeur du plexus, neuroépithéliomes, tumeur de l'épiphyse, épendymoblastomes, méningiomes malins, sarcomatoses, mélanomes malins, schwénnomes), les désordres du système immunitaire, notamment les maladies auto-immunes : psoriasis, lupus érythémateux, maladies du tissu conjonctif ou connectivités, syndrome de Sjögrer's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire, les maladies allergiques : l'hypersensibilité immédiate ou retardée, rhinites ou conjonctivites allergiques, dermatites de contact, les maladies infectieuses parasitaires, virales ou bactériennes : SIDA, méningites, les maladies inflammatoires, notamment les maladies articulaires : arthrite, arthrite rhumatoïde, ostéoarthrite, spondylite, goutte, vascularite, maladie de Crohn, syndrome du colon irritable, l'ostéoporose, les affections oculaires : hypertension oculaire, glaucome, les affections pulmonaires : maladies des voies respiratoires, bronchospasmes, toux, asthme, bronchite chronique, obstruction chronique des voies respiratoires, emphysème, les maladies gastro-intestinales: syndrome du colon irritable, désordres inflammatoires intestinaux, ulcères, diarrhées, l'incontinence urinaire et l'inflammation vésicale.

Les composés selon l'invention, à l'état de base, de sel d'addition à un acide, d'hydrate ou de solvat pharmaceutiquement acceptable, pour leur utilisation dans le traitement des pathologies ci-dessus mentionnées fait partie intégrante de l'invention.

L'invention a également pour objet des médicaments qui comprennent un composé de formule (I), ou un sel d'addition à un acide, ou encore un hydrate ou un solvat pharmaceutiquement acceptable du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel d'addition à un acide, ou un hydrate, ou un solvat pharmaceutiquement acceptable dudit composé, et éventuellement un ou plusieurs excipients pharmaceutiquement acceptables.

Lesdits excipients sont choisis selon la forme pharmaceutique et le mode d'administration souhaité, parmi les excipients habituels qui sont connus de l'homme du métier.

Selon encore un autre de ses aspects, la présente invention a pour objet un composé de formule (I), à l'état de base ou de sel d'addition à un acide pharmaceutiquement acceptable, pour son utilisation comme médicament.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son sel d'addition à un acide, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

L'invention selon un autre de ses aspects, concerne également une méthode de traitement des pathologies ci-dessus indiquées qui comprend l'administration d'une dose efficace d'un composé selon l'invention, d'un de ses sels d'addition à un acide pharmaceutiquement acceptable, d'un solvat ou d'un hydrate dudit composé.

## Revendications

1. Composé répondant à la formule (I) 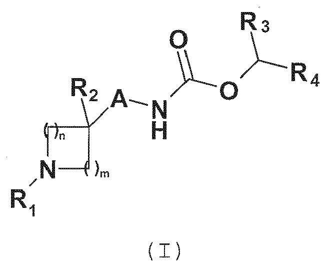 dans laquelle
R₂ représente un atome d'hydrogène ou de fluor ou un groupe hydroxyle, C₁₋₆-alkyle ou NR₈R₉ ;
n représente un nombre entier égal à 1, 2 ou 3 et m représente un nombre entier égal à 1 ou 2;
A représente une liaison covalente ou un groupe C₁₋₈-alkylène ;
R₁ représente un groupe R₅ éventuellement substitué par un ou plusieurs groupes R₆ et/ou R₇ ;
R₅ représente un groupe choisi parmi un phényle, pyridinyle, pyridazinyle, pyrimidinyle, pyrazinyle, triazinyle, naphtalènyle, quinolinyle, isoquinolinyle, phtalazinyle, quinazolinyle, quinoxalinyle, cinnolinyle, naphthyridinyle ; R₆ représente un atome d'halogène, un groupe cyano, -CH₂CN, nitro, hydroxyle, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₃-alkylène, C₃₋₇-cycloalkyle-C₁₋₃-alkylène-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ ou -O-(C₁₋₃-alkylène)-O- ;
R₇ représente un groupe choisi parmi un furanyle, pyrrolyle, thiènyle, oxazolyle, isoxazolyle, thiazolyle, isothiazolyle, imidazolyle, pyrazolyle, oxadiazolyle, thiadiazolyle, phényle, pyridinyle, pyridazinyle, pyrimidinyle, pyrazinyle, triazinyle, naphtalènyle, quinolinyle, isoquinolinyle, phtalazinyle, quinazolinyle, quinoxalinyle, cinnolinyle, naphthyridinyle, imidazopyrimidinyle, thiènopyrimidinyle, benzofuranyle, benzothiènyle, benzimidazolyle, benzoxazolyle, benzisoxazolyle, benzothiazolyle, benzisothiazolyle, indolyle, isoindolyle, indazolyle, pyrrolopyridinyle, furopyridinyle, thiènopyridinyle, imidazopyridinyle, pyrazolopyridinyle, oxazolopyridinyle, isoxazolopyridinyle, thiazolopyridinyle, phényloxy, benzyloxy, pyrimidinoxy; le ou les groupes R₇ pouvant être substitués par un ou plusieurs groupes R₆ identiques ou différents l'un de l'autre ;
R₃ représente un trifluorométhyle, un C₁₋₆-alkyle ou un atome d'hydrogène;
R₄ représente un groupe choisi parmi un furanyle, pyrrolyle, thiènyle, isothiazolyle, oxazolyle, isoxazolyle, pyrazolyle, oxadiazolyle, thiadiazolyle, imidazolyle, triazolyle, tétrazolyle;
ce groupe étant éventuellement substitué par un ou plusieurs substituants choisis parmi un atome d'halogène, un groupe C₁₋₆-alkyle, C₁₋₆-haloalkyle, C₃₋₇-cycloalkyle, C₃₋₇-cycloalkyle-C₁₋₃-alkylène, C₁₋₆-haloalcoxy, cyano, NR₈R₉, NR₈COR₉, NR₈CO₂R₉, NR₈SO₂R₉, NR₈SO₂NR₈R₉, COR₈, CO₂R₈, CONR₈R₉, CON(R₈) (C₁₋₃-alkylène-NR₁₀R₁₁), SO₂R₈, SO₂NR₈R₉, -O-(C₁₋₃-alkylène)-O-, phényle, phényloxy, benzyloxy, pyridinyle, pyrazinyle, pyridazinyle, triazinyle ou pyrimidinyle; les groupes phényle, phényloxy, pyridinyle, pyrazinyle, pyridazinyle, triazinyle et pyrimidinyle pouvant être substitués par un ou plusieurs substituants choisis parmi un atome d'halogène, un groupe cyano, nitro, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₃-alkylène ;
R₈, R₉, R₁₀ et R₁₁ représentent indépendamment l'un de l'autre un atome d'hydrogène ou un groupe C₁₋₆-alkyle,
ou forment avec le ou les atomes qui les portent,
dans le cas de NR₈R₉, un cycle choisi parmi les cycles azétidine, pyrrolidine, pipéridine, morpholine, thiomorpholine, azépine, oxazépine ou pipérazine, ce cycle étant éventuellement substitué par un groupe C₁₋₆-alkyle ou benzyle ;
dans le cas de NR₈COR₉, un cycle lactame ; dans le cas de NR₈CO₂R₉, un cycle oxazolidinone, oxazinone ou oxazépinone; dans le cas de NR₈SO₂R₉, un cycle sultame ; dans le cas de NR₈SO₂NR₈R₉, un cycle dioxyde de thiazolidine ou dioxyde de thiadiazinane ;
à l'état de base ou de sel d'addition à un acide.

2. Composé de formule (I) selon la revendication 1 **caractérisé en ce que** n représente un nombre entier égal à 2 et m représente un nombre entier égal à 2 ;
à l'état de base ou de sel d'addition à un acide.

3. Composé de formule (I) selon la revendication 1 ou 2 **caractérisé en ce que** A représente un groupe C₁₋₈-alkylène ; à l'état de base ou de sel d'addition à un acide.

4. Composé de formule (I) selon l'une quelconque des revendications 1 à 3 **caractérisé en ce que** R₁ représente un groupe R₅ éventuellement substitué par un ou plusieurs groupes R₆ et/ou R₇ ;
R₅ représente un groupe phényle, pyridinyle, pyridazinyle, pyrimidinyle, pyrazinyle, quinolinyle, phtalazinyle ou quinoxalinyle ;
R₆ représente un groupe nitro, C₁₋₆-alkyle, C₁₋₆-alcoxy, C₁₋₆-haloalkyle, C₁₋₆-haloalcoxy, -O-(C₁₋₃-alkylène)-O- ou un atome d'halogène;
R₇ représente un groupe phényle pouvant être substitué par un ou plusieurs groupes R₆ identiques ou différents l'un de l'autre; à l'état de base ou de sel d'addition à un acide.

5. Composé de formule (I) selon l'une quelconque des revendications 1 à 4 **caractérisé en ce que** R₄ représente un groupe choisi parmi un oxazolyle, isoxazolyle, furanyle, imidazolyle, pyrazolyle, oxadiazolyle, thiadiazolyle, triazolyle ; ce groupe éventuellement substitué par un ou
plusieurs substituants choisis parmi un groupe C₁₋₆-alkyle, COOR₈, CON(R₈) (C₁₋₃-alkylène-NR₁₀R₁₁), CONR₈R₉, phényle ; le groupe phényle pouvant être substitué par un ou plusieurs substituants choisis parmi un atome d'halogène ou un groupe C₁₋₆-alcoxy;
R₈ et R₉ représentent indépendamment l'un de l'autre un atome d'hydrogène ou un groupe C₁₋₆-alkyle, ou forment ensemble avec l'atome qui les porte un cycle pipérazine,
R₁₀ et R₁₁ représentent indépendamment l'un de l'autre un atome d'hydrogène ou un groupe C₁₋₆-alkyle;
à l'état de base ou de sel d'addition à un acide.

6. Composé de formule (I) selon l'une quelconque des revendications 1 à 5 **caractérisé en ce que**
R₁ représente un groupe R₅ éventuellement substitué par un ou plusieurs groupes R₆ et/ou R₇ ;
R₅ représente un groupe phényle, pyridinyle, pyrazinyle ou quinolinyle ; R₆ représente un atome d'halogène;
R₇ représente un groupe phényle pouvant être substitué par un ou plusieurs groupes R₆ identiques ou différents l'un de l'autre.
R₂ et R₃ représentent un atome d'hydrogène ;
R₄ représente un groupe 3-carbamoyl-isoxazol-5-yle ;
n représente un nombre entier égal à 2 et m représente un nombre entier égal à 2;
A représente un groupe alkylène;
à l'état de base ou de sel d'addition à un acide.

7. Procédé de préparation d'un composé de formule (I) selon l'une quelconque des revendications 1 à 6, comprenant l'étape consistant à
faire réagir une amine de formule générale (II), 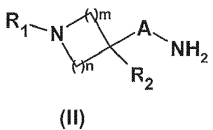 dans laquelle A, R₁, R₂, m et n sont tels que définis dans la formule générale (I) selon la revendication 1,
avec un carbonate de formule générale (III) 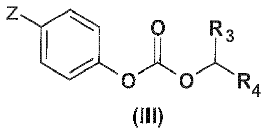 dans laquelle Z représente un atome d'hydrogène ou un groupe nitro, R₃ et R₄ sont tels que définis dans la formule générale (I) selon la revendication 1,
en présence d'une base, dans un solvant à une température comprise entre la température ambiante et la température de reflux du solvant.

8. Procédé de préparation d'un composé de formule (I) selon l'une quelconque des revendications 1 à 6, comprenant l'étape consistant à
faire réagir une amine de formule générale (II), 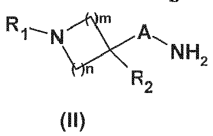 dans laquelle A, R₁, R₂, m et n sont tels que définis dans la formule générale (I) selon la revendication 1,
avec le chloroformiate de phényle ou de 4-nitro-phényle,
en présence d'une base, dans un solvant à une température comprise entre 0°C et la température ambiante,
pour conduire au dérivé carbamate de formule générale (IV), 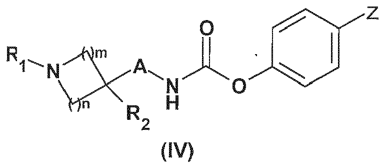 dans laquelle A, R₁, R₂, m et n sont tels que définis dans la formule générale (I) selon la revendication 1, et Z représente un atome d'hydrogène ou un groupe nitro ;
puis à transformer le dérivé carbamate de formule générale (IV) ainsi obtenu en composé de formule générale (I), par action d'un alcool de formule générale HOCHR₃R₄ (IIIa), dans laquelle R₃ et R₄ sont tels que définis dans la formule générale (I) selon la revendication 1,
en présence d'une base, dans un solvant à une température comprise entre la température ambiante et la température de reflux du solvant.

9. Procédé de préparation d'un composé de formule (I) selon l'une quelconque des revendications 1 à 6 et dans laquelle R₁ représente un groupe R₅ substitué notamment par un groupe R₆ de type C₁₋₆-alkyle, C₃₋₇-cycloalkyle ou C₃₋₇-cycloalkyle-C₁₋₃-alkylène, ou par un groupe R₇ tel que défini dans la formule générale (I), comprenant l'étape consistant à effectuer sur le composé de formule générale (Ia), 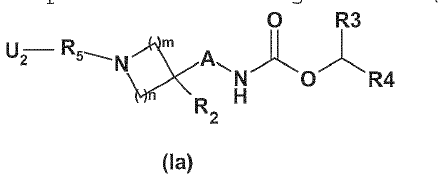 dans laquelle A, R₂, R₃, R₄, R₅, n et m sont tels que définis dans la formule générale (I) selon la revendication 1 et U₂ représente un atome de chlore, de brome, d'iode ou un groupement O-triflate, U₂ étant dans la position où l'on souhaite introduire le groupe R₆ ou R₇,
une réaction catalysée au moyen d'un métal de transition choisie parmi :
- une réaction de type Suzuki, au moyen d'un acide boronique d'alkyle, de cycloalkyle, d'aryle ou d'hétéroaryle,
- une réaction de type Stille, en utilisant un dérivé *tri-*alkylstanneux d'aryle ou d'hétéroaryle,
- une réaction de type Négishi, en utilisant un dérivé zincate d'halogénure d'alkyle, de cycloalkyle, d'aryle ou d'hétéroaryle.

10. Composé de formule (I) selon l'une quelconque des revendications 1 à 6, à l'état de base ou de sel d'addition à un acide pharmaceutiquement acceptable, pour son utilisation comme médicament.

11. Composition pharmaceutique contenant au moins un composé de formule (I) selon l'une quelconque des revendications 1 à 6, à l'état de base ou de sel d'addition à un acide pharmaceutiquement acceptable et éventuellement un ou plusieurs excipients pharmaceutiquement acceptables.

12. Composé de formule (I) selon l'une quelconque des revendications 1 à 6, à l'état de base ou de sel d'addition à un acide pharmaceutiquement acceptable, pour son utilisation dans le traitement d'une pathologie dans laquelle les cannabinoïdes endogènes et/ou tous autres substrats métabolisés par l'enzyme FAAH, sont impliqués, ladite pathologie étant choisie parmi les douleurs aiguës ou chroniques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires, virales ou bactériennes, les maladies inflammatoires, l'ostéoporose, les affections oculaires, les affections pulmonaires, les maladies gastro-intestinales ou l'incontinence urinaire.

## Patentansprüche

1. Verbindung der Formel (I) 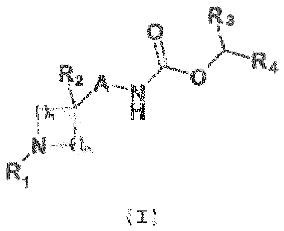 worin
R₂ für ein Wasserstoff- oder Fluoratom oder eine Hydroxyl-, C₁₋₆-Alkyl- oder NR₈R₉-Gruppe steht;
n eine ganze Zahl gleich 1, 2 oder 3 darstellt und m eine ganze Zahl gleich 1 oder 2 darstellt;
A für eine kovalente Bindung oder eine C₁₋₈-Alkylengruppe steht;
R₁ für eine Gruppe R₅ steht, die gegebenenfalls mit einer oder mehreren Gruppen R₆ und/oder R₇ substituiert ist;
R₅ für eine Gruppe steht, die aus Phenyl, Pyridinyl, Pyridazinyl, Pyrimidinyl, Pyrazinyl, Triazinyl, Naphthalinyl, Chinolinyl, Isochinolinyl, Phthalazinyl, Chinazolinyl, Chinoxalinyl, Cinnolinyl, Naphthyridinyl ausgewählt wird;
R₆ für ein Halogenatom, eine Gruppe Cyano, -CH₂CN, Nitro, Hydroxyl, C₁₋₆-Alkyl, C₁₋₆-Alkoxy, C₁₋₆-Thioalkyl, C₁₋₆-Halogenalkyl, C₁₋₆-Halogenalkoxy, C₁₋₆-Halogenthioalkyl, C₃₋₇-Cycloalkyl, C₃₋₇-Cycloalkyl-C₁₋₃-alkylen, C₃₋₇-Cycloalkyl-C₁₋₃-alkylen-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ oder -O-(C₁₋₃-Alkylen)-O- steht;
R₇ für eine Gruppe steht, die aus einem Furanyl, Pyrrolyl, Thienyl, Oxazolyl, Isoxazolyl, Thiazolyl, Isothiazolyl, Imidazolyl, Pyrazolyl, Oxadiazolyl, Thiadiazolyl, Phenyl, Pyridinyl, Pyridazinyl, Pyrimidinyl, Pyrazinyl, Triazinyl, Naphthalinyl, Chinolinyl, Isochinolinyl, Phthalazinyl, Chinazolinyl, Chinoxalinyl, Cinnolinyl, Naphthyridinyl, Imidazopyrimidinyl, Thienopyrimidinyl, Benzofuranyl, Benzothienyl, Benzimidazolyl, Benzoxazolyl, Benzisoxazolyl, Benzothiazolyl, Benzisothiazolyl, Indolyl, Isoindolyl, Indazolyl, Pyrrolopyridinyl, Furopyridinyl, Thienopyridinyl, Imidazopyridinyl, Pyrazolopyridinyl, Oxazolopyridinyl. Isoxazolopyridinyl, Thiazolopyridinyl, Phenyloxy, Benzyloxy, Pyrimidinoxy ausgewählt wird, wobei die Gruppe(n) R₇ mit einer oder mehreren identischen oder voneinander verschiedenen Gruppen R₆ substituiert sein können;
R₃ für ein Trifluormethyl, ein C₁₋₆-Alkyl oder ein Wasserstoffatom steht;
R₄ für eine Gruppe steht, die aus einem Furanyl, Pyrrolyl, Thienyl, Isothiazolyl, Oxazolyl, Isoxazolyl, Pyrazolyl, Oxadiazolyl, Thiadiazolyl, Imidazolyl, Triazolyl, Tetrazolyl ausgewählt wird;
wobei diese Gruppe gegebenenfalls mit einem oder mehreren Substituenten substituiert ist, der/die aus einem Halogenatom, einer Gruppe C₁₋₆-Alkyl, C₁₋₆-Halogenalkyl, C₃₋₇-Cycloalkyl, C₃₋₇-Cycloalkyl-C₁₋₃-alkylen, C₁₋₆-Halogenalkoxy, Cyano, NR₈R₉, NR₈COR₉, NR₈CO₂R₉, NR₈SO₂R₉, NR₈SO₂NR₈R₉, COR₈, CO₂R₈, CONR₈R₉, CON(R₈) (C₁₋₃-Alkylen-NR₁₀R₁₁), SO₂R₈, SO₂NR₈R₉, -O-(C₁₋₃-Alkylen)-O-, Phenyl, Phenyloxy, Benzyloxy, Pyridinyl, Pyrazinyl, Pyridazinyl, Triazinyl oder Pyrimidinyl ausgewählt wird/werden; wobei die Phenyl-, Phenyloxy-, Pyridinyl-, Pyrazinyl-, Pyridazinyl-, Triazinyl- und Pyrimidinylgruppen mit einem oder mehreren Substituenten substituiert sein können, der/die aus einem Halogenatom, einer Gruppe Cyano, Nitro, C₁₋₆-Alkyl, C₁₋₆-Alkoxy, C₁₋₆-Thioalkyl, C₁₋₆-Halogenalkyl, C₁₋₆-Halogenalkoxy, C₁₋₆-Halogenthioalkyl, C₃₋₇-Cycloalkyl, C₃₋₇-Cycloalkyl-C₁₋₃-alkylen ausgewählt wird/werden;
R₈, R₉, R₁₀ und R₁₁ unabhängig voneinander ein Wasserstoffatom oder eine C₁₋₆-Alkylgruppe darstellen oder mit dem Atom oder den Atomen, die sie tragen,
im Fall von NR₈R₉ einen Ring bilden, der aus Azetidin-, Pyrrolidin-, Piperidin-, Morpholin-, Thiomorpholin-, Azepin-, Oxazepin- oder Piperazinringen ausgewählt wird, wobei dieser Ring gegebenenfalls mit einer C₁₋₆-Alkyl- oder Benzylgruppe substituiert ist;
im Fall von NR₈COR₉ einen Lactamring bilden; im Fall von NR₈CO₂R₉ einen Oxazolidinon-, Oxazinon- oder Oxazepinonring bilden; im Fall von NR₈SO₂R₉ einen Sultamring bilden; im Fall von NR₈SO₂NR₈R₉ einen Thiazolidindioxid- oder Thiadiazinandioxidring bilden;
in Form einer Base oder eines Additionssalzes mit einer Säure.

2. Verbindung der Formel (I) nach Anspruch 1, **dadurch gekennzeichnet, dass** n eine ganze Zahl gleich 2 darstellt und m eine ganze Zahl gleich 2 darstellt;
in Form einer Base oder eines Additionssalzes mit einer Säure.

3. Verbindung der Formel (I) nach Anspruch 1 oder 2, **dadurch gekennzeichnet, dass** A für eine C₁₋₈-Alkylengruppe steht;
in Form einer Base oder eines Additionssalzes mit einer Säure.

4. Verbindung der Formel (I) nach einem der Ansprüche 1 bis 3, **dadurch gekennzeichnet, dass** R₁ für eine Gruppe R₅ steht, die gegebenenfalls mit einer oder mehreren Gruppen R₆ und/oder R₇ substituiert ist;
R₅ für eine Phenyl-, Pyridinyl-, Pyridazinyl-, Pyrimidinyl-, Pyrazinyl-, Chinolinyl-, Phthalazinyl- oder Chinoxalinylgruppe steht;
R₆ für eine Gruppe Nitro, C₁₋₆-Alkyl, C₁₋₆-Alkoxy, C₁₋₆-Halogenalkyl, C₁₋₆-Halogenalkoxy, -O-(C₁₋₃-Alkylen)-O-oder ein Halogenatom steht;
R₇ eine Phenylgruppe darstellt, die mit einer oder mehreren identischen oder voneinander verschiedenen Gruppen R₆ substituiert sein kann; in Form einer Base oder eines Additionssalzes mit einer Säure.

5. Verbindung der Formel (I) nach einem der Ansprüche 1 bis 4, **dadurch gekennzeichnet, dass** R₄ für eine aus Oxazolyl, Isoxazolyl, Furanyl, Imidazolyl, Pyrazolyl, Oxadiazolyl, Thiadiazolyl, Triazolyl ausgewählte Gruppe steht; wobei diese Gruppe gegebenenfalls mit einem oder mehreren Substituenten substituiert ist, der/die aus einer Gruppe C₁₋₆-Alkyl, COOR₈, CON(R₈) (C₁₋₃-Alkylen-NR₁₀R₁₁), CONR₈R₉, Phenyl ausgewählt wird/werden; wobei die Phenylgruppe mit einem oder mehreren Substituenten substituiert sein kann, der/die aus einem Halogenatom oder einer C₁₋₆-Alkoxygruppe ausgewählt wird/werden;
R₈ und R₉ unabhängig voneinander ein Wasserstoffatom oder eine C₁₋₆-Alkylgruppe darstellen oder zusammen mit dem Atom, das sie trägt, einen Piperazinring bilden,
R₁₀ und R₁₁ unabhängig voneinander ein Wasserstoffatom oder eine C₁₋₆-Alkylgruppe darstellen;
in Form einer Base oder eines Additionssalzes mit einer Säure.

6. Verbindung der Formel (I) nach einem der Ansprüche 1 bis 5, **dadurch gekennzeichnet, dass**
R₁ für eine Gruppe R₅ steht, die gegebenenfalls mit einer oder mehreren Gruppen R₆ und/oder R₇ substituiert ist;
R₅ eine Phenyl-, Pyridinyl-, Pyrazinyl- oder Chinolinylgruppe darstellt; R₆ für ein Halogenatom steht;
R₇ eine Phenylgruppe darstellt, die mit einer oder mehreren identischen oder voneinander verschiedenen Gruppen R₆ substituiert sein kann;
R₂ und R₃ ein Wasserstoffatom darstellen;
R₄ für eine 3-Carbamoylisoxazol-5-ylgruppe steht;
n eine ganze Zahl gleich 2 darstellt und m eine ganze Zahl gleich 2 darstellt;
A eine Alkylengruppe darstellt;
in Form einer Base oder eines Additionssalzes mit einer Säure.

7. Verfahren zur Herstellung einer Verbindung der Formel (I) nach einem der Ansprüche 1 bis 6, umfassend einen Schritt der Umsetzung eines Amins der allgemeinen Formel (II) 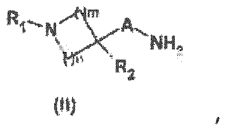 worin A, R₁, R₂, m und n wie in der allgemeinen Formel (I) nach Anspruch 1 definiert sind,
mit einem Carbonat der allgemeinen Formel (III) 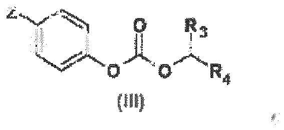 worin Z für ein Wasserstoffatom oder eine Nitrogruppe steht, R₃ und R₄ wie in der allgemeinen Formel (I) nach Anspruch 1 definiert sind,
in Gegenwart einer Base in einem Lösungsmittel bei einer Temperatur zwischen Umgebungstemperatur und der Rückflusstemperatur des Lösungsmittels.

8. Verfahren zur Herstellung einer Verbindung der Formel (I) nach einem der Ansprüche 1 bis 6, umfassend den Schritt der Umsetzung eines Amins der allgemeinen Formel (II) 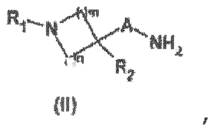 worin A, R₁, R₂, m und n wie in der allgemeinen Formel (I) nach Anspruch 1 definiert sind,
mit Phenyl- oder 4-Nitrophenylchlorameisensäureester in Gegenwart einer Base in einem Lösungsmittel bei einer Temperatur zwischen 0 °C und Umgebungstemperatur,
um das Carbamatderivat der allgemeinen Formel (IV) zu erhalten 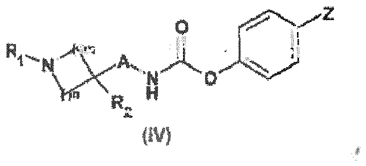 worin A, R₁, R₂, m und n wie in der allgemeinen Formel (I) nach Anspruch 1 definiert sind, und Z für ein Wasserstoffatom oder eine Nitrogruppe steht;
und das anschließende Umwandeln des so erhaltenen Carbamatderivats der allgemeinen Formel (IV) in eine Verbindung der allgemeinen Formel (I) durch Einwirkung eines Alkohols der allgemeinen Formel HOCHR₃R₄ (IIIa), worin R₃ und R₄ wie in der allgemeinen Formel (I) nach Anspruch 1 definiert sind,
in Gegenwart einer Base in einem Lösungsmittel bei einer Temperatur zwischen Umgebungstemperatur und der Rückflusstemperatur des Lösungsmittels.

9. Verfahren zur Herstellung einer Verbindung der Formel (I) nach einem der Ansprüche 1 bis 6, worin R₁ für eine Gruppe R₅ steht, die insbesondere mit einer Gruppe R₆ des Typs C₁₋₆-Alkyl, C₃₋₇-Cycloalkyl oder C₃₋₇-Cycloalkyl-C₁₋₃-alkylen oder einer Gruppe R₇, wie in der allgemeinen Formel (I) definiert, substituiert ist, umfassend den Schritt der Durchführung an der Verbindung der allgemeinen Formel (Ia) 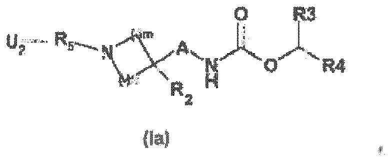 worin A, R₂, R₃, R₄, R₅, n und m wie in der allgemeinen Formel (I) nach Anspruch 1 definiert sind und U₂ für ein Chlor-, Brom-, Iodatom oder eine O-Triflatgruppe steht, wobei sich U₂ an der Position befindet, an der die Gruppe R₆ oder R₇ eingeführt werden soll,
einer mit einem Übergangsmetall katalysierten Reaktion, ausgewählt aus:
- einer Reaktion des Suzuki-Typs mittels einer Alkyl-, Cycloalkyl-, Acryl- oder Heteroarylboronsäure,
- einer Reaktion des Stille-Typs unter Verwendung eines Aryl- oder Heteroaryltrialkylzinnderivats,
- einer Reaktion des Negishi-Typs unter Verwendung eines Alkyl-, Cycloalkyl-, Aryl- oder Heteroarylhalogenidzinkatderivats.

10. Verbindung der Formel (I) nach einem der Ansprüche 1 bis 6 in Form einer Base oder eines Additionssalzes mit einer pharmazeutisch verträglichen Säure zur Verwendung als Medikament.

11. Pharmazeutische Zusammensetzung, die mindestens eine Verbindung der Formel (I) nach einem der Ansprüche 1 bis 6 in Form einer Base oder eines Additionssalzes mit einer pharmazeutisch annehmbaren Säure und gegebenenfalls einen oder mehrere pharmazeutisch annehmbare Excipienten enthält.

12. Verbindung der Formel (I) nach einem der Ansprüche 1 bis 6 in Form einer Base oder eines Additionssalzes mit einer pharmazeutisch annehmbaren Säure für die Verwendung zur Behandlung einer Pathologie, an der endogene Cannabinoide und/oder alle anderen durch das Enzym FAAH metabolisierten Substrate beteiligt sind, wobei die Pathologie aus akuten oder chronischen Schmerzen, Schwindel, Erbrechen, Übelkeit, Störungen des Essverhaltens, neurologischen und psychiatrischen Pathologien, akuten oder chronischen neurodegenerativen Erkrankungen, Epilepsie, Schlafstörungen, kardiovaskulären Erkrankungen, Nierenischämie, Krebserkrankungen, Störungen des Immunsystems, allergischen Krankheiten, parasitischen, viralen oder bakteriellen Infektionskrankheiten, entzündlichen Erkrankungen, Osteoporose, Augenleiden, Lungenleiden, Magen-Darm-Erkrankungen oder Harninkontinenz ausgewählt ist.

## Claims

1. Compound corresponding to the general formula (I) 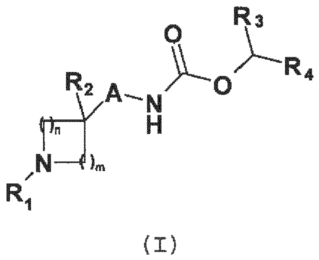 in which
R₂ represents a hydrogen or fluorine atom or a hydroxyl, C₁₋₆-alkyl or NR₈R₉ group;
n represents an integer equal to 1, 2 or 3 and m represents an integer equal to 1 or 2;
A represents a covalent bond or a C₁₋₈-alkylene group;
R₁ represents a R₅ group optionally substituted with one or more R₆ and/or R₇ groups;
R₅ represents a group chosen from phenyl, pyridyl, pyridazinyl, pyrimidinyl, pyrazinyl, triazinyl, naphthyl, quinolinyl, isoquinolinyl, phthalazinyl, quinazolinyl, quinoxalinyl, cinnolinyl and naphthyridinyl;
R₆ represents a halogen atom or a cyano, -CH₂CN, nitro, hydroxyl, C₁₋₆-alkyl, C₁₋₆-alkoxy, C₁₋₆-thioalkyl, C₁₋₆-haloalkyl, C₁₋₆-haloalkoxy, C₁₋₆-halothioalkyl, C₃₋₇-cycloalkyl, C₃₋₇-cycloalkyl-C₁₋₃-alkylene, C₃₋₇-cycloalkyl-C₁₋₃-alkylene-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ or -O-(C₁₋₃-alkylene)-O- group;
R₇ represents a group chosen from furyl, pyrrolyl, thienyl, oxazolyl, isoxazolyl, thiazolyl, isothiazolyl, imidazolyl, pyrazolyl, oxadiazolyl, thiadiazolyl, phenyl, pyridyl, pyridazinyl, pyrimidinyl, pyrazinyl, triazinyl, naphthyl, quinolinyl, isoquinolinyl, phthalazinyl, quinazolinyl, quinoxalinyl, cinnolinyl, naphthyridinyl, imidazopyrimidinyl, thienopyrimidinyl, benzofuryl, benzothienyl, benzimidazolyl, benzoxazolyl, benzisoxazolyl, benzothiazolyl, benzisothiazolyl, indolyl, isoindolyl, indazolyl, pyrrolopyridyl, furopyridyl, thienopyridyl, imidazopyridyl, pyrazolopyridyl, oxazolopyridyl, isoxazolopyridyl, thiazolopyridyl, phenyloxy, benzyloxy and pyrimidinoxy; the group(s) R₇ possibly being substituted with one or more R₆ groups that may be identical to or different from each other;
R₃ represents a trifluoromethyl, a C₁₋₆-alkyl or a hydrogen atom;
R₄ represents a group chosen from furyl, pyrrolyl, thienyl, isothiazolyl, oxazolyl, isoxazolyl, pyrazolyl, oxadiazolyl, thiadiazolyl, imidazolyl, triazolyl and tetrazolyl;
this group being optionally substituted with one or more substituents chosen from a halogen atom, a C₁₋₆-alkyl, C₁₋₆-haloalkyl, C₃₋₇-cycloalkyl, C₃₋₇-cycloalkyl-C₁₋₃-alkylene, C₁₋₆-haloalkoxy, cyano, NR₈R₉, NR₈COR₉, NR₈CO₂R₉, NR₈SO₂R₉, NR₈SO₂NR₈R₉, COR₈, CO₂R₈, CONR₈R₉, CON (R₈) (C₁₋₃-alkylene-NR₁₀R₁₁), SO₂R₈, SO₂NR₈R₉, -O-(C₁₋₃-alkylene)-O-, phenyl, phenyloxy, benzyloxy, pyridyl, pyrazinyl, pyridazinyl, triazinyl or pyrimidinyl group; the phenyl, phenyloxy, pyridyl, pyrazinyl, pyridazinyl, triazinyl and pyrimidinyl groups possibly being substituted with one or more substituents chosen from a halogen atom, a cyano, nitro, C₁₋₆-alkyl, C₁₋₆-alkoxy, C₁₋₆-thioalkyl, C₁₋₆-haloalkyl, C₁₋₆-haloalkoxy, C₁₋₆-halothioalkyl, C₃₋₇-cycloalkyl, C₃₋₇-cycloalkyl-C₁₋₃-alkylene group;
R₈, R₉, R₁₀ and R₁₁ represent, independently of each other,
a hydrogen atom or a C₁₋₆-alkyl group,
or form, with the atom(s) that bear(s) them,
in the case of NR₈R₉, a ring chosen from azetidine, pyrrolidine, piperidine, morpholine, thiomorpholine, azepine, oxazepine or piperazine, this ring being optionally substituted with a C₁₋₆-alkyl or benzyl group; in the case of NR₈COR₉, a lactam ring; in the case of NR₈CO₂R₉, a oxazolidinone, oxazinone or oxazepinone ring; in the case of NR₈SO₂R₉, a sultam ring; in the case of NR₈SO₂NR₈R₉, a thiazolidine dioxide or thiadiazinane dioxide ring;
in the form of base or of an acid-addition salt.

2. Compound of formula (I) according to Claim 1, **characterized in that** n represents an integer equal to 2 and m represents an integer equal to 2;
in the form of base or of an acid-addition salt.

3. Compound of formula (I) according to Claim 1 or 2, **characterized in that** A represents a C₁₋₈-alkylene group; in the form of base or of an acid-addition salt.

4. Compound of formula (I) according to any one of Claims 1 to 3, **characterized in that** R₁ represents a R₅ group optionally substituted with one or more R₆ and/or R₇ groups;
R₅ represents a phenyl, pyridyl, pyridazinyl, pyrimidinyl, pyrazinyl, quinolinyl, phthalazinyl or quinoxalinyl group;
R₆ represents a nitro, C₁₋₆-alkyl, C₁₋₆-alkoxy, C₁₋₆-haloalkyl, C₁₋₆-haloalkoxy, -O-(C₁₋₃-alkylene)-O- or a halogen atom;
R₇ represents a phenyl group that may be substituted with one or more groups R₆ that may be identical to or different from each other; in the form of base or of an acid-addition salt.

5. Compound of formula (I) according to any one of Claims 1 to 4, **characterized in that** R₄ represents a group chosen from oxazolyl, isoxazolyl, furyl, imidazolyl, pyrazolyl, oxadiazolyl, thiadiazolyl and triazolyl groups; this group optionally substituted with one or more substituents chosen from a group C₁₋₆-alkyl, COOR₈, CON(R₈) (C₁₋₃-alkylene-NR₁₀R₁₁), CONR₈R₉, phenyl; the phenyl group possibly being substituted with one or more substituents chosen from a halogen atom and a C₁₋₆-alkoxy group;
R₈ and R₉ represent, independently of each other, a hydrogen atom or a C₁₋₆-alkyl group, or form, together with the atom that bears them, a piperazine ring,
R₁₀ and R₁₁ represent, independently of each other, a hydrogen atom or a C₁₋₆-alkyl group;
in the form of base or of an acid-addition salt.

6. Compound of formula (I) according to any one of Claims 1 to 5, **characterized in that**
R₁ represents a group R₅ optionally substituted with one or more groups R₆ and/or R₇;
R₅ represents a phenyl, pyridyl, pyrazinyl or quinolinyl group; R₆ represents a halogen atom;
R₇ represents a phenyl group that may be substituted with one or more groups R₆ that may be identical to or different from each other.
R₂ and R₃ represent a hydrogen atom;
R₄ represents a 3-carbamoylisoxazol-5-yl group;
n represents an integer equal to 2 and m represents an integer equal to 2;
A represents an alkylene group;
in the form of base or of an acid-addition salt.

7. Process for preparing a compound of formula (I) according to any one of Claims 1 to 6, comprising the step consisting in reacting an amine of general formula (II), 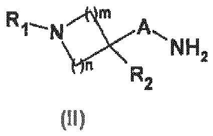 in which A, R₁, R₂, m and n are as defined in the general formula (I) according to Claim 1,
with a carbonate of general formula (III) 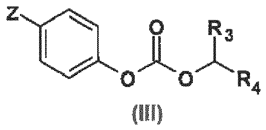 in which Z represents a hydrogen atom or a nitro group, R₃ and R₄ are as defined in the general formula (I) according to Claim 1,
in the presence of a base, in a solvent at a temperature between room temperature and the reflux point of the solvent.

8. Process for preparing a compound of formula (I) according to any one of Claims 1 to 6, comprising the step that consists in reacting an amine of general formula (II), 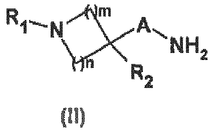 in which A, R₁, R₂, m and n are as defined in the general formula (I) according to Claim 1,
with phenyl or 4-nitrophenyl chloroformate,
in the presence of a base, in a solvent at a temperature between 0°C and room temperature,
to give the carbamate derivative of general formula (IV), 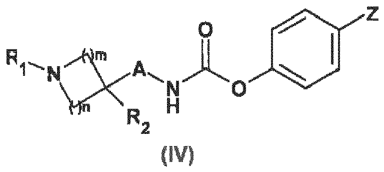 in which A, R₁, R₂, m and n are as defined in the general formula (I) according to Claim 1, and Z represents a hydrogen atom or a nitro group;
and then in converting the carbamate derivative of general formula (IV) thus obtained into a compound of general formula (I), via the action of an alcohol of general formula HOCHR₃R₄ (IIIa), in which R₃ and R₄ are as defined in the general formula (I) according to Claim 1, in the presence of a base, in a solvent at a temperature between room temperature and the reflux point of the solvent.

9. Process for preparing a compound of formula (I) according to any one of Claims 1 to 6 and in which R₁ represents a group R₅ substituted especially with a group R₆ of the type C₁₋₆-alkyl, C₃₋₇-cycloalkyl or C₃₋₇-cycloalkyl-C₁₋₃-alkylene, or with a group R₇ as defined in the general formula (I), comprising the step that consists in performing on the compound of general formula (Ia), 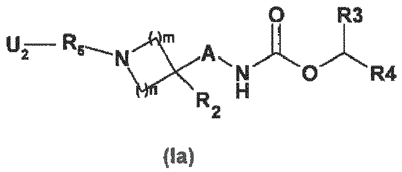 in which A, R₂, R₃, R₄, R₅, n and m are as defined in the general formula (I) according to Claim 1 and U₂ represents a chlorine, bromine or iodine atom or an O-triflate group, U₂ being in the position in which it is desired to introduce the R₆ or R₇ group,
a reaction catalysed by means of a transition metal chosen from:
- a reaction of Suzuki type, by means of an alkyl, cycloalkyl, aryl or heteroaryl boronic acid,
- a reaction of Stille type, using an aryl or heteroaryl trialkylstannous derivative,
- a reaction of Negishi type, using an alkyl, cycloalkyl, aryl or heteroaryl halide zincate derivative.

10. Compound of formula (I) according to any one of Claims 1 to 6, in the form of base or of a pharmaceutically acceptable acid-addition salt, for its use as a medicament.

11. Pharmaceutical composition containing at least one compound of formula (I) according to any one of Claims 1 to 6, in the form of base or of a pharmaceutically acceptable acid-addition salt and optionally one or more pharmaceutically acceptable excipients.

12. Compound of formula (I) according to any one of Claims 1 to 6, in the form of base or of a pharmaceutically acceptable acid-addition salt, for use in the treatment of a pathology in which the endogenous cannabinoids and/or any other substrate metabolized by the enzyme FAAH are involved, said pathology being chosen from acute or chronic pain, vertigo, vomiting, nausea, eating disorders, neurological and psychiatric pathologies, acute or chronic neurodegenerative diseases, epilepsy, sleeping disorders, cardiovascular diseases, renal ischaemia, cancers, immune system disorders, allergic diseases, parasitic, viral or bacterial infectious diseases, inflammatory diseases, osteoporosis, ocular complaints, pulmonary complaints, gastrointestinal diseases or urinary incontinence.
